# EUROPEAN PATENT SPECIFICATION

(11) **EP 2 704 688 B1**
(45) Date of publication and mention of the grant of the patent: **10.07.2019**
(21) Application number: 12779548.2
(22) Date of filing: 04.05.2012
(51) Int. Cl.: A61K 9/00, A61K 31/70, A61K 38/02, A61K 39/00, A61K 31/7048, A61K 31/704, A61K 39/13, A61K 31/472, A61K 31/496, A61K 47/12, A61K 31/07, A61K 39/145, A61K 31/20, A61K 33/30, A61K 33/26, A61K 33/00, A61K 36/54

(54) **COCHLEATE COMPOSITIONS AND METHODS OF MAKING AND USING SAME**
COCHLEATZUSAMMENSETZUNGEN SOWIE VERFAHREN ZU IHRER HERSTELLUNG UND VERWENDUNG
COMPOSITIONS DE COCHLÉATE ET LEURS PROCÉDÉS DE FABRICATION ET D'UTILISATION

(30) Priority: 05.05.2011 US 201161482996 P; 13.09.2011 US 201161534075 P; 14.10.2011 US 201161547325 P; 13.12.2011 US 201161570067 P; 25.01.2012 US 201261590531 P; 08.03.2012 US 201261608272 P; 05.04.2012 US 201261620656 P; 23.04.2012 US 201261636793 P
(43) Date of publication of application: 12.03.2014
(73) Proprietor: Matinas BioPharma Nanotechnologies, Inc., Bedminster, NJ 07921 (US); Rutgers, the State University of New Jersey, New Brunswick, NJ 08909 (US)
(72) Inventor: LU, Ruying, New Providence, NJ 07974 (US); MANNINO, Raphael, Glen Gardner, NJ 08826 (US)
(74) Representative: Hoffmann Eitle
(86) International application number: PCT/US2012/036576
(87) International publication number: WO 2012/151517

(56) References cited:
- WO-A2-03/082209
- WO-A2-2005/110361
- US-A- 5 643 574
- US-A1- 2007 237 814
- US-A1- 2009 028 904
- US-A1- 2010 178 325
- GIBSON, BRIAN ET AL.: 'A Novel Gene Delivery System for Mammalian Cells.' ANTICANCER RESEARCH vol. 24, 2004, pages 483 - 488, XP009037646

## Description

### Background of the Invention

Cochleates are anhydrous, stable, multi-layered lipid crystals which spontaneously form upon the interaction of phosphatidylserine and calcium (see, for example, U.S. Pat. Nos. 4,078,052; 5,643,574; 5,840,707; 5,994,318; 6,153,217; 6,592,894, as well as PCT Publ. Nos. WO 200404/091572; WO 2004/091578; WO 2005/110361 and U.S. Pat. Publ. 2010/0178325
. Cochleate formulations remain intact in physiological fluids, including mucosal secretions, plasma and gastrointestinal fluid, thereby mediating the delivery of biologically active compounds by many routes of administration, including oral, mucosal and intravenous. Traditionally, such cochleate formulations have been restricted to the incorporation of hydrophobic active pharmaceutical ingredients (APIs), such as amphotericin B (AmB). Accordingly, there is a great need in the art to provide compositions and methods for making and using cochleate compositions suitable for the stable and enhanced encochleation of hydrophilic APIs.

### Summary of the Invention

The present invention is based, at least in part, on the discovery that stable cochleate formulations can be made to encochleate biologically relevant molecules of interest and especially to enhance the encochleation of hydrophilic biologically relevant molecules.

The present invention provides a cochleate composition comprising a population of cochleates, wherein the cochleates comprise: a) a negatively charged first lipid; b) a cation, wherin the cation is a divalent cation selected from the group consisting of calcium, zinc, barium and magnesium cations; c) a neutral second lipid; and d) a biologically relevant molecule, wherein the biologically relevant molecule is an aminoglycoside; wherein the ratio of the negatively charged first lipid to the neutral second lipid is between 1:1 to 9:1; wherein the negatively charged first lipid is phosphatidylserine and comprises at least 50% of the total lipid; wherein the neutral second lipid is phosphatidylcholine or sphinomyelin.
Pharmaceutical compositions comprising such cochleate compositions, methods making such cochleate compositions, and methods of using such cochleate compositions for therapeutic applications are also provided.

### Brief Description of the Drawings

**Figure 1** shows a representative schematic of an exemplary scheme for preparing cochleate formulations.
**Figures 2A-2B** show the results of treating *M. avium* 101-infected (Figure 2A) and *M.* avium 109-infected (Figure 2B) macrophages with amikacin-cochleate preparations.
**Figures 3A-3B** show *in vivo* efficacy results of *M. avium* 101-infected C57/B16 mice treated with oral (Figure 3A) or intraperitoneal (Figure 3B) amikacin-cochleate preparations.
**Figure 4** shows a structural diagram of amphotericin B (AmB).
**Figure 5** shows a schematic representation of a manufacturing strategy for making amphotericin B-cochleates (CAMB).
**Figure 6** shows the results of treating *Candida* infection in macrophage *in vitro* using CAMB and Fungizone (DAMB).
**Figure 7** shows a schematic protocol for assessing survival and tissue burden analysis of systemic *Aspergillosis* infection of mice and subsequent treatment with oral amphotericin B-cochleates (CAMB).
**Figure 8** shows the results of treating mice infected with *Aspergillus f.* using CAMB and DAMB.
**Figure 9** shows Apergillus fungal tissue burden in mice infected with *Aspergillus f.* and treated with CAMB.
**Figure 10** shows the results of CAMB pharmacokinetic data from administration to dogs and humans.
**Figure 11** shows anti-influenza specific serum antibody titers resulting from oral or intramuscular administration of influenza protein-cochleates.
**Figure 12** shows the percentage of mice with no detectable influenza virus levels resulting from oral administration of influenza protein-cochleates.
**Figures 13A-13B** show a schematic diagram of an encochleation process (Figure 13A) and product (Figure 13B) useful for enhancing the encochleation of hydrophilic molecules.
**Figure 14** shows the results of hydrophilic API encochleation amounts according to different lipid compositions present within the cochleates presented for encochleation of the API.

### Detailed Description of the Invention

The present invention relates to the discovery that stable cochleate formulations can be made to encochleate biologically relevant molecules of interest and especially to enhance the encochleation of hydrophilic biologically relevant molecules. In particular, it was surprising and unexpected that the addition of a second lipid having characteristics defined herein can dramatically increase the efficiency with which hydrophilic biologically relevant molecules can be encochleated.

In order that the present invention may be more readily understood, certain terms are first defined. Additional definitions are set forth throughout the detailed description.

### I. Definitions

The articles "a" and "an" are used herein to refer to one or to more than one (*i.e.* to at least one) of the grammatical object of the article. By way of example, "an element" means one element or more than one element.

As used herein, the term "acyl" refers to a carbonyl group that is attached through its carbon atom to a hydrogen (i.e., a formyl) or an aliphatic group (e.g., acetyl), and the like.

As used herein, the term "aliphatic group" includes organic molecules characterized by straight or branched-chains, typically having between 1 and 24 carbon atoms. In complex structures, the chains may be branched, bridged, or cross-linked. Aliphatic groups include aliphatic groups, alkenyl groups, alkynyl groups, and any combination thereof.

As used herein, the term "alkyl" refers to chemical groups including saturated hydrocarbons having one or more carbon atoms, e.g., between 1 and 24 carbon atoms, including straight-chain alkyl groups (e.g., methyl, ethyl, propyl, butyl, pentyl, hexyl, heptyl, octyl, nonyl, decyl, etc.) and branched-chain alkyl groups (isopropyl, tert-butyl, sec-butyl, isobutyl, etc.). It is to be understood that both straight-chain and branched-chain alkyl groups are encompassed by the term. In certain embodiments, a straight-chain or branched-chain alkyl group may have 30 or fewer carbon atoms in its backbone, e.g., C1-C30 for straight-chain or C3-C30 for branched-chain. In certain embodiments, a straight-chain or branched-chain alkyl group may have 20 or fewer carbon atoms in its backbone, e.g., C1-C20 for straight-chain or C3-C20 for branched-chain, and in more particular embodiments 18 or fewer. For example, the term "C4-C24" as in "C4-C24 alkyl" means alkyl groups containing 4 to 24 carbon atoms.

As used herein, the terms "alkenyl", "alkynyl" and "alkenylene" refer to unsaturated aliphatic groups analogous to alkyls, but which contain at least one double or triple carbon-carbon bond respectively.

As used herein, the term "body fluid" refers to fluids that are excreted or secreted from the body as well as fluid that are normally not (*e*.*g*. amniotic fluid, aqueous humor, bile, blood and blood plasma, cerebrospinal fluid, cerumen and earwax, cowper's fluid or pre-ejaculatory fluid, chyle, chyme, stool, female ejaculate, interstitial fluid, intracellular fluid, lymph, menses, breast milk, mucus, pleural fluid, pus, saliva, sebum, semen, serum, sweat, synovial fluid, tears, urine, vaginal lubrication, vitreous humor, vomit).

As used herein, the terms "cochleate," "lipid precipitate" and "precipitate" are used interchangeably to refer to a lipid precipitate component that generally includes alternating cationic and lipid bilayer sheets with little or no internal aqueous space, typically stacked and/or rolled up, wherein the cationic sheet is comprised of one or more multivalent cations. Additionally, the term "encochleated" means associated with the cochleate structure, e.g., by incorporation into the cationic sheet, and/or inclusion in the lipid bilayer.

As used herein "contacting cells" is defined as exposing the cells to one or more combinations of agents described herein. In one embodiment, such combinations can be administered to cells, directly or indirectly, using local, regional or systemic means.

The term "modulate" includes downregulation and upregulation. The term "downregulate," "decrease," "reduce," "inhibit," and the like are all used herein generally to mean a decrease by a statistically significant amount. The term "upregulate," "increase," "enhance," and the like are all used herein generally to mean an increase by a statistically significant amount. For example, an increase or a decrease can be by at least about 10%, 20%, 25%, 30%, 35%, 40%, 45%, 50%, 55%, 60%, 65%, 70%, 75%, 80%, 85%, 90%, 95%, 98%, 99%, 100%, 150%, 200%, 250%, 300%, 350%, 400%, 450%, 500%, 1000%, or more or any range in between 10-1000% inclusive as compared to a control. In some embodiments, the control can be a change in a cell state such as cell proliferation in the presence versus the absence of treatment. In another embodiment, the control can be activity of a wild type polypeptide of interest. An "overactivity" or "significantly higher level of activity" refers to an activity level of a molecule or test sample that is greater than the standard error of the assay used to assess the activity, and is preferably at least twice, and more preferably three, four, five or ten or more times the activity relative to a reference or control sample and preferably, the average activity in several control samples. The term "underactivity" refers to the opposite of "overactivity."

As used herein, the term "multivalent cation" refers to a divalent cation or higher valency cation, or any compound that has at least two positive charges, including mineral cations such as calcium, barium, zinc, iron and magnesium and other elements, such as drugs and other compounds, capable of forming ions or other structures having multiple positive charges capable of chelating and bridging negatively charged lipids. Additionally or alternatively, the multivalent cation can include other multivalent cationic compounds, e.g., cationic or protonized biologically relevant molecules.

As used herein, the term "protonizable" refers to a molecule that has the ability to gain or more protons. Protonizable molecules can be weakly basic, and can be protonized by acidification or addition of a proton. Additionally or alternatively, the protonizable molecules can be neutral or weakly acidic and can be protonized in the same manner. Thus, the protonizable molecules can be an anionic or neutral, which is rendered cationic by protonization, or the protonizable molecule can be cationic, and be rendered more cationic upon protonization. Optionally, the protonized state can be induced, e.g., by acidification or other methods, as described herein. Protonization renders the molecule cationic or increases the valency of a biologically relevant molecule that is already cationic, e.g., from monovalent to divalent or trivalent. Similarly, the term "protonization," as used herein, refers to the process of increasing the valency of a molecule. "Protonized" refers to a biologically relevant molecule that has undergone protonization. Thus, valency can be increased, e.g. from 0 to 1, from 1 to 2, from 2 to 3, from 3 to 4, or any combination thereof, e.g., from 0 to 3. Any method to increase valency, e.g., increasing pH, can be used to protonize a molecule. The term "weakly basic" refers to molecules that, at neutral pH, have the ability to accept protons. That is, weakly basic biologically relevant molecules are capable of being rendered cationic or more cationic by protonization. As such, weakly basic molecules can be anionic or neutral, and be rendered cationic by protonization. Alternatively, weakly basic molecules can be cationic, and can be rendered more cationic, i.e., polycationic, by protonization. By contrast, "weakly acidic" molecules, at neutral pH, have the ability to give up protons. "Protonizable weakly acidic" molecules, due to their weak acidity, have the ability to accept protons at decreased pH.

As used herein, the term "relevant biological molecule," refers to a molecule to be encochleated, and generally does not refer to the lipid and ion used to precipitate the cochleate. Biologically relevant molecules include any compounds having a property of biological interest, e.g., ones that have a role in the life processes of a living organism. A biologically relevant molecule may be organic or inorganic, a monomer or a polymer, endogenous to a host organism or not, naturally occurring or synthesized in vitro and the like.

As used herein, the term "survival" includes all of the following: survival until mortality, also known as overall survival (wherein said mortality may be either irrespective of cause or tumor related); "recurrence-free survival" (wherein the term recurrence shall include both localized and distant recurrence); metastasis free survival; disease free survival (wherein the term disease shall include cancer and diseases associated therewith). The length of said survival may be calculated by reference to a defined start point (*e*.*g*., time of diagnosis or start of treatment) and end point (*e*.*g*., death, recurrence or metastasis). In addition, criteria for efficacy of treatment can be expanded to include response to chemotherapy, probability of survival, probability of metastasis within a given time period, and probability of tumor recurrence.

As used herein, the term "subject" shall mean any animal including, without limitation, a human, a mouse, a rat, a rabbit, a non-human primate, or any other mammal. In one embodiment, the subject is a primate. In another embodiment, the subject is a human.

As used herein, the term "synergistic" refers to a combination of therapeutic agents described herein, which, when taken together, is more effective than the additive effects of the individual therapies. A synergistic effect of a combination of therapies (*e*.*g*., a combination of therapeutic agents) permits the use of lower dosages of one or more of the therapeutic agent(s) and/or less frequent administration of the agent(s) to a subject with a disease or disorder, *e*.*g*., a proliferative disorder. The ability to utilize lower the dosage of one or more therapeutic agent and/or to administer the therapeutic agent less frequently reduces the toxicity associated with the administration of the agent to a subject without reducing the efficacy of the therapy in the treatment of a disease or disorder. In addition, a synergistic effect can result in improved efficacy of agents in the prevention, management or treatment of a disease or disorder, *e*.*g*. a proliferative disorder. Finally, a synergistic effect of a combination of therapies may avoid or reduce adverse or unwanted side effects associated with the use of either therapeutic agent alone. As used herein, the term "in combination" refers to the use of more than one therapeutic agent. The use of the term "in combination" does not restrict the order in which the therapeutic agents are administered to a subject with a disease or disorder, *e.g*., a proliferative disorder. A first therapeutic agent, such as a compound described herein, can be administered prior to (*e.g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapeutic agent, such as an aminoglycoside-cochleatc composition, to a subject with a disease or disorder.

A "transcribed polynucleotide" or "nucleotide transcript" is a polynucleotide (*e*.*g*. an mRNA, hnRNA, a cDNA, or an analog of such RNA or cDNA) which is complementary to or homologous with all or a portion of a mature mRNA made by transcription of a marker of the disclosure and normal post-transcriptional processing (*e*.*g*. splicing), if any, of the RNA transcript, and reverse transcription of the RNA transcript.

There is a known and definite correspondence between the amino acid sequence of a particular protein and the nucleotide sequences that can code for the protein, as defined by the genetic code (shown below). Likewise, there is a known and definite correspondence between the nucleotide sequence of a particular nucleic acid and the amino acid sequence encoded by that nucleic acid, as defined by the genetic code.

**GENETIC CODE**

| | |
|---|---|
| Alanine (Ala, A) | GCA, GCC, GCG, GCT |
| Arginine (Arg, R) | AGA, ACG, CGA, CGC, CGG, CGT |
| Asparagine (Asn, N) | AAC, AAT |
| Aspartic acid (Asp, D) | GAC, GAT |
| Cysteine (Cys, C) | TGC, TGT |
| Glutamic acid (Glu, E) | GAA, GAG |
| Glutamine (Gln, Q) | CAA, CAG |
| Glycine (Gly, G) | GGA, GGC, GGG, GGT |
| Histidine (His, H) | CAC, CAT |
| Isoleucine (Ile, I) | ATA, ATC, ATT |
| Leucine (Leu, L) | CTA, CTC, CTG, CTT, TTA, TTG |
| Lysine (Lys, K) | AAA, AAG |
| Methionine (Met, M) | ATG |
| Phenylalanine (Phe, F) | TTC, TTT |
| Proline (Pro, P) | CCA, CCC, CCG, CCT |
| Serine (Ser, S) | AGC, AGT, TCA, TCC, TCG, TCT |
| Threonine (Thr, T) | ACA, ACC, ACG, ACT |
| Tryptophan (Trp, W) | TGG |
| Tyrosine (Tyr, Y) | TAC, TAT |
| Valine (Val, V) | GTA, GTC, GTG, GTT |
| Termination signal (end) | TAA, TAG, TGA |

An important and well known feature of the genetic code is its redundancy, whereby, for most of the amino acids used to make proteins, more than one coding nucleotide triplet may be used (illustrated above). Therefore, a number of different nucleotide sequences may code for a given amino acid sequence. Such nucleotide sequences are considered functionally equivalent since they result in the production of the same amino acid sequence in all organisms (although certain organisms may translate some sequences more efficiently than they do others). Moreover, occasionally, a methylated variant of a purine or pyrimidine may be found in a given nucleotide sequence. Such methylations do not affect the coding relationship between the trinucleotide codon and the corresponding amino acid.

In view of the foregoing, the nucleotide sequence of a DNA or RNA coding for a fusion protein or polypeptide of the disclosure or any portion thereof) can be used to derive the fusion protein or polypeptide amino acid sequence, using the genetic code to translate the DNA or RNA into an amino acid sequence. Likewise, for fusion protein or polypeptide amino acid sequence, corresponding nucleotide sequences that can encode the fusion protein or polypeptide can be deduced from the genetic code (which, because of its redundancy, will produce multiple nucleic acid sequences for any given amino acid sequence). Thus, description and/or disclosure herein of a nucleotide sequence which encodes a fusion protein or polypeptide should be considered to also include description and/or disclosure of the amino acid sequence encoded by the nucleotide sequence. Similarly, description and/or disclosure of a fusion protein or polypeptide amino acid sequence herein should be considered to also include description and/or disclosure of all possible nucleotide sequences that can encode the amino acid sequence.

### II. Cochleate Compositions

### A. Cochleates

Cochleates and methods for making and using same have been disclosed in, for example, U.S. Pat. Nos. 4,078,052; 5,643,574; 5,840,707; 5,994,318; 6,153,217; 6,592,894, as well as PCT Publ. Nos. WO 200404/091572; WO 2004/091578; WO 2005/110361 and U.S. Pat. Publ. 2010/0178325 . Cochleate delivery vehicles are stable lipid-cation precipitates that can be composed of simple, naturally occurring materials, e.g., phosphatidylserine, and calcium. Mixtures of naturally occurring molecules (e.g., soy lipids) and/or synthetic or modified lipids can be utilized.

The cochleate structure provides protection from degradation for associated "encochleated" molecules. Divalent cation concentrations *in vivo* in serum and mucosal secretions are such that the cochleate structure is maintained. Hence, the majority of cochleate-associated molecules, e.g., biologically relevant molecules, are present in the inner layers of a primarily solid, non-aqueous, stable, impermeable structure. Since the cochleate structure includes a series of solid layers, components within the interior of the cochleate structure remain substantially intact, even though the outer layers of the cochleate may be exposed to harsh environmental conditions or enzymes.

The cochleate interior is primarily free of water and resistant to penetration by oxygen. Oxygen and water are primarily responsible for the decomposition and degradation of molecules which can lead to reduced shelf-life. Accordingly, encochleation can also impart extensive shelf-life stability to encochleated biologically relevant molecules.

With respect to storage, cochleates can be stored in cation-containing buffer, or lyophilized or otherwise converted to a powder, and stored at room temperature. If desired, the cochleates also can be reconstituted with liquid prior to administration. Cochleate preparations have been shown to be stable for more than two years at 4°C in a cation-containing buffer, and at least one year as a lyophilized powder at room temperature.

In one embodiment, the cochleate comprises a) a negatively charged lipid component, b) a divalent cation component, and c) a neutral second lipid that
interacts with the cation.

Cochleates readily can be prepared from safe, simple, well-defined, naturally occurring substances, e.g., PS and calcium. Mixtures of naturally occurring (e.g., soy lipids), synthetic lipids, and/or modified lipids can also be utilized. Phosphatidylserine is a natural component of all biological membranes, and is most concentrated in the brain. The phospholipids used can be produced synthetically, or prepared from natural sources. Soy PS is inexpensive, available in large quantities and suitable for use in humans. Clinical studies indicate that PS is safe and may play a role in the support of mental functions in the aging brain. Unlike many cationic lipids, cochleates (which are at least partially composed of anionic lipids) are non-inflammatory and biodegradable. The tolerance in vivo of mice to multiple administrations of cochleates by various routes, including intravenous, intraperitoneal, intranasal and oral, has been evaluated. Multiple administrations of high doses of cochleate formulations to the same animal show no toxicity, and do not result in either the development of an immune response to the cochleate matrix, or any side effects relating to the cochleate vehicle.

As used herein, the term, "neutral lipids," include any of a number of lipid species which exist either in an uncharged or neutral zwitterionic form at physiological pH and thus are included within the group of lipids lacking an anionic function. Such lipids include, for example diacylphosphatidylcholine, diacylphosphatidylethanolamine, ceramide, sphingomyelin, dihydrosphingomyelin, cephalin, and cerebrosides. The selection of neutral lipids for use in the cochleate compositions described herein is generally guided by consideration of, e.g., cochleate size and stability. Lipids having a variety of acyl chain groups of varying chain length and degree of saturation are available or may be isolated or synthesized by well-known techniques. In one group of embodiments, lipids containing saturated fatty acids with carbon chain lengths in the range of C14 to C22 can be used. In another group of embodiments, lipids with mono or diunsaturated fatty acids with carbon chain lengths in the range of C14 to C22 can be used. Additionally, lipids having mixtures of saturated and unsaturated fatty acid chains can be used. In some embodiments, the neutral lipids used in the invention are DSPC, DPPC, POPC, or any related phosphatidylcholine. The neutral lipids useful in the invention may also be composed of sphingomyelin, dihydrosphingomyeline, or phospholipids with other head groups, such as serine and inositol.

It is noted that net neutral lipids can nevertheless contain head groups that contain an anionic functionality (*i.e.*, capable of ionically interacting with cations). Without wishing to be bound to any particular theory, it is believed that hydrophilic molecules or large molecules with hydrophilic domains, such as active pharmaceutical ingredients (APIs) of interest, can be formulated into cochleated in an unexpectedly enhanced manner by associating the API with a lipid domain that acts like a "raft" that remains intact and imbedded within the cochleate crystal matrix.
In other embodiments of the disclosure, "amphiphilic lipids" or "amphipathic lipids" are useful for the cochleate compositions described herein and refer to any suitable material, wherein the hydrophobic portion of the lipid material orients into a hydrophobic phase, while the hydrophilic portion orients toward the aqueous phase. In one embodiment, amphiphilic molecules have a polar water-soluble group attached to a water-insoluble hydrocarbon chain. Amphiphilic lipids are included with the group of lipids lacking an anionic function. Such compounds include, but are not limited to, phospholipids, aminolipids, and sphingolipids. Representative phospholipids include sphingomyelin, phosphatidylcholine, phosphatidylethanolamine, phosphatidylserine, phosphatidylinositol, phosphatidic acid, palmitoyloleoyl phosphatdylcholine, lysophosphatidylcholine, lysophosphatidylethanolamine, dipalmitoylphosphatidylcholine (DPPC), dioleoylphosphatidylcholine (DOPC), distearoylphosphatidylcholine (DSPC), or dilinoleylphosphatidylcholine. Other phosphorus-lacking compounds, such as sphingolipids, glycosphingolipid families, diacylglycerols, and beta-acyloxyacids, can also be used. Additionally, such amphiphilic lipids can be readily mixed with other lipids, such as triglycerides and sterols. The sterol component of the lipid mixture, when present, can be any of those sterols conventionally used in the field of liposome, lipid vesicle or lipid particle preparation. In one embodiment, the sterol is cholesterol.

Some amphiphilic lipids are negatively charged lipids that can be used in the present invention. As used herein, the term "negatively charged lipid" includes lipids having a head group bearing a formal negative charge in aqueous solution at an acidic, basic or physiological pH, and also includes lipids having a zwitterionic head group. Anionic lipids suitable for use in the present invention include, but are not limited to, phosphotidyl serine (PS), dioleoylphosphatidylserine (DOPS),
diacylphosphatidylserine.

Other amphiphilic lipids are positively charged lipids that can have cationic functionality (i.e., the capacity to ionically interact with anions). Exemplary cationic lipids, which carry a net positive charge at about physiological pH, include, but are not limited to, N,N-dioleyl-N,N-dimethylammonium chloride ("DODAC"); N-(2,3-dioleyloxy)propyl-N,N-N-triethylammonium chloride ("DOTMA"); N,N-distearyl-N,N-dimethylammonium bromide ("DDAB"); N-(2,3-dioleoyloxy)propyl)-N,N,N-trimethylammonium chloride ("DOTAP"); 1,2-Dioleyloxy-3-trimethylaminopropane chloride salt ("DOTAP.Cl"), 3.beta.-(N--(N',N'-dimethylaminoethane)-carbamoyl)cholesterol ("DC-Chol"), N-(1-(2,3-dioleyloxy)propyl)-N-2-(sperminecarboxamido)ethyl)-N,N-dimethyl-ammonium trifluoracetate ("DOSPA"), dioctadecylamidoglycyl carboxyspermine ("DOGS"), 1,2-dileoyl-sn-3-phosphoethanolamine ("DOPE"), 1,2-dioleoyl-3-dimethylammonium propane ("DODAP"), N,N-dimethyl-2,3-dioleyloxy)propylamine ("DODMA"), and N-(1,2-dimyristyloxyprop-3-yl)-N,N-dimethyl-N-hydroxyethyl ammonium bromide ("DMRIE"). Additionally, a number of commercial preparations of cationic lipids can be used, such as, e.g., LIPOFECTIN (including DOTMA and DOPE, available from GIBCO/BRL), and LIPOFECTAMINE (comprising DOSPA and DOPE, available from GIBCO/BRL). In some embodiments, a cationic lipid can be an amino lipid.
The lipid is a mixture of lipids, wherein the negatively charged lipid, wherein the negatively charged first lipid is phosphatidylserine, comprises at least 50% of the total lipid. The ratio of the negatively charged lipid to a neutral second lipid, wherein the neutral second lipid is phosphatidylcholine, is between 1:1 to 9:1.

The lipids used herein can include soy-based lipids. In some embodiments, the lipid includes phospholipids, such as soy-based phospholipids. The lipids can be natural or synthetic. For example, the lipid can include esterified fatty acid acyl chains, or organic chains attached by non-ester linkages such as ether linkages (as described in U.S. Pat. No. 5,956,159), disulfide linkages, and their analogs.

In one embodiment the lipid chains are from about 6 to about 26 carbon atoms, and the lipid chains can be saturated or unsaturated. Fatty acyl lipid chains useful in the present invention include, but are not limited to, n-tetradecanoic, n-hexadecanoic acid, n-octadecanoic acid, n-eicosanoic acid, n-docosanoic acid, n-tetracosanoic acid, n-hexacosanoic acid, cis-9-hexadecenoic acid, cis-9-octadecenoic acid, cis,cis-9,12-octadecedienoic acid, all-cis-9,12,15-octadecetrienoic acid, all-cis-5,8,11,14-eicosatetraenoic acid, all-cis-4,7,10,13,16,19-docosahexaenoic acid, 2,4,6,8-tetramethyl decanoic acid, and lactobacillic acid, and the like.

The cochleate compositions of the present invention can further include additional compounds known to be used in lipid preparations, e.g., pegylated lipid. Pegylated lipid includes lipids covalently linked to polymers of polyethylene glycol (PEG). PEG's are conventionally classified by their molecular weight, thus PEG 6,000 MW, e.g., has a molecular weight of about 6000. Adding pegylated lipid generally will result in an increase of the amount of compound (e.g., peptide, nucleotide, and nutrient) that can be incorporated into the cochleate. An exemplary pegylated lipid is dipalmitoylphosphatidylehtanolamine (DPPE) bearing PEG 5,000 MW.

### B. Biologically Relevant Molecules

The cochleates of the present invention are associated or "loaded" with a biologically relevant molecule, wherein the biologically relevant molecule is an aminoglycoside. A "biologically relevant molecule" is a molecule to be
encochleated, and generally does not refer to the lipid and ion used to precipitate the cochleate. Biologically relevant molecules include any compounds having a property of biological interest, e.g., ones that have a role in the life processes of a living organism. A biologically relevant molecule may be organic or inorganic, a monomer or a polymer, endogenous to a host organism or not, naturally occurring or synthesized in vitro and the like.

Non-limiting examples of biologically relevant molecules include vitamins, minerals, nutrients, micronutrients, amino acids, toxins, microbicides, microbistats, co-factors, enzymes, polypeptides, polypeptide aggregates, polynucleotides, lipids, carbohydrates, nucleotides, starches, pigments, fatty acids, saturated fatty acids, monounsaturated fatty acids, polyunsaturated fatty acids, flavorings, essential oils, extracts, hormones, cytokines, viruses, organelles, steroids and other multi-ring structures, saccharides, metals, metabolic poisons, antigens, imaging agents, porphyrins, tetrapyrrolic pigments, drugs and the like.

The biologically relevant molecule can be a diagnostic agent, such as an imaging agent. Imaging agents include nuclear agents and fluorescent probes, e.g., porphyrins. Porphyrins include tetrapyrrolic agents or pigments. One such tetrapyrrolic agent is Zinc Tetra-Phenyl Porphyrin (ZnTPP), which is a hydrophobic, fluorescent molecule that has high absorption in the visible spectrum (dark purple).

The polynucleotide can be one that is expressed to yield a biologically active polypeptide or polynucleotide. Thus, the polypeptide may serve as an immunogen or, for example, have enzymatic activity. The polynucleotide may have catalytic activity, for example, be a ribosome, or may serve as an inhibitor of transcription or translation, e.g., a small interfering RNA (siRNA) or an antisense molecule. The polynucleotide can be an antisense molecule including modified antisense molecule, such as a morpholino antisense molecule. The polynucleotide can be modified, e.g., it can be synthesized to have a morpholino backbone. If expressed, the polynucleotide preferably includes the necessary regulatory elements, such as a promoter, as known in the art. A specific example of a polypeptide is insulin.

The biologically relevant molecule can be an organic molecule that is hydrophobic in aqueous media. The biologically relevant molecule can also be a water-soluble monovalent or polyvalent cationic molecule, anionic, or net neutral at physiological pH.

The drug can be, but is not limited to, a protein, a small peptide, a bioactive polynucleotide, an antibiotic, an antiviral, an anesthetic, antipsychotic, an anti-infectious, an antifungal, an anticancer, an immunosuppressant, an immunostimulant, a steroidal anti-inflammatory, a non-steroidal anti-inflammatory, an antioxidant, an antidepressant which can be synthetically or naturally derived, a substance which supports or enhances mental function or inhibits mental deterioration, an anticonvulsant, an HIV protease inhibitor, a non-nucleophilic reverse transcriptase inhibitor, a cytokine, a tranquilizer, a mucolytic agent, a dilator, a vasoconstrictor, a decongestant, a leukotriene inhibitor, an anti-cholinergic, an anti-histamine, a cholesterol lipid metabolism modulating agent or a vasodilatory agent. The drug can also be any over the counter (non-prescription) medication.

An antifungal drug can be a polyene macrolide, tetraene macrolide, pentaenic macrolide, fluorinated pyrimidine, imidazole, azole, triazole, halogenated phenolic ether, thiocarbamate, allylamine, sterol inhibitor, and an agent that interpolates fungal cell wall components.

Nonsteroidal anti-inflammatory drugs (NSAIDS) are typically used to treat inflammation, muscle strains, and high fever. NSAIDS function by inhibiting. cyclooxygenase-1 (COX1) and cyclooxygenase-2 (COX2). COX1 enzymes are responsible for protecting the lining of the stomach and COX2 enzymes are responsible for the production of prostaglandins, which are important in the inflammatory process. Unfortunately, commercially available preparations of NSAIDS are active against both COX1 and COX2, and therefore have unwanted side effects such as ulcers, upset stomach or nausea.

Examples of suitable drugs include Amphotericin B, acyclovir, adriamycin, carbamazepine, ivermectin, melphalen, nifedipine, indomethacin, curcumin, aspirin, ibuprofen, naproxen, acetaminophen, rofecoxib, diclofenac, ketoprofen, meloxicam, nabumetone, estrogens, testosterones, steroids, phenyloin, ergotamines, cannabinoids, rapamycin, propanadid, propofol, alphadione, echinomycin, miconazole, miconazole nitrate, ketoconazole, itraconazole, fluconazole, griseofulvin, clotrimazole, econazole, terconazole, butoconazole, oxiconazole, sulconazole, saperconazole, voriconazole, ciclopirox olamine, haloprogin, tolnaftate, naftifine, terbinafine hydrochloride, morpholines, flucytosine, natamycin, butenafine, undecylenic acid, Whitefield's ointment, propionic acid, caprylic acid, clioquinol, selenium sulfide, teniposide, hexamethylmelamine, taxol, taxotere, 18-hydroxydeoxycorticosterone, prednisolone, dexamethasone, cortisone, hydrocortisone, piroxicam, diazepam, verapamil, vancomycin, tobramycin, teicoplanin, bleomycin, peptidolglycan, ristocetin, sialoglycoproteins, orienticin, avaporcin, helevecardin, galacardin, actinoidin, gentamycin, netilmicin, amikacin, kanamycin A, kanamycin B, neomycin, paromomycin, neamine, streptomycin, dihydrostreptomycin, apramycin, ribostamycin, spectinomycin, caspofungin, echinocandin B, aculeacin A, micafungin, anidulafungin, cilofungin, pneumocandin, geldanamycin, nystatin, rifampin, tyrphostin, a glucan synthesis inhibitor, vitamin A acid, mesalamine, risedronate, nitrofurantoin, dantrolene, etidronate, nicotine, amitriptyline, clomipramine, citalopram, dothiepin, doxepin, fluoxetine, imipramine, lofepramine, mirtazapine, nortriptyline, paroxetine, reboxetine, sertraline, trazodone, venlafaxine, dopamine, St. John's wort, phosphatidylserine, phosphatidic acid, amastatin, antipain, bestatin, benzamidine, chymostatin, 3,4-dichloroisocoumarin, elastatinal, leupeptin, pepstatin, 1,10-phenanthroline, phosphoramidon, ethosuximide, ethotoin, felbamate, fosphenytoin, lamotrigine, levitiracetam, mephenyloin, methsuximide, oxcatbazepine, phenobarbital, phensuximide, primidone, topirimate, trimethadione, zonisamide, saquinavir, ritonavir, indinavir, nelfinavir, and amprenavir.

The drug can be a polypeptide such as cyclosporin, Angiotensin I, II and III, enkephalins and their analogs, ACTH, anti-inflammatory peptides I, II, III, bradykinin, calcitonin, b-endorphin, dinorphin, leucokinin, leutinizing hormone releasing hormone (LHRH), insulin, neurokinins, somatostatin, substance P, thyroid releasing hormone (TRH) and vasopressin.

The drug can be an antigen, but is not limited to a protein antigen. The antigen can also be a carbohydrate or DNA. Examples of antigenic proteins include membrane proteins, carbohydrates, envelope glycoproteins from viruses, animal cell proteins, plant cell proteins, bacterial proteins, and parasitic proteins.

The antigen can be extracted from the source particle, cell, tissue, or organism by known methods. Biological activity of the antigen need not be maintained. However, in some instances (e.g., where a protein has membrane fusion or ligand binding activity or a complex conformation which is recognized by the immune system), it is desirable to maintain the biological activity. In these instances, an extraction buffer containing a detergent which does not destroy the biological activity of the membrane protein is used. Suitable detergents include ionic detergents such as cholate salts, deoxycholate salts and the like or heterogeneous polyoxyethylene detergents such as Tween, BRIG or Triton.

Utilization of this method allows reconstitution of antigens into the liposomes with retention of biological activities, and efficient association with the cochleates. The method may also be used without sonication, extreme pH, temperature, or pressure all of which may have an adverse effect upon efficient reconstitution of the antigen in a biologically active form.

Suitable nutrients include, but are not limited to lycopene, micronutrients such as phytochemicals or zoochemicals, vitamins, minerals, fatty acids, amino acids, fish oils, fish oil extracts, saccharides, herbal products and essential oils and flavor agents. Specific examples include vitamins A, B, B1, B2, B3, B12, B6, B-complex, C, D, E, and K, vitamin precursors, caroteniods, and beta-carotene, resveratrol, biotin, choline, inositol, gingko, lutein, zeaxanthine, quercetin, silibinin, perillyl alcohol, genistein, sulfurophane, and essential fatty acids, including eicosapentaenoic acid (EPA), gamma-3, omega-3, gamma-6 and omega-6 fatty acids, herbs, spices, and iron. Minerals include, but are not limited to boron, chromium, colloidal minerals, colloidal silver, copper, manganese, potassium, selenium, vanadium, vanadyl sulfate, calcium, magnesium, barium, iron and zinc.

As used herein, "micronutrient" is a nutrient that the body must obtain from outside sources. Generally micronutrients are essential to the body in small amounts.

The biologically relevant molecule can be a saccharide or sweetener, e.g., saccharine, isomalt, maltodextrin, aspartame, glucose, maltose, dextrose, fructose and sucrose. Flavor agents include oils, essential oils, or extracts, including but not limited to oils and extracts of cinnamon, vanilla, almond, peppermint, spearmint, chamomile, geranium, ginger, grapefruit, hyssop, jasmine, lavender, lemon, lemongrass, marjoram, lime, nutmeg, orange, rosemary, sage, rose, thyme, anise, basil, black pepper, tea or tea extracts, an herb, a citrus, a spice or a seed.

In some embodiments, the biologically relevant molecule can be a protonized biologically relevant molecule. In one embodiment, the biologically relevant molecule is a protonized weakly basic biologically relevant molecule. The pharmacokinetics of weakly basic biologically relevant molecules (e.g., vancomycin and tobramycin), conventionally has been dominated by their poor solubility in lipids such as milk. Additionally, biologically relevant molecules suitable for use in accordance with the present invention can include protonized weakly acidic biologically relevant molecules or protonized amphoteric biologically relevant molecules. Weakly acidic biologically relevant molecules or amphoteric biologically relevant molecules may or may not include an initial positive charge. Such biologically relevant molecules would also be rendered cationic by protonization. Protonizable biologically relevant molecules can be negatively charged, positively charged, uncharged or zwitterionic. In one embodiment, the protonized biologically relevant molecule is monovalent. In other embodiments, the protonized biologically relevant molecule is multivalent, e.g., divalent, trivalent, etc. In certain embodiments, a higher valency may be preferable due to the size and/or conformation of the biologically relevant molecule. In one embodiment, the protonized biologically relevant molecule is a protonized peptide, such as a protonized protein. In another embodiment, the protonized biologically relevant molecule is a protonized nucleotide. The protonized nucleotide can be, but is not limited to a protonized DNA, a protonized RNA, a protonized morpholino, a protonized siRNA molecule, a protonized ribozyme, a protonized antisense molecule, or a protonized plasmid.

The biologically relevant molecule is an aminoglycoside.

As used herein, the term "aminioglycoconjugates," refers to compounds that include an amino sugar or carbohydrate covalently linked with another molecule. Exemplary subgroups of aminoglycoconjugates include, but are not limited to, aminoglycoproteins, aminoglycosides, glycosaminoglycans, and aminoglycopeptides. "Aminoglycopeptides" are compounds that include an amino sugar or carbohydrate covalently linked to one or more peptides, including synthetic or chemically modified derivatives. Aminoglycopeptides include, but are not limited to vancomycin, teicoplanin, bleomycin, peptidolglycan, ristocetin, sialoglycoproteins, orienticin, avaporcin, helevecardin, galacardin, and actinoidin. Derivatives of these compounds also are included, e.g., those provided by reductive alkylation of reactive amines. See, Sundram et al., J. Org. Chem. 60:1102-03 (1995). U.S. Pat. Nos. 4,639,433,4,643,987, and 4,698,327, teach N-alkyl and N-acyl derivatives of vancomycin. European Patent Nos. 435 503A1 and 667 353 A1, described reductive alkylations of a variety of aminoglycopeptides including vancomycin and orienticin A. Similarly, "aminoglycosides" are compounds that include at least two amino sugars linked by glycoside bonds to a streptidine or a 2-deoxystreptamine or their analogs. Analogs are meant to include aminoglycosides modified, e.g., to increase resistance to enzyme cleavage. Such derivatives (e.g., amikacin, a semisynthetic derivative of kanamycin), arc necessary for treatment of individuals or populations that have built up resistance to other aminoglycosides, and all such derivatives developed presently or in the future are aminoglycosides that fall within the scope of the present invention. Analogs also are meant to include the structurally related aminocyclitols (e.g., spectinomycin). Aminoglycosides include, but are not limited to, gentamicin, netilmicin, tobramycin, amikacin, kanamycin A, kanamycin B, neomycin, paromomycin, neamine, streptomycin, dihydrostreptomycin, apramycin, ribostamycin, and spectinomycin. Aminoglycosides can optionally be grouped as streptomycins (e.g., streptomycin and dihydrostreptomycin), kanamycins (e.g., kanamycin, amikacin, tobramycin), gentamicins (e.g., gentamicin and netilmicin), and neomycins. Apramycin and specinomycin are aminoglycosides typically used by veterinarians to treat non-human animals.

In another embodiment of the disclosure, the biologically relevant molecule is an echinocandin. In a particularly preferred embodiment, the echinocandin is one or more of the following:
caspofungin, echinocandin B, aculeacin A, micafungin, anidulafungin, cilofungin, and
pneumocandin.

In some embodiments of the disclosure, the biologically relevant molecule is a nucleic acid, polypeptide, and/or an antibody.

For example, encochleation of antisense nucleic acid molecules, *i.e*., molecules which are complementary to a sense nucleic acid target, *e*.*g*., complementary to the coding strand of a double-stranded cDNA or mRNA molecule, can be useful. Accordingly, an antisense nucleic acid molecule can hydrogen bond to (*i*.*e*. anneal with) a sense nucleic acid target. The antisense nucleic acid can be complementary to an entire coding strand, or to only a portion thereof, *e*.*g*., all or part of the protein coding region (or open reading frame). An antisense nucleic acid molecule can also be antisense to all or part of a non-coding region of the coding strand of a nucleotide sequence encoding a polypeptide of the disclosure. The non-coding regions ("5' and 3' untranslated regions") are the 5' and 3' sequences which flank the coding region and are not translated into amino acids. An antisense oligonucleotide can be, for example, about 5, 10, 15, 20, 25, 30, 35, 40, 45, or 50 or more nucleotides in length. An antisense nucleic acid of the disclosure can be constructed using chemical synthesis and enzymatic ligation reactions using procedures known in the art. The antisense nucleic acid molecules of the disclosure are typically administered to a subject or generated *in situ* such that they hybridize with or bind to cellular mRNA and/or genomic DNA encoding a polypeptide corresponding to a selected marker of the disclosure to thereby inhibit expression of the marker, *e.g*., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. Examples of a route of administration of antisense nucleic acid molecules of the disclosure includes direct injection at a tissue site or infusion of the antisense nucleic acid into a blood- or bone marrow-associated body fluid. Alternatively, antisense nucleic acid molecules can be modified to target selected cells and then administered systemically. For example, for systemic administration, antisense molecules can be modified such that they specifically bind to receptors or antigens expressed on a selected cell surface, *e.g.*, by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

An antisense nucleic acid molecule of the disclosure can be an α-anomeric nucleic acid molecule. An α-anomeric nucleic acid molecule forms specific double-stranded hybrids with complementary RNA in which, contrary to the usual α-units, the strands run parallel to each other (Gaultier et al., 1987, Nucleic Acids Res. 15:6625-6641). The antisense nucleic acid molecule can also comprise a 2'-o-methylribonucleotide (Inoue et al., 1987, Nucleic Acids Res. 15:6131-6148) or a chimeric RNA-DNA analogue (Inoue et al., 1987, FEBS Lett. 215:327-330).

An "RNA interfering agent" as used herein, is defined as any agent which interferes with or inhibits expression of a target gene, *e*.*g*., a marker of the : disclosure, by RNA interference (RNAi). Such RNA interfering agents include, but are not limited to, nucleic acid molecules including RNA molecules which are homologous to the target gene, *e*.*g*., a marker of the disclosure, or a fragment thereof, short interfering RNA (siRNA), and small molecules which interfere with or inhibit expression of a target gene by RNA interference (RNAi).

"RNA interference (RNAi)" is an evolutionally conserved process whereby the expression or introduction of RNA of a sequence that is identical or highly similar to a target gene results in the sequence specific degradation or specific post-transcriptional gene silencing (PTGS) of messenger RNA (mRNA) transcribed from that targeted gene (*see* Coburn, G. and Cullen, B. (2002) J. of Virology 76(18):9225), thereby inhibiting expression of the target gene (see, *e.g*., U.S. Patent Application Nos: 20030153519A1; 20030167490A1; and U.S. Pat. Nos: 6,506,559; 6,573,099. In one embodiment, the RNA is double stranded RNA (dsRNA). This process has been described in plants, invertebrates, and mammalian cells. In nature, RNAi is initiated by the dsRNA-specific endonuclease Dicer, which promotes processive cleavage of long dsRNA into double-stranded fragments termed siRNAs. siRNAs are incorporated into a protein complex that recognizes and cleaves target mRNAs. RNAi can also be initiated by introducing nucleic acid molecules, *e*.*g*., synthetic siRNAs or RNA interfering agents, to inhibit or silence the expression of target genes. As used herein, "inhibition of target gene expression" or "inhibition of marker gene expression" includes any decrease in expression or protein activity or level of the target gene (*e.g*., a marker gene of the disclosure) or protein encoded by the target gene, *e.g.*, a marker protein of the disclosure. The decrease may be of at least 30%, 40%, 50%, 60%, 70%, 80%, 90%, 95% or 99% or more as compared to the expression of a target gene or the activity or level of the protein encoded by a target gene which has not been targeted by an RNA interfering agent.

The present disclosure also contemplates "short interfering RNA" (siRNA), also referred to herein as "small interfering RNA." Such a molecule is defined as an agent which functions to inhibit expression of a target gene, *e*.*g*., by RNAi. As used herein, the term siRNA is intended to be equivalent to any term in the art defined as a molecule capable of mediating sequence-specific RNAi. Such equivalents include, for example, double-stranded RNA (dsRNA), microRNA (mRNA), short hairpin RNA (shRNA), short interfering oligonucleotide, and post-transcriptional gene silencing RNA (ptgsRNA). An siRNA may be chemically synthesized, may be produced by *in vitro* transcription, or may be produced within a host cell. In one embodiment, siRNA. is a double stranded RNA (dsRNA) molecule of about 15 to about 40 nucleotides in length, preferably about 15 to about 28 nucleotides, more preferably about 19 to about 25 nucleotides in length, and more preferably about 19,20,21, or 22 nucleotides in length, and may contain a 3' and/or 5' overhang on each strand having a length of about 0, 1, 2, 3, 4, or 5 nucleotides. The length of the overhang is independent between the two strands, *i.e*., the length of the overhang on one strand is not dependent on the length of the overhang on the second strand. Preferably the siRNA is capable of promoting RNA interference through degradation or specific post-transcriptional gene silencing (PTGS) of the target messenger RNA (mRNA).

In another embodiment, an siRNA is a small hairpin (also called stem loop) RNA (shRNA). In one embodiment, these shRNAs are composed of a short (*e*.*g*., 19-25 nucleotide) antisense strand, followed by a 5-9 nucleotide loop, and the analogous sense strand. Alternatively, the sense strand may precede the nucleotide loop structure and the antisense strand may follow. These shRNAs may be contained in plasmids, retroviruses, and lentiviruses and expressed from, for example, the pol III U6 promoter, or another promoter (*see. e.g.*, Stewart, et al. (2003) RNA Apr; 9(4):493-501.
RNA interfering agents, *e.g.*, siRNA molecules, may be administered to a subject having or at risk for having cancer, to inhibit expression of a marker gene of the disclosure, *e*.*g*., a marker gene which is overexpressed in cancer (such as the markers listed in Table 3) and thereby treat, prevent, or inhibit cancer in the subject.

The present disclosure also encompasses ribozymes. Ribozymes are catalytic RNA molecules with ribonuclease activity which are capable of cleaving a single-stranded nucleic acid, such as an mRNA, to which they have a complementary region. Thus, ribozymes (*e.g.,* hammerhead ribozymes as described in Haselhoff and Gerlach, 1988, Nature 334:585-591) can be used to catalytically cleave mRNA transcripts to thereby inhibit translation of the protein encoded by the mRNA. A ribozyme having specificity for a nucleic acid molecule encoding a target polypeptide can be designed based upon the nucleotide sequence of a cDNA corresponding to the marker. For example, a derivative of a *Tetrahymena* L-19 IVS RNA can be constructed in which the nucleotide sequence of the active site is complementary to the nucleotide sequence to be cleaved (see Cech et al. U.S. Patent No. 4,987,071; and Cech et al. U.S. Patent No. 5,116,742). Alternatively, an mRNA encoding a polypeptide of the disclosure can be used to select a catalytic RNA having a specific ribonuclease activity from a pool of RNA molecules (see. *e.g.*, Bartel and Szostak, 1993, Science 261:1411-1418).

The present disclosure also encompasses nucleic acid molecules which form triple helical structures. For example, expression of a polypeptide of the disclosure can be inhibited by targeting nucleotide sequences complementary to the regulatory region of the gene encoding the polypeptide (*e*.*g*., the promoter and/or enhancer) to form triple helical structures that prevent transcription of the gene in target cells. See generally Helene (1991) Anticancer Drug Des. 6(6):569-84; Helene (1992) Ann. N.Y. Acad. Sci. 660:27-36; and Maher (1992) Bioassays 14(12):807-15.

In various embodiments, nucleic acid molecules can be modified at the base molecule, sugar molecule or phosphate backbone to improve, *e*.*g*., the stability, hybridization, or solubility of the molecule. For example, the deoxyribose phosphate backbone of the nucleic acid molecules can be modified to generate peptide nucleic acid molecules (see Hyrup et al., 1996, Bioorganic & Medicinal Chemistry 4(1): 5-23). As used herein, the terms "peptide nucleic acids" or "PNAs" refer to nucleic acid mimics, *e.g.*, DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup *et al.* (1996), *supra*; Perry-O'Keefe et al. (1996) Proc. Natl. Acad. Sci. USA 93: 14670-675.

PNAs can be used in therapeutic and diagnostic applications. For example, PNAs can be used as antisense or antigene agents for sequence-specific modulation of gene expression by, *e*.*g*., inducing transcription or translation arrest or inhibiting replication. PNAs can also be used, *e.g*., in the analysis of single base pair mutations in a gene by, *e.g*., PNA directed PCR clamping; as artificial restriction enzymes when used in combination with other enzymes, *e*.*g*., S1 nucleases (Hyrup (1996), *supra*; or as probes or primers for DNA sequence and hybridization (Hyrup, 1996, *supra*; Perry-O'Keefe et al., 1996, Proc. Natl. Acad. Sci. USA 93: 14670-675).

In another embodiment, PNAs can be modified, *e*.*g*., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras can be generated which can combine the advantageous properties of PNA and DNA. Such chimeras allow DNA recognition enzymes, *e*.*g*., RNASE H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup, 1996, *supra*). The synthesis of PNA-DNA chimeras can be performed as described in Hyrup (1996), *supra*, and Finn et al. (1996) Nucleic Acids Res. 24(17):3357-63. For example, a DNA chain can be synthesized on a solid support using standard phosphoramidite coupling chemistry and modified nucleoside analogs. Compounds such as 5'-(4-methoxytrityl)amino-5'-deoxy-thymidine phosphoramidite can be used as a link between the PNA and the 5' end of DNA (Mag et al., 1989, Nucleic Acids Res. 17:5973-88). PNA monomers are then coupled in a step-wise manner to produce a chimeric molecule with a 5' PNA segment and a 3' DNA segment (Finn et al., 1996, Nucleic Acids Res. 24(17):3357-63). Alternatively, chimeric molecules can be synthesized with a 5' DNA segment and a 3' PNA segment (Peterser et al., 1975, Bioorganic Med. Chem. Lett. 5:1119-11124).

In other embodiments, the oligonucleotide can include other appended groups such as peptides (*e*.*g*., for targeting host cell receptors *in vivo*), or agents facilitating transport across the cell membrane (see, *e.g*., Letsinger et al., 1989, Proc. Natl. Acad. Sci. USA 86:6553-6556; Lemaitre et al., 1987, Proc. Natl. Acad. Sci. USA 84:648-652; PCT Publication No. WO 88/09810) or the blood-brain barrier (see, *e.g*., PCT Publication No. WO 89/10134). In addition, oligonucleotides can be modified with hybridization-triggered cleavage agents (see, *e.g.*, Krol et al., 1988, Bio/Techniques 6:958-976) or intercalating agents (see, *e.g.,* Zon, 1988, Pharm. Res. 5:539-549). To this end, the oligonucleotide can be conjugated to another molecule, *e.g.*, a peptide, hybridization triggered cross-linking agent, transport agent, hybridization-triggered cleavage agent, *etc.*

The present disclosure also includes molecular beacon nucleic acid molecules having at least one region which is complementary to a nucleic acid molecule of the disclosure, such that the molecular beacon is useful for quantitating the presence of the nucleic acid molecule of the disclosure in a sample. A "molecular beacon" nucleic acid is a nucleic acid molecule comprising a pair of complementary regions and having a fluorophore and a fluorescent quencher associated therewith. The fluorophore and quencher are associated with different portions of the nucleic acid in such an orientation that when the complementary regions are annealed with one another, fluorescence of the fluorophore is quenched by the quencher. When the complementary regions of the nucleic acid molecules are not annealed with one another, fluorescence of the fluorophore is quenched to a lesser degree. Molecular beacon nucleic acid molecules are described, for example, in U.S. Patent 5,876,930.

In addition, aptamers are contemplated and can be produced using the methodology disclosed in a U.S. Pat. No. 5,270,163 and WO 91/19813.

The present disclosure also pertains to isolated proteins having a desired biological activity. Native polypeptides of interest can be isolated from cells or tissue sources by an appropriate purification scheme using standard protein purification techniques, produced by recombinant DNA techniques, or synthesized chemically using standard peptide synthesis methods. Typically, biologically active portions comprise a domain or motif with at least one activity of the corresponding protein. A biologically active portion of a protein can be a polypeptide which is, for example, 10, 25, 50, 100 or more amino acids in length. Moreover, other biologically active portions, in which other regions of the protein are deleted, can be prepared by recombinant techniques and evaluated for one or more of the functional activities of the native form of a polypeptide of the disclosure.

The disclosure also provides chimeric or fusion proteins. As used herein, a "chimeric protein" or "fusion protein" comprises all or part (preferably a biologically active part) of a polypeptide corresponding to a biomarker of the disclosure operably linked to a heterologous polypeptide (*i.e.,* a polypeptide other than the polypeptide corresponding to the biomarker). Within the fusion protein, the term "operably linked" is intended to indicate that the polypeptide of the disclosure and the heterologous polypeptide are fused in-frame to each other. The heterologous polypeptide can be fused to the amino-terminus or the carboxyl-terminus of the polypeptide of the disclosure. Such fusion proteins are well known in the art and include, for example, target proteins or polypeptides that enhance or inhibit the activity of such target proteins fused to heterologous signal sequences, peptide tags, immunoglobulin fusion proteins, and the like. Chimeric and fusion proteins of the disclosure can be produced by standard recombinant DNA techniques. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and re-amplified to generate a chimeric gene sequence (see, *e.g*., Ausubel *et al*., *supra*). Moreover, many expression vectors are commercially available that already encode a fusion molecule (*e.g*., a GST polypeptide). A nucleic acid encoding a polypeptide of the disclosure can be cloned into such an expression vector such that the fusion molecule is linked in-frame to the polypeptide of the invention.

Also contemplated are antibodies that bind to target molecule of interest or have a biological activity of interest (e.g., to appropriately enhance or inhibit the activity of a biological process). An isolated target polypeptide or a fragment thereof (or a nucleic acid encoding such a polypeptide) can be used as an immunogen to generate antibodies that bind to said immunogen, using standard techniques for polyclonal and monoclonal antibody preparation.

Unless otherwise specified herein, the terms "antibody" and "antibodies" broadly encompass naturally-occurring forms of antibodies (*e*.*g*. IgG, IgA, IgM, IgE) and recombinant antibodies such as single-chain antibodies, chimeric and humanized antibodies and multi-specific antibodies, as well as fragments and derivatives of all of the foregoing, which fragments and derivatives have at least an antigenic binding site. Antibody derivatives may comprise a protein or chemical molecule conjugated to an antibody.

The term "antibody" as used herein also includes an "antigen-binding portion" of an antibody (or simply "antibody portion"). The term "antigen-binding portion", as used herein, refers to one or more fragments of an antibody that retain the ability to specifically bind to a target antigen. It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent polypeptides (known as single chain Fv (scFv); see *e.g*., Bird et al. (1988) Science 242:423-426; and Huston et al. (1988) Proc. Natl. Acad. Sci. USA 85:5879-5883; and Osbourn et al. 1998, Nature Biotechnology 16: 778). Such single chain antibodies are also intended to be encompassed within the term "antigen-binding portion" of an antibody. Any VH and VL sequences of specific scFv can be linked to human immunoglobulin constant region cDNA or genomic sequences, in order to generate expression vectors encoding complete IgG polypeptides or other isotypes. VH and VL can also be used in the generation of Fab, Fv or other fragments of immunoglobulins using either protein chemistry or recombinant DNA technology. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g*., Holliger, P., et al. (1993) Proc. Natl. Acad. Sci. USA 90:6444-6448; Poljak, R. J., et al. (1994) Structure 2:1121-1123).

Still further, an antibody or antigen-binding portion thereof may be part of larger immunoadhesion polypeptides, formed by covalent or noncovalent association of the antibody or antibody portion with one or more other proteins or peptides. Examples of such immunoadhesion polypeptides include use of the streptavidin core region to make a tetrameric scFv polypeptide (Kipriyanov, S.M., et al. (1995) Human Antibodies and Hybridomas 6:93-101) and use of a cysteine residue, a marker peptide and a C-terminal polyhistidine tag to make bivalent and biotinylated scFv polypeptides (Kipriyanov, S.M., et al. (1994) Mol. Immunol. 31:1047-1058). Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion polypeptides can be obtained using standard recombinant DNA techniques, as described herein.

Antibodies may be polyclonal or monoclonal; xenogeneic, allogeneic, or syngeneic; or modified forms thereof (*e*.*g*., humanized, chimeric, etc.). Antibodies may also be fully human. Preferably, antibodies of the disclosure bind specifically or substantially specifically to pro-survival signaling pathway polypeptides of interest or fragments thereof. The terms "monoclonal antibodies" and "monoclonal antibody composition", as used herein, refer to a population of antibody polypeptides that contain only one species of an antigen binding site capable of immunoreacting with a particular epitope of an antigen, whereas the term "polyclonal antibodies" and "polyclonal antibody composition" refer to a population of antibody polypeptides that contain multiple species of antigen binding sites capable of interacting with a particular antigen. A monoclonal antibody composition typically displays a single binding affinity for a particular antigen with which it immunoreacts.

The cochleates of the present invention can be prepared with a wide range of biologically relevant molecule to lipid ratios. By way of example, the ratio of biologically relevant molecule to lipid can be between about 20,000:1 and about 0.5:1 by weight or any range in between. In one embodiment the ratio is about 1:1 by weight. In others the ratio is about 2:1, 3:1, 4:1, 5:1, 10:1, 20:1, 50:1, 100:1, 200:1, or 400:1 by weight or any range in between. All individual ranges and values between 20,000:1 and 0.5:1 are encompassed by the invention.

The cochleates of the present invention can optionally include one or more additional biologically relevant molecules, such as in combination therapies.

In one embodiment, the biologically relevant molecule is bound by an electrostatic, hydrophobic, covalent, or ionic interaction with a lipid component of the cochleate, such as a hydrophobic tail or bound to a component of the bilayer of the cochleate, e.g., a phospholipid or other lipid. Covalently binding the biologically relevant molecule to the lipid by cross-linking can be accomplished by known methods. Alternatively, the covalent bond is reversible so that the biologically relevant molecule can be detached from the lipid component or hydrophobic tail under suitable conditions. For example, a biologically relevant molecule can be attached to a phospholipid via a linker that can be cleaved by an enzyme endogenous to a target tissue, organ, or structure (e.g., a plasma protein, interstitial protein, an endosome or the intracellular milieu), such that the biologically relevant molecule is delivered to the target tissue, organ or other structure. In alternative embodiments, the biologically relevant molecule can be attached by any other means, for example, by electrostatic interactions and/or hydrophobic interactions.

In another embodiment, the biologically relevant molecule is bound by an electrostatic, hydrophobic, covalent, or ionic interaction with a a head group of the lipid component of the cochleate.

The biologically relevant molecule can be associated with the lipid component or hydrophobic tail or head group of the lipid component in any of the methods described herein. For example, in one embodiment, the biologically relevant molecule is associated with the lipid component, such that the biologically relevant molecule dissociates with the lipid component upon contact with a target environment. The biologically relevant molecule can be bound to a component of the cochleate with any of the linkers described herein, e.g., a linker that is reducible, or otherwise reversible or digestible by an enzyme, protein, or molecule endogenous to the target environment. The enzyme can be an extracellular, intracellular or endosomal enzyme endogenous to the subject. In another embodiment, the biologically relevant molecule component is electrostatically associated with the lipid component and dissociates with the cochleate upon contact with a pH gradient in a cell or organ of the subject.

### C. Aggregation Inhibitors

In some embodiments, the cochleates of the present invention can optionally include one or more aggregation inhibitors. The term "aggregation inhibitor," as used herein, refers to an agent that inhibits aggregation of cochleates. The aggregation inhibitor typically is present at least on the surface of the cochleate, and may only be present on the surface of the cochleate (e.g., when the aggregation inhibitor is introduced after cochleate formation). Aggregation inhibitors can be added before, after, or during cochleate formation. The type and/or amount of aggregation inhibitor can be adjusted to obtain a desired cochleate size and/or distribution. Additionally or alternatively, aggregation inhibitor(s) can be used to stabilize cochleate size and/or size distribution such that aggregation of cochleates is minimized or eliminated.

The formation of cochleates can be envisioned as a crystallization event that spontaneously occurs upon the interaction of charged lipids and oppositely charged multivalent cations. Modulating of the size of cochleate crystals formed, however, has prior to the present invention proved difficult.

In aqueous suspension, plain cochleates generally aggregate and upon long term storage form larger masses which can be several microns in size. Because of the association of the calcium with the lipid head group, the surfaces of cochleates have a hydrophobic character. When suspended in aqueous buffer, cochleate aggregation is a consequence of hydrophobic interactions, minimizing the amount of surface area exposed to water.

Such aggregation can be inhibited and even reversed, and individual cochleate particles can be stabilized by changing the surface properties of the cochleates and thereby inhibiting cochleate-cochleate interaction.

Aggregation can be inhibited by including in the liposome suspension a material that prevents liposome-liposome interaction at the time of calcium addition and thereafter. Alternatively, the aggregation inhibitor can be added after formation of cochleates. Additionally, the amount of aggregation inhibitor can be varied, thus allowing modulation of the particle size of the cochleates.

Such compositions are advantageous for several reasons including that smaller cochleates can allow for greater uptake by cells and rapid efficacy. Such a composition is suitable, e.g., when it is desired to rapidly and effectively deliver a biologically relevant molecule (e.g., an antifungal or antibacterial agent against a fungal or bacterial infection). Moreover, particle size can have a targeting affect in that some cells may take up particles of a certain size more or less effectively. Size may also affect the manner in which cochleates interact with a cell (e.g., fusion events or uptake).

Aggregation inhibitors work in part by modifying the surface characteristics of the cochleates such that aggregation is inhibited. Aggregation can be inhibited, for example, by steric bulk around the cochleate, which inhibits aggregation and/or changes the nature of the cochleate structure, e.g., a change in the surface hydrophobicity and/or surface charge.

The terms "coat," "coated," "coating," and the like, unless otherwise indicated, refer to an agent (e.g. an aggregation inhibitor) present at least on the outer surfaces of a cochleate. Such agents may be associated with the bilayer by incorporation of at least part of the agent into the bilayer, and/or may be otherwise associated, e.g., by ionic attraction to the cation or hydrophobic or ionic attraction to the lipid.

As discussed herein, cochleates can be formed by the calcium induced restructuring and fusion of lipid, e.g., phospholipid such as phosphatidylserine (PS). Due to the hydrophobic nature of the surfaces of cochleates in aqueous, calcium containing solutions, cochleates formed without the aggregation inhibitors of the invention can aggregate and form larger masses, e.g., needle-like structures in aspirin cochleates. Restricting and/or inhibiting the interaction of liposomes that can coalesce into cochleates at the time of cation addition limits the size of the resultant cochleate crystal, and prevents aggregation into larger particles. The addition of an aggregation inhibitor (e.g., casein) to liposomes prior to the addition of calcium results in stable non-aggregated nanocochleate structures.

The type and/or amount of aggregation inhibitor used can also determine the size of resulting cochleate. The presence of an aggregation inhibitor in differing concentrations also allows regulation of cochleate size distribution.

Addition of one or more aggregation inhibitors after formation of cochleates also inhibits and even reverses aggregation.

Suitable aggregation inhibitors that can be used in accordance with the present invention, include but are not limited to at least one of the following: casein, kappa-casein, milk, albumin, serum albumin, bovine serum albumin, rabbit serum albumin, methylcellulose, ethylcellulose, propylcellulose, hydroxycellulose, hydroxymethyl cellulose, hydroxyethyl cellulose, hydroxypropyl cellulose, hydroxypropylmethyl cellulose, polyvinyl pyrrolidone, carboxymethyl cellulose, carboxyethyl cellulose, pullulan, polyvinyl alcohol, sodium alginate, polyethylene glycol, polyethylene oxide, xanthan gum, tragacanth gum, guar gum, acacia gum, arabic gum, polyacrylic acid, methylmethacrylate copolymer, carboxyvinyl polymer, amylose, high amylose starch, hydroxypropylated high amylose starch, dextrin, pectin, chitin, chitosan, levan, elsinan, collagen, gelatin, zein, gluten, carrageenan, carnauba wax, shellac, latex polymers, milk protein isolate, soy protein isolate, whey protein isolate and mixtures thereof.

A preferred aggregation inhibitor is casein. Casein is a highly phosphorylated, calcium binding protein. Without wishing to be bound to any particular theory, it is believed that calcium mediates an interaction between negatively charged lipid (e.g., PS) and casein, thereby changing the surface properties of cochleates such that aggregation is inhibited. Another preferred aggregation inhibitor is milk and other milk products such as Half and Half, cream, etc. Preferred milk products also contain casein. Another preferred aggregation inhibitor is an excipient, e.g., methylcellulose. Other preferred aggregation inhibitors include albumin, serum albumin, bovine serum albumin and rabbit serum albumin.

More than one aggregation inhibitor may be used in the compositions of the invention. For example, both milk and methylcellulose may be used as an aggregation inhibitor.

In one embodiment, the cochleate compositions of the invention include between about 10% and about 0.1% aggregation inhibitor. Preferably, the aggregation inhibitor comprises about 1% of the cochleate composition. In another embodiment, the cochleate compositions of the invention include an aggregation inhibitor to lipid ratio of between about 0.1:1 to about 4:1 by weight. The aggregation inhibitor to lipid ratio can be about 1:1. A person of ordinary skill in the art will readily be able to determine the amount of aggregation inhibitor needed to form cochleates of the desired size with no more than routine experimentation.

In another embodiment, the aggregation inhibitor can be used in an amount to achieve cochleate compositions having a particle size relatively larger than that which can be achieved without or with other aggregation inhibitors (e.g., if more and/or a different aggregation inhibitor used). Such a composition can be useful, e.g., when delayed uptake and/or release of the biologically relevant molecule is desired, or when targeted cells or organs more effectively take up cochleates in the relatively larger size range. Such compositions also may have sustained activity (relative to smaller cochleate compositions) because it can take longer for the biologically relevant molecule to be released from a larger cochleate, e.g., if multiple fusion events are required.

In yet another embodiment, the amount and/or types of aggregation inhibitor can be chosen to manufacture a cochleate composition that has a wide particle size distribution such that the biologically relevant molecule is released over a period of time because smaller cochleates are rapidly taken up initially followed by take up or fusion events with increasingly larger cochleates. In addition, size may not only affect what type of cells take up the cochleate, but also how the cochleates interact with certain cells, e.g., size may effect whether a cochleate is taken up by a cell or undergoes one or more fusion events with a cell.

Moreover, in still another embodiment, several compositions can be combined for desired release profiles, e.g., a pulsed released, or combined release. For example, a rapid release nanocochleate composition can be mixed with a delayed-release larger size or even standard cochleate composition, such that an immediate and a delayed release are both realized. In an exemplary case, both small and large antibiotic cochleates are administered in order to treat a subject with a high initial dose (small cochleates) and to maintain enough antibiotic in the serum to be effective against remaining bacteria (large cochleates). In addition, the cochleate compositions may have different biologically relevant molecules, e.g., a stomach protecting medication can be formulated with nanocochleates for initial release (or a large distribution for long term release), and one or more non-steroidal anti-inflammatory drugs can be formulated with larger cochleates (NSAID) for release after the stomach protecting medication is released.

An aggregation inhibitor can also be used to stabilize particle size and particle size distribution. For example, it can be used to "lock-in" the cochleate size and distribution of standard cochleates and/or cochleates having an aggregation inhibitor. While the cochleate compositions of the present invention typically are stable over long periods of time, standard cochleates (cochleates formed without aggregation inhibitors) can tend to aggregate over time. Thus, standard cochleates can be reduced in size and/or stabilized by addition to such aggregation inhibitors, e.g., addition of methylcellulose after cochleate formation.

Cochleates formed in the presence of aggregation inhibitors have no or reduced aggregation relative to the absence of the aggregation inhibitor. Accordingly, such compositions are advantageous for several reasons including, e.g., greater uptake by cells, and increased efficacy. Cochleate compositions of the invention preferably have a mean diameter less than about 5, 4, 3, 2, or 1 micrometer. Cochleate compositions can have a mean diameter less than about 900 nm, 800 nm, 700 nm, 600 nm, 500 nm, 400 nm, 300 nm, 200 nm, or 100 nm. All individual values between these values (880, 435, 350), are meant to be included and are within the scope of the present invention. In another embodiment, cochleate compositions of the invention include cochleate populations having a mean diameter about equal to or greater than about 1 micrometer, e.g., 2, 3, 4, 5, 10, 50, or 100 micrometers. All individual values and ranges within these ranges are meant to be included and are within the scope of the present invention.

The size distribution can be narrow relative to that observed in standard cochleates (cochleates formed without aggregation inhibitors). The size distribution of cochleate compositions with aggregation inhibitors can be significantly improved relative to that observed in standard cochleate compositions. Preferably, the cochleates have a size distribution of less than about 30, 20, 10, 5, 3 or 1 µm, 900 nm, 800 nm, 700 nm, 600 nm, 500 nm, 400 nm, 300 nm, 200 nm, or 100 nm. All individual values between these values (550 nm, 420 nm, 475 nm, etc.) are meant to be included and are within the scope of the present invention. Such compositions are particularly desirable where uptake by macrophages is desired. It can readily be appreciated that particle size can be adjusted to a size suitable for uptake by desired organs or cells and/or unsuitable for uptake by organs or cells. In another embodiment, a wider size distribution of cochleates is used, e.g., about 10, 20, 50, 100, 200, 300, 400, 500, and larger micrometers. All individual values within these ranges are meant to be included and are within the scope of the present invention. Such compositions can be useful for long term release of biologically relevant molecules.

Additionally, as discussed above, combinations of cochleate populations with one or more biologically relevant molecules, one or more size distributions, and one or more mean diameter, to achieve a desired release pattern, e.g., pulsed release, delayed release and/or timed release of different biologically relevant molecules are contemplated.

### II. Methods of Making Cochleates

The invention provides methods for forming cochleates acording to claim 11. Any known method can be used to form cochleates, including but not limited to those described in U.S. Pat. Nos. 4,078,052; 5,643,574; 5,840,707; 5,994,318; 6,153,217; 6,592,894, as well as PCT Publ. Nos. WO 200404/091572; WO 2004/091578; WO 2005/110361 and U.S. Pat. Publ. 2010/0178325

In one embodiment, the method generally includes introducing a biologically relevant molecule to a lipid in the presence of a solvent, adding an aqueous solution to form liposomes, and precipitating the liposomes to form a cochleate. For example, the method generally includes introducing a biologically relevant molecule to a liposome in the presence of a solvent such that the biologically relevant molecule associates with the liposome, and precipitating the liposome to form a biologically relevant molecule cochleate.

The step of introducing a biologically relevant molecule to a liposome in the presence of a solvent can be achieved in a variety of ways, all of which are encompassed within the scope of the present invention. In one embodiment, the biologically relevant molecule is introduced by introducing a solution of the solvent and the biologically relevant molecule to the liposome. Preferably, the liposome is in a liposomal suspension, preferably, an aqueous liposomal suspension. In a preferred embodiment, the solution is introduced to the liposome by dropwise addition of the solution. In other embodiments, the solution can be added by continuous flow or as a bolus. In addition the solution may be introduced to dried lipid, with water added before, after or with the solution.

In another embodiment, the biologically relevant molecule is introduced to the liposome prior to or after the solvent. For example, the biologically relevant molecule may be introduced to a liposomal suspension that includes the solvent. The mixture can then be agitated, mixed, vortexed, etc. to facilitate association of the biologically relevant molecule with the liposome. The biologically relevant molecule introduced may be in a powder or a liquid form.

The liposome may be prepared by any known method of preparing liposomes. Thus, the liposomes may be prepared for example by solvent injection, lipid hydration, reverse evaporation, freeze drying by repeated freezing and thawing. The liposomes may be multilamellar (MLV) or unilamellar (ULV), including small unilamellar vesicles (SUV). The concentration of lipid in these liposomal solutions can be from about 0.1 mg/ml to 500 mg/ml. Preferably, the concentration of lipid is from about 0.5 mg/ml to about 50 mg/ml, more preferably from about 1 mg/ml to about 25 mg/ml.

The liposomes may be large unilamellar vesicles (LUV), stable plurilamellar vesicles (SPLV) or oligolamellar vesicles (OLV) prepared, e.g., by detergent removal using dialysis, column chromatography, bio beads SM-2, by reverse phase evaporation (REV), or by formation of intermediate size unilamellar vesicles by high pressure extrusion. Methods in Biochemical Analysis, 33:337 (1988). Liposomes made by all these and other methods known in the art can be used in practicing the present invention.

In one embodiment at least majority of the liposomes are unilamellar. The method can further include the step of filtering a liposomal suspension and/or mechanically extruding the suspension through a small aperture that includes both MLV and ULV liposomes, such that a majority of the liposomes are ULV. In preferred embodiments, at least 70%, 80%, 90% or 95% of the liposomes are ULV.

In another embodiment, the cochleate is formed by precipitation. The liposome can be precipitated with a multivalent cation to form a biologically relevant molecule-cochleate. The multivalent cation can consist entirely or consist essentially of a cationic metal, including, but not limited to calcium, magnesium, barium, zinc, and/or iron. Additionally or alternatively, the multivalent cation can include other multivalent cationic compounds. As used herein, the term "multivalent" refers to ions having a valency of at least 2, e.g., divalent, trivalent, etc.

Any suitable solvent can be used in connection with the present invention. Solvents suitable for a given application can be readily identified by a person of skill in the art. Preferably, the solvent is an FDA acceptable solvent.

The solvent can be an organic solvent or an inorganic solvent. In one embodiment, the solvent is a water miscible solvent. Suitable solvents include but are not limited to dimethylsulfoxide (DMSO), a methylpyrrolidone, N-methylpyrrolidone (NMP), acetonitrile, alcohols, e.g., ethanol (EtOH), dimethylformamide (DMF), tetrahydrofuran (THF), and combinations thereof. In general, the biologically relevant molecule concentration within the solvent is between about 0.01 mg/ml and 200 mg/ml. Preferably, the biologically relevant molecule concentration is between about 0.05 mg/ml and about 100 mg/ml, more preferably between about 0.1 mg/ml and 20 mg/ml.

The solvent can optionally be removed, e.g., before the formation of liposomes, at the liposome stage and/or after the cochleates are formed. Any known solvent removal method can be used. For example, solvent may be removed from the liposomal suspension by tangential flow and/or filtration and/or dialysis, or from the cochleates by washing, filtration, centrifugation, and/or dialysis. The cochleates can be washed, e.g., with buffer or water, optimally with calcium or another cation.

Utilizing the methods of the present invention, a wide range of lipid to biologically relevant molecule ratios can be achieved. Different ratios can have varying biological activity. The amount of biologically relevant molecule incorporated into the cochleates can be varied as desired. The optimal lipid:biologically relevant molecule ratio for a desired purpose can readily be determined. The cochleates can be administered to the targeted host to ascertain the nature and magnitude of the biological response to the administered cochleates. It is evident that the optimized ratio for any one use may range from a high ratio to a low ratio to obtain maximal amount of biologically relevant molecule in the cochleates. All ratios disclosed herein are w/w, unless otherwise indicated. In one embodiment, the ratio of lipid to biologically relevant molecule is between about 10,000:1 and 1000:1. Ratios in this range may be suitable when it is desired to administer small amounts of the molecule, (e.g., in the case of administration of radioactive agents or highly active, rare or expensive molecules). In another embodiment, the ratio is between about 8,000:1 and 4,000:1, e.g., about 6,000:1. Such a ratio may be suitable, e.g., in delivering porphyrins. In yet another embodiment, the ratio is between about 5,000:1 and 50:1. In yet another embodiment, the ratio of the lipid to the biologically relevant molecule is between about 20:1 and about 0.5:1. In another embodiment, the ratio of the lipid to the biologically relevant molecule is between about 1:1 and about 10:1. Such a ratio may be suitable, e.g., for delivery of an antifungal agent. In yet another embodiment, the ratio of lipid to the biologically relevant molecule is about 2:1, about 3:1, or between about 1.5:1 and 3.5:1. All individual values and ranges between about 0.25:1 and about 40,000:1 are within the scope of the invention. Further values also are within the scope of the invention. The cochleate formulations also can be prepared both with and without targeting molecules (e.g., fusogenic molecules, such as Sendai virus envelope polypeptides), to target specific cells and/or tissues.

In some embodiments, the biologically relevant molecule is hydrophobic. In others, it is amphipathic. In still others, it is hydrophilic and/or hydrosoluble.

Hydrophobic biologically relevant molecule encochleated within cochleates (e.g., beta-carotene cochleates) can be formed by introducing a hydrophobic biologically relevant molecule to a liposome in the presence of a solvent such that the hydrophobic biologically relevant molecule associates with the liposome, and precipitating the liposome to form a hydrophobic biologically relevant molecule-cochleate. In particularly preferred embodiments, the loading of biologically relevant molecule in the cochleate (e.g., hydrophobic, amphiphilic, hydrophilic, and/or hydrosoluble) is considerably higher than the loading observed when cochleates are formed using conventional methods, i.e., those described in U.S. Pat. No. 5,994,318.

Formation of the cochleate compositions of the present invention in the above methods involves crystallization of multivalent cation with negatively charged lipids. It is evident, therefore, that all of the parameters that govern crystallization, e.g., temperature, lipid concentration, multivalent cation concentrations, rate of cation addition, pH and rate of mixing, can be utilized to regulate cochleate formation. In certain embodiments, ionic conditions can be created or adjusted to affect the efficiency of the association and/or the encochleation of the biologically relevant molecule. For example, increasing the salt concentration in a liposomal suspension can render the environment less hospitable to a hydrophobic or amphipathic biologically relevant molecule, thereby increasing liposome and cochleate loading efficiency. Ionic conditions can also affect the ultimate structure of the cochleate generated. High loads of a biologically relevant molecule can also affect the highly ordered structure observed in cochleates formed, e.g., exclusively from calcium and phosphatidylserine. Additionally or alternatively, pH conditions can be created or adjusted to affect the loading and structure of the resulting cochleates. Such variations can readily be manipulated by the skilled practitioner using no more than the instant specification and routine experimentation. In addition, because a cochleate is highly thermodynamically stable, once a cochleate formulation method is developed for a given product, the end product can be made predictably and reliably.

Accordingly, in another aspect, the present invention provides methods of making anhydrous cochleates with protonized biologically relevant molecules. The method generally includes the step of contacting a negatively charged lipid with a protonized biologically relevant molecule and a divalent metal cation. Without wishing to be bound by any particular theory, it is believed that the negatively charged lipid forms an ionic interaction with the cationic protonized-biologically relevant molecule. The divalent metal cation then precipitates the lipid and protonized biologically relevant molecule to form an anhydrous cochleate. Such methods are well known in the art and are described, at least in U.S. Pat. Publ. 2007/0237814.

In another aspect, the present invention generally is directed to methods of making cochleates that include an aggregation inhibitor. The aggregation inhibitor can be introduced prior to, during or after formation of cochleates. That is, the aggregation inhibitor can be added to the lipid-biologically relevant molecule solution, to the liposomal solution or to the precipitated cochleate. For example, in one embodiment, the aggregation inhibitor is introduced to a liposomal suspension from which cochleates will subsequently be formed (e.g., by addition of cation or dialysis). That is, the aggregation inhibitor may be introduced prior to formation of liposomes, e.g., it may be added to dried lipid prior to suspension or added directly to a liposomal suspension, before of after addition of a biologically relevant molecule. In such embodiments, the cochleates may be initially formed in the desired size range and aggregation thereafter prevented by the presence of the aggregation inhibitor.

In other embodiments, the methods of the invention can include the step of introducing an aggregation inhibitor to a cochleate composition. For example, the method can further include forming cochleates (prior to introducing the aggregation inhibitor). The method can include providing cochleates already formed, e.g., cochleates obtained from a supplier. The method can further include the step of disaggregating cochleates by adding an aggregation inhibitor to aggregated cochleates.

In still other embodiments, aggregated cochleates may be disaggregated using alternative disaggregation methods, e.g., homogenization, and an aggregation inhibitor can be introduced in order to prevent reaggregation. In yet another embodiment, the aggregation inhibitor can be introduced during the formation of the cochleate, e.g., it can be added with the cation or during dialysis. In a preferred embodiment, the aggregation inhibitor is added in an amount suitable for modulating the resulting cochleate to the desired size.

The method can include forming cochleates with any or all of the optional ingredients disclosed herein. For example, the cochleates can include additional cationic compounds, protonized biologically relevant molecules, non-negative lipids, and/or aggregation inhibitors.

Any of the methods described herein can be utilized to produce anywhere from about 1 mg to about 500 g of cochleates in one batch. A smaller batch may be preferred in a laboratory setting where characterization of cochleates is desired. On the other hand, larger batches may be preferred in a manufacturing setting where mass production is desired. Preferably, larger batches are at least 50 g, and more preferably at least 75 g.

### III. Methods of the Present Invention

In yet another aspect, the present invention provides for both prophylactic and therapeutic methods of treating a subject at risk of (or susceptible to) a disorder or having a disorder which can be treated with one or more cochleate compositions described herein.

Many naturally occurring membrane fusion events involve the interaction of calcium with negatively charged phospholipids (e.g. PS and phosphatidylglycerol). Calcium-induced perturbations of membranes containing negatively charged lipids, and the subsequent membrane fusion events, are important mechanisms in many natural membrane fusion processes. Therefore, cochleates can be envisioned as membrane fusion intermediates.

A fusion event occurs between the outer layer of the cochleate and the cell membrane, resulting in the delivery of encochleated material into the cytoplasm of the target cell. As the calcium rich, highly ordered membrane of a cochleate first comes into close approximation to a natural membrane, a perturbation and reordering of the cell membrane is induced, resulting in a fusion event between the outer layer of the cochleate and the cell membrane. This fusion results in the delivery of a small amount of the encochleated material into the cytoplasm of the target cell. The cochleate can then break free of the cell and be available for another fusion event, either with the same or another cell.

Additionally or alternatively, particularly with active phagocytic cells, cochleates may be taken up by endocytosis and fuse from within the endocytic vesicle. Cochleates made with trace amounts of fluorescent lipids have been shown to bind and gradually transfer lipids to the plasma membrane and interior membranes of white blood cells in vitro.

Cochleates are useful for the delivery of a biologically relevant molecule to cultured cells, tissues or organisms by a variety of administration routes. The term "delivery," as used herein, refers to any means of bringing or transporting a biologically relevant molecule to a host, a food item, a formulation, a pharmaceutical composition, or any other system, wherein the biologically relevant molecule maintains at least a portion of its activity.

The cochleates can be coadministered with a further agent. The second agent can be delivered in the same cochleate preparation, in a separate cochleate preparation mixed with the cochleate preparation of the invention, separately in another form (e.g., capsules or pills), or in a carrier with the cochleate preparation. The cochleates can further include one or more additional biologically relevant molecules, such as other drugs, peptides, nucleotides (e.g., DNA and RNA), antigens, nutrients, flavors and/or proteins.

The cochleate compositions of the present invention also can include a reporter molecule for use in in vitro diagnostic assays, which can be a fluorophore, radiolabel or imaging agent. The cochleates can include molecules that direct binding of the cochleate to a specific cellular target, or promotes selective entry into a particular cell type.

One advantage of the cochleates of the present invention is the stability of the composition, especially for enhanced encochleation of hydrophilic APIs. Cochleates can be administered by any route, e.g., mucosal or systemic, without concern. Cochleates can be administered orally or by instillation without concern, as well as by the more traditional routes, such as oral, intranasal, intraoculate, intrarectal, intravaginal, intrapulmonary, topical, subcutaneous, intradermal, intramuscular, intravenous, transdermal, systemic, intrathecal (into CSF), and the like. Direct application to mucosal surfaces is an attractive delivery means made possible with cochleates. Delivery can be effected by, e.g., a nasal spray or nasal bath or irrigation.

Another advantage of the present invention is the ability to modulate cochleate size. Modulation of the size of cochleates and cochleate compositions changes the manner in which the biologically relevant molecule is taken up by cells. For example, in general, small cochleates are taken up quickly and efficiently into cells, whereas larger cochleates are taken up more slowly, but tend to retain efficacy for a longer period of time. Also, in some cases small cochleates are more effective than large cochleates in certain cells, while in other cells large cochleates are more effective than small cochleates.

Cochleates and cochleate compositions can also be administered to humans and non-human animals, such as dog, cats, and farm animals, in food or beverage preparations. Such compositions can be introduced to the food or beverage compositions by the manufacturer (e.g., to supplement food with nutrients), or by the consumer (e.g., where the cochleate composition is sold separately as a food additive). For example, nutrients and/or flavorings may be incorporated into dog or cat food, particularly where such nutrient and/or flavoring is fragile and normally decomposes or loses activity when exposed to oxygen and/or water. Cochleates may be added at any stage into the preparation of dog or cat food, as the cochleates are stable under extreme pressure and temperature conditions.

Another advantage of cochleates and cochleate compositions of the present invention is their ability to reduce a number of unwanted side effects. A number of drugs currently on the market cause gastrointestinal distress and often high circulating blood levels lead to toxicity in a number of vital organs. The ingestion of, e.g., aspirin may result in epigastric distress, nausea, and vomiting.

Another advantage of the present invention is that the cochleates can be formulated for uptake by particular cells or organs. Conventionally, high levels of drugs are often administered intravenously to obtain moderate levels at the sites of infection in order to combat opportunistic infections. This can cause undesirable side effects, for example, in the case of vancomycin, macular skin rashes, anaphylaxis, phlebitis and pain at the site of intravenous injection, chills, rash, and fever may occur. Also, rapid intravenous infusion may cause a variety of symptoms, including erythematous or urticarial reactions, flushing, tachycardia, and hypotension, generally non-permanent auditory impairment, ototoxicity associated with excessively high concentrations of the drug in plasma and less commonly, nephrotoxicity. By employing the cochleates of the present invention, toxicity levels can be lowered by decreasing the free drug in the circulating blood. Additionally, the biologically relevant molecule can be delivered directly to the site of infection, which can lower or eliminate the incidence of gastrointestinal distress.

For example, aminoglycosides are very poorly absorbed from the gastrointestinal tract. Less than 1% of the dose typically is absorbed following either oral or rectal administration. Also, inadequate concentrations of aminoglycosides are found in cerebrospinal fluid. Additionally, the drugs are not inactivated in the intestine, and are excreted relatively rapidly by the normal kidney, i.e., they are eliminated quantitatively in the feces. Long-term oral or rectal administration, however, may result in accumulation of aminoglycosides to toxic concentrations in patients with renal impairment. Instillation of these drugs into body cavities with serosal surfaces may result in rapid absorption and unexpected toxicity, i.e., neuromuscular blockade. Similarly, intoxication may occur when aminoglycosides are applied topically for long periods to large wounds, burns, or cutaneous ulcers, particularly if there is renal insufficiency.

Moreover, due to their polar nature, aminoglycosides largely are excluded from most cells, from the central nervous system, and from the eye. Concentrations of conventionally administered aminoglycosides in secretions and tissues are low. High concentrations, however, are found in the renal cortex and in the endolymph and perilymph of the inner ear; this is thought to contribute to the nephrotoxicity and ototoxicity caused by these drugs. Although they are widely used agents, serious toxicity is a major limitation to the usefulness of the aminoglycosides. Constant concentrations of drug in plasma above a critical level, which is manifested by elevated trough serum concentrations, correlate with toxicity in human beings. Aminoglycosides have the potential to produce reversible and irreversible vestibular, cochlear, and renal toxicity. These side effects complicate the use of these compounds and make their proper administration difficult.

The cochleates and cochleate compositions of the present invention can be administered to animals, including both human and non-human animals. It can be administered to animals, e.g., in animal feed or water. For example, antibiotic-cochleates of the present invention can be administered to poultry and other farm animals, including the ruminants and pigs, to control infection or to promote growth or milk production. Among a number of conditions which can be treated with these agents is enteritis, a disease which can cause severe economic losses to livestock producers. Enteritis occurs in chickens, swine, cattle and sheep and is attributed mainly to anaerobic bacteria, particularly Clostridium perfungens. Enterotoxemia in ruminants, an example of which is "overeating disease" in sheep, is a condition caused by C. perfungens infection. The treatment of such conditions is therefore also encompassed within the methods of the present invention.

### a. Prophylactic Methods

In one aspect, the present invention provides a method for preventing in a subject, a disease or disorder which can be treated with at least one biologically relevant molecule, e.g., a protein, a small peptide, an antiviral, an anesthetic, an anti-infectious, an antifungal, an anticancer, an immunosuppressant, a steroidal anti-inflammatory, a non-steroidal anti-inflammatory, a tranquilizer, a mucolytic agent, a dilator, a vasoconstrictor, a decongestant, a leukotriene inhibitor, an anti-cholinergic, an anti-histamine or a vasodilatory agent. Subjects at risk for a disease or condition which can be treated with the agents mentioned herein can be identified by, for example, any or a combination of diagnostic or prognostic assays known to those skilled in the art. Administration of a prophylactic agent can occur prior to the manifestation of symptoms characteristic of the disease or disorder, such that the disease or disorder is prevented or, alternatively, delayed in its progression.

### b. Therapeutic Methods

In another aspect, the present invention provides a method of administering a cochleate composition for therapeutic purposes. In one embodiment, the present invention provides a method for treating a subject that would benefit from administration of a composition of the present invention. Any therapeutic indication that would benefit from a cochleate composition of the invention can be treated by the methods of the present invention. The present invention provides methods of treating a subject at risk for or having a disease or disorder that can be treated with one or more biologically relevant molecule. The method includes the step of administering to the subject a composition of the invention, such that the disease or disorder is prevented, ameliorated, terminated or delayed in its progression. The disease or disorder can be any of the diseases or disorders discussed herein.

The compositions of the invention can be administered to a subject alone or in combination with a second therapy as described above. The compositions of the invention can be administered to a subject prior to, at the same time, or after a second therapy is administered.

Therapeutic agents can be tested in an appropriate animal model. For example, cochleate compositions of the present invention can be used in an animal model to determine the efficacy, toxicity, or side effects of treatment with said agent. Alternatively, a therapeutic agent can be used in an animal model to determine the mechanism of action of such an agent. For example, an agent can be used in an animal model to determine the efficacy, toxicity, or side effects of treatment with such an agent. Alternatively, an agent can be used in an animal model to determine the mechanism of action of such an agent.

As used herein, the term "treatment" or "treating," is defined as the application or administration of a therapeutic agent (e.g., antibiotics encochleated by cochleate compositions of the present invention) to a patient, or application or administration of a therapeutic agent to an isolated tissue or cell line from a patient, who has a disease or disorder, a symptom of disease or disorder or a predisposition toward a disease or disorder, with the purpose to cure, heal, alleviate, relieve, alter, remedy, ameliorate, improve or affect the disease or disorder, the symptoms of the disease or disorder, or the predisposition toward disease or disorder. The term, "treated," as used herein, refers to the disease or disorder being cured, healed, alleviated, relieved, altered, remedied, ameliorated improved or affected. For example, certain methods of treatment of the instant invention provide for administration of anti-inflammatory cochleates, such that inflammation is lessened or alleviated. Other methods of treatment of the instant invention include the administration of antifungal cochleates, such that fungal infection is relieved or remedied.

The terms "cure," "heal," "alleviate," "relieve," "alter," "remedy," "ameliorate," "improve" and "affect" are evaluated in terms of a suitable or appropriate control. A "suitable control" or "appropriate control" is any control or standard familiar to one of ordinary skill in the art useful for comparison purposes. In one embodiment, a "suitable control" or "appropriate control" is a value, level, feature, characteristic, property, etc. determined prior to administration of a cochleate encochleating a biologically relevant molecule, as described herein. For example, the number of colony forming units can be determined prior to administering a cochleate composition of the present invention to a host. In another embodiment, a "suitable control" or "appropriate control" is a value, level, feature, characteristic, property, etc. determined in a subject, e.g., a control or normal subject exhibiting, for example, normal traits. In yet another embodiment, a "suitable control" or "appropriate control" is a predefined value, level, feature, characteristic, property, etc.

The methods of the present invention include methods of administering a biologically relevant molecule to a host, wherein the biologically relevant molecule is associated with a cochleate or cochleate composition of the invention. The cochleates and cochleate compositions of the present invention may be administered orally, nasally, topically, intravenously, transdermally, buccally, sublingually, rectally, vaginally or parenterally.

The present invention provides a method for treating a subject that would benefit from administration of a composition of the present invention. Any therapeutic indication that would benefit from a biologically relevant molecule, e.g., a drug or nutrient, can be treated by the methods of the invention. Accordingly, the present invention provides methods of treating a subject at risk for or having a disease or disorder which can be treated with, for example, a protein, a small peptide, a bioactive polynucleotide, an antibiotic, an antiviral, an anesthetic, antipsychotic, an anti-infectious, an antifungal, an anticancer, a immunosuppressant, an immunostimulant, a steroidal anti-inflammatory, a non-steroidal anti-inflammatory, an antioxidant, an antidepressant which can be synthetically or naturally derived, a substance which supports or enhances mental function or inhibits mental deterioration, an anticonvulsant, an HIV protease inhibitor, a non-nucleophilic reverse transcriptase inhibitor, a cytokine, a tranquilizer, a mucolytic agent, a dilator, a vasoconstrictor, a decongestant, a leukotriene inhibitor, an anti-cholinergic, an anti-histamine, a cholesterol lipid metabolism modulating agent or a vasodilatory agent. The method includes the step of administering to the subject a cochleate composition of the invention, such that the disease or disorder is treated. The disease or disorder can be, e.g., inflammation, pain, infection, fungal infection, bacterial infection, viral infection, parasitic disorders, an immune disorder, genetic disorders, degenerative disorders, cancer, proliferative disorders, obesity, depression, hair loss, impotence, hypertension, hypotension, dementia, senile dementia, or malnutrition, acute and chronic leukemia and lymphoma, sarcoma, adenoma, carcinomas, epithelial cancers, small cell lung cancer, non-small cell lung cancer, prostate cancer, breast cancer, pancreatic cancer, hepatocellular carcinoma, renal cell carcinoma, biliary cancer, colorectal cancer, ovarian cancer, uterine cancer, melanoma, cervical cancer, testicular cancer, esophageal cancer, gastric cancer, mesothelioma, glioma, glioblastoma, pituitary adenomas, schizophrenia, obsessive compulsive disorder (OCD), bipolar disorder, Alzheimer's disease, Parkinson's disease, cell proliferative disorders, blood coagulation disorders, Dysfibrinogenaemia and hemophilia (A and B), autoimmune disorders, e.g., systemic lupus erythematosis, multiple sclerosis, myasthenia gravis, autoimmune hemolytic anemia, autoimmune thrombocytopenia, Grave's disease, allogenic transplant rejection, ankylosing spondylitis, psoriasis, scleroderma, uveitis, eczema, dermatological disorders, hyperlipidemia, hyperglycemia, and hypercholesterolemia.

Cochleate compositions of the present invention can also be used to promote greater health or quality of life, for example limit cholesterol uptake or regulate lipid metabolism, weight gain, hunger, aging, or growth. Cosmetic effects such as wrinkle reduction, hair growth, pigmentation, or dermatologic disorders may also be treated. Cochleates may also treat hereditary disease such as cystic fibrosis or muscular dystrophy.

The cochleate compositions of the present invention can be used to treat a variety of inflammations, including headache, arthritis, rheumatoid arthritis, osteoarthritis, atherosclerosis, acute gout, acute or chronic soft tissue damage associated with, e.g., a sports injury, tennis elbow, bursitis, tendonitis, acute or chronic back pain, such as a herniated disc, carpal tunnel syndrome, glomerulonephritis, carditis, ulcerative colitis, asthma, sepsis, and plantar fasciitis. The cochleate compositions of the present invention can also be used to relieve pain resulting from surgery or other medical procedure. The cochleate compositions of the present invention can further be used to treat a variety of fungal infections, including candida, e.g., yeast infection, tinea, e.g., Athlete's foot, pityriasis, thrush, cryptococcal meningitis, histoplasmosis, and blastomycosis.

The cochleate compositions of the present invention can also be used to treat a variety of bacterial infections, including but not limited to moderate to severe lower respiratory tract infections, skin infections, biliary tract infections, bone infections, antibiotic prophylaxis, pseudomembranous enterocolitis, central nervous system infections (e.g., meningitis and ventriculitis), intra-abdominal infections (e.g., peritonitis), pneumonia, septicemia, soft tissue infections, neutropenic sepsis, joint infections, infective endocartidis, and urinary tract infections.

Exemplary bacteria that can be treated with the antibiotic preparation of the present invention include, but are not limited to, Mycobacteria such as Mycobacterium avium, Staphylococcus aureus, Staphylococcus epidermidis, Streptococcus pyogenes, Streptococcus pneumuoniae, Streptococcus Group D, Clostridium perfungens, Haemophilus influenzae, Escherichia coli, Pseudomonas aeruginosa, and Klebsiella pneumonia.

Because intracellular residence of infectious agents can complicate treatment, a complete cure can require the eradication of all intracellular bacteria. Therefore, a therapeutic approach that increases the intracellular antibiotic concentration may enhance the bactericidal killing and ensure complete elimination of infection.

In a preferred embodiment, antibacterial cochleates of the present invention have the ability to reduce the number of bacterial colonies by at least 10%. More preferably, antibacterial cochleates can reduce the number of bacterial colonies by at least 25% and even more preferably by 50%, 75%, 85%, 95%, 96%, 97%, 98%, 99%, 99.5%, 99.9%, or 100%. All individual values and ranges falling between these ranges and values are within the scope of the present invention.

The present invention also provides a means for treating a variety of fungal infections, including, but not limited to, asthma, chronic rhinosinusitis, allergic fungal sinusitis, sinus mycetoma, non-invasive fungus induced mucositis, non-invasive fungus induced intestinal mucositis, chronic otitis media, chronic colitis, inflammatory bowel diseases, ulcerative colitis, Crohn's disease, candidemia, intraabdominal abscesses, peritonitis, pleural space infections, esophageal candidiasis and invasive aspergillosis. Exemplary fungi that can be treated using antifungal cochleate compositions of the present invention include, without limitation, Absidia, Aspergillus flavus, Aspergillus fumigatus, Aspergillus glaucus, Aspergillus nidulans, Aspergillus terreus, Aspergillus versicolor, Alternaria, Basidiobolus, Bipolaris, Candida albicans, Candida glabrata, Candida guilliermondii, Candida krusei, Candida lypolytica, Candida parapsilosis, Candida tropicalis, Cladosporium, Conidiobolus, Cunninahamella, Curvularia, Dreschlera, Exserohilum, Fusarium, Malbranchia, Paecilonvces, Penicillium, Pseudallescheria, Rhizopus, Schizophylum, Sporothrix, Acremonium, Arachniotus citrinus, Aurobasidioum, Beauveria, Chaetomium, Chryosporium, Epicoccum, Exophilia jeanselmei, Geotrichum, Oidiodendron, Phoma, Pithomyces, Rhinocladiella, Rhodoturula, Sagrahamala, Scolebasidium, Scopulariopsis, Ustilago, Trichoderma, and Zygomycete.

In a preferred embodiment, antifungal-cochleates of the present invention have the ability to reduce fungal colony forming units (CFU's) by at least 10%. More preferably, echinocandin cochleates can reduce CFU's by at least 25% and even more preferably by 50%, 75%, 85%, 95%, 96%, 97%, 98%, 99%, 99.5%, 99.9%, or 100%. All individual values and ranges falling between these ranges and values are within the scope of the present invention. Reduction in colony forming units may be in vivo or in vitro. The host of the fungal infection can be a human or non-human animal.

The above methods can be used in the absence of other treatment, or in combination with other treatments. Such treatments can be started prior to, concurrent with, or after the administration of the compositions of the instant invention. Accordingly, the methods of the invention can further include the step of administering a second treatment, such as for example, a second treatment for the disease or disorder or to ameliorate side effects of other treatments. Such second treatment can include, e.g., radiation, chemotherapy, transfusion, operations (e.g., excision to remove tumors), and gene therapy. Additionally or alternatively, further treatment can include administration of drugs to further treat the disease or to treat a side effect of the disease or other treatments (e.g., anti-nausea drugs).

With regard to both prophylactic and therapeutic methods of treatment, such treatments may be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and on the market.

More specifically, the term refers the study of how a patient's genes determine his or her response to a drug (e.g., a patient's "drug response phenotype", or "drug response genotype"). Thus, another aspect of the invention provides methods for tailoring an individual's prophylactic or therapeutic treatment according to that individual's drug response genotype. Pharmacogenomics allows a clinician or physician to target prophylactic or therapeutic treatments to patients who will most benefit from the treatment and to avoid treatment of patients who will experience toxic drug-related side effects.

The language "therapeutically effective amount" is that amount necessary or sufficient to produce the desired physiologic response. The effective amount may vary depending on such factors as the size and weight of the subject, or the particular compound. The effective amount may be determined through consideration of the toxicity and therapeutic efficacy of the compounds by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it may be expressed as the ratio LD₅₀/ED₅₀. Compounds which exhibit large therapeutic indices are preferred. While compounds that exhibit toxic side effects may be used, care should be taken to design a delivery system that targets such compounds to the site of affected tissue in order to minimize potential damage to unaffected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form used and the route of administration utilized. For any composition used in the method of the invention, the therapeutically effective dose can be estimated initially from cell culture assays. A dose may be formulated in animal models to achieve a circulating plasma concentration range that includes the EC₅₀ (i.e., the concentration of the test composition that achieves a half-maximal response) as determined in cell culture. Such information can be used to more accurately determine useful doses in humans. Levels in plasma may be measured, for example, by high performance liquid chromatography.

### IV. Pharmaceutical Compositions

The invention pertains to uses of the cochleate compositions of the invention for prophylactic and therapeutic treatments as described herein. Accordingly, the compounds of the present invention can be incorporated into pharmaceutical compositions suitable for administration. Such compositions typically comprise the compositions of the invention and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art.

Wetting agents, emulsifiers and lubricants, such as sodium lauryl sulfate and magnesium stearate, as well as coloring agents, release agents, sweetening, flavoring and perfuming agents, preservatives and antioxidants may also be present in the compositions.

Examples of pharmaceutically acceptable antioxidants, which may also be present in formulations of therapeutic compounds of the invention, include water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal chelating agents, such as citric acid, ethylenediamine tetraacetic acid (EDTA), sorbitol, tartaric acid, phosphoric acid, and the like.

Furthermore, the present invention can further include one or more additional agents, including water, antimicrobial agents, plasticizing agents, flavoring agents, surfactants, stabilizing agents, emulsifying agents, thickening agents, binding agents, coloring agents, sweeteners, fragrances, and the like.

Suitable antimicrobial agents include triclosan, cetyl pyridium chloride, domiphen bromide, quaternary ammonium salts, zinc compounds, sanguinarine, fluorides, alexidine, octonidine, EDTA, and essential oils such as thymol, methyl salicylate, menthol and eucalyptol.

Suitable plasticizing agents include, for example, polyols such as sugars, sugar alcohols, or polyethylene glycols (PEGs), urea; glycol, propylene glycol, triethyl citrate, dibutyl or dimethyl phthalate, monoacetin, diacetin or triacetin.

Suitable surfactants include pluronic acid, sodium lauryl sulfate, mono and diglycerides of fatty acids and polyoxyethylene sorbitol esters, such as, Atmos 300 and Polysorbate 80. Suitable stabilizing agents include xanthan gum, locust bean gum, guar gum, and carrageenan. Suitable emulsifying agents include triethanolamine stearate, quaternary ammonium compounds, acacia, gelatin, lecithin, bentonite, veegum, and the like. Suitable thickening agents include methylcellulose, carboxyl methylcellulose, and the like. Suitable binding agents include starch.

Suitable sweeteners that can be included are those well known in the art, including both natural and artificial sweeteners. Suitable sweeteners include water-soluble sweetening agents such as monosaccharides, disaccharides and polysaccharides; water-soluble artificial sweeteners such as soluble saccharin salts, cyclamate salts, or the free acid form of saccharin, and the like; dipeptide based sweeteners, such as L-aspartic acid derived sweeteners; water-soluble sweeteners derived from naturally occurring water-soluble sweeteners, such as a chlorinated derivative of ordinary sugar (sucrose), known, under the product description of sucralose; and protein based sweeteners such as thaumatoccous danielli (Thaumatin I and II).

In general, an effective amount of auxiliary sweetener is utilized to provide the level of sweetness desired for a particular composition, and this amount will vary with the sweetener selected. This amount will normally be 0.01% to about 10% by weight of the composition when using an easily extractable sweetener.

The flavorings that can be used include those known to the skilled artisan, such as natural and artificial flavors. These flavorings may be chosen from synthetic flavor oils and flavoring aromatics, and/or oils, oleo resins and extracts derived from plants, leaves, flowers, fruits and so forth, and combinations thereof. Representative flavor oils include: spearmint oil, cinnamon oil, peppermint oil, clove oil, bay oil, thyme oil, cedar leaf oil, oil of nutmeg, oil of sage, and oil of bitter almonds. Also useful are artificial, natural or synthetic fruit flavors such as vanilla, chocolate, coffee, cocoa and citrus oil, and fruit essences. These flavorings can be used individually or in admixture. Flavorings such as aldehydes and esters including cinnamyl acetate, cinnamaldehyde, citral, diethylacetal, dihydrocarvyl acetate, eugenyl formate, p-methylanisole, and so forth may also be used. Generally, any flavoring or food additive, such as those described in Chemicals Used in Food Processing, publication 1274 by the National Academy of Sciences, pages 63-258, may be used.

The amount of flavoring used is normally a matter of preference subject to such factors as flavor type, individual flavor, and strength desired. Thus, the amount may be varied in order to obtain the result desired in the final product. Such variations are within the capabilities of those skilled in the art without the need for undue experimentation.

The compositions of the present invention can also contain coloring agents or colorants. The coloring agents are used in amounts' effective to produce the desired color. The coloring agents useful in the present invention include pigments such as titanium dioxide, which may be incorporated in amounts of up to about 5 wt %, and preferably less than about 1 wt %. Colorants can also include natural food colors and dyes suitable for food, drug and cosmetic applications. These colorants are known as FD&C dyes and lakes. A full recitation of all FD&C and D&C dyes and their corresponding chemical structures may be found in the Kirk-Othmer Encyclopedia of Chemical Technology, Volume 5, Pages 857-884.

Formulations of the present invention include those suitable for oral, nasal, topical, transdermal, buccal, sublingual, rectal, vaginal or parenteral administration. The formulations may conveniently be presented in unit dosage form and may be prepared by any methods well known in the art of pharmacy. The amount of active ingredient which may be combined with a carrier material to produce a single dosage form will generally be that amount of the composition which produces a therapeutic effect. Generally, out of 100%, this amount will range from about 1% to about 99% of active ingredient preferably from about 5% to about 70%, most preferably from about 10% to about 30%.

Methods of preparing these formulations or compositions include the step of bringing into association a composition of the present invention with the carrier and, optionally, one or more accessory ingredients. In general, the formulations are prepared by uniformly and intimately bringing into association a composition of the present invention with liquid carriers, or finely divided solid carriers, or both, and then if necessary, shaping the product.

Formulations of the invention suitable for oral administration may be in the form of capsules, cachets, pills, tablets, gelcaps, crystalline substances, lozenges (using a flavored basis, usually sucrose and acacia or tragacanth), powders, granules, gel, partial liquid, spray, nebulae, mist, atomized vapor, aerosol, tincture, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) or as mouth washes and the like, each containing a predetermined amount of a composition of the present invention as an active ingredient. A composition of the present invention may also be administered as a bolus, electuary, or paste.

In solid dosage forms of the invention for oral administration (capsules, tablets, pills, dragees, powders, granules, and the like), the active ingredient is mixed with one or more pharmaceutically acceptable carriers, such as sodium citrate or dicalcium phosphate, or any of the following: fillers or extenders, such as starches, lactose, sucrose, glucose, mannitol, or silicic acid; binders, such as, for example, carboxymethylcellulose, alginates, gelatin, polyvinyl pyrrolidone, sucrose or acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol and glycerol monostearate; absorbents, such as kaolin and bentonite clay; lubricants, such a talc, calcium stearate, magnesium stearate, solid polyethylene glycols, sodium lauryl-sulfate, and mixtures thereof; and coloring agents.

In the case of capsules, tablets and pills, the pharmaceutical compositions may also comprise buffering agents. Solid compositions of a similar type may also be used as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing-agent. Molded tablets may be made by molding in a suitable machine a mixture of the powdered composition moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes or microspheres.

They may be sterilized by, for example, filtration through a bacteria-retaining filter, or by incorporating sterilizing agents in the form of sterile solid compositions which may be dissolved in sterile water, or some other sterile injectable medium immediately before use.

These compositions may also optionally contain opacifying agents and may be of a composition that they release the active ingredient(s) only, or preferentially, in a certain portion of the gastrointestinal tract, optionally, in a delayed manner. Examples of embedding compositions which may be used include polymeric substances and waxes. The active ingredient may also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert dilutents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof. Besides inert dilutents, the oral compositions may also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented in liquid or aerosol form, or as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound. Liquid or aerosol forms include, but are not limited to, gels, pastes, ointments, salves, creams, solutions, suspensions, partial liquids, sprays, nebulaes, mists, atomized vapors, and tinctures. Formulations of the present invention which are suitable for vaginal administration also include pessaries, tampons, creams, gels, pastes, foams or spray formulations containing such carriers as are known in the art to be appropriate.

Formulations of the pharmaceutical compositions of the invention for nasal administration can be in solid, liquid, or aerosol form (e.g., powder, crystalline substance, gel, paste, ointment, salve, cream, solution, suspension, partial liquid, spray, nebulae, irrigant, wash, mist, atomized vapor or tincture).

Dosage forms for the topical or transdermal administration of a composition of the present invention include powders, sprays, ointments, pastes, creams, lotions, gels, solutions, patches and inhalants. The composition may be mixed under sterile conditions with a pharmaceutically acceptable carrier, and with any preservatives, buffers, or propellants which may be required.

The ointments, pastes, creams and gels may contain, in addition to an composition of the present invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays may contain, in addition to a composition of the present invention, excipients such as lactose, talc, silicic acid, aluminum hydroxide, calcium silicates and polyamide powder, or mixtures of these substances. Sprays may additionally contain customary propellants, such as chlorofluorohydrocarbons and volatile unsubstituted hydrocarbons, such as butane and propane.

Transdermal patches have the added advantage of providing controlled delivery of a composition of the present invention to the body. Such dosage forms may be made by dissolving or dispersing the composition in the proper medium. Absorption enhancers may also be used to increase the flux of the composition across the skin. The rate of such flux may be controlled by either providing a rate controlling membrane or dispersing the composition in a polymer matrix or gel.

Ophthalmic formulations, eye ointments, powders, solutions and the like are also within the scope of the present invention.

Pharmaceutical compositions of the present invention suitable for parenteral administration comprise a cochleate or cochleate composition of the invention in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be used in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity may be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some embodiments, such adjuvants can be immunomodulators that can be added to the compositions of the present invention to stimulate an immune response. The immunomodulator can include comprise envelope proteins derived from human or animal viruses, oligonucleotides, e.g., CpG oligonucleotides, or can be chemical in nature. Specific chemical immunomodulators include, but are not limited to cytokines, chemokines and lymphokines, including, but not limited to, interferon alpha, interferon gamma, and interleuken 12. Examples of suitable animal viruses as a source of envelope protein include, but are not limited to, viruses from the following families: Arenaviridae, Bunyaviridae, Coronaviridae, Deltaviridae, Flaviviridae, Herpesviridae, Rhabdoviridae, Retroviridae, Poxyiridae, Paramyxoviridae, Orthomyxoviridae, and Togaviridae. Envelope proteins from influenza virus, Newcastle disease virus, and vaccinia virus, and Sendai virus are also encompassed in the present invention.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). The composition should be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride, in the composition. Prolonged absorption of the injectable compositions can be brought about by including in the composition an agent which delays absorption, for example, aluminum monostearate and gelatin.

Sterile injectable solutions can be prepared by incorporating a composition of the invention in the desired amount in an appropriate solvent with one or a combination of ingredients enumerated above, as required, followed by filtered sterilization. Generally, dispersions are prepared by incorporating the composition into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the cochleate compositions of the invention plus any additional desired ingredient from a previously sterile-filtered solution thereof.

Injectable depot forms are made by forming microencapsule matrices of the subject compounds in biodegradable polymers such as polylactide-polyglycolide. Depending on the ratio of drug to polymer, and the nature of the particular polymer used, the rate of drug release may be controlled. Examples of other biodegradable polymers include poly(orthoesters) and poly(anhydrides). Depot injectable formulations are also prepared by entrapping the drug in liposomes or microemulsions which are compatible with body tissue.

Oral compositions generally include an inert diluent or an edible carrier. They can be enclosed in gelatin capsules or compressed into tablets. For the purpose of oral therapeutic administration, the composition can be incorporated with excipients and used in the form of tablets, troches, or capsules. Oral compositions can also be prepared using a fluid carrier for use as a mouthwash, wherein the composition in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal-administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compositions of the invention also can be prepared in the form of suppositories (e.g., with conventional suppository bases such as cocoa butter and other glycerides) or retention enemas for rectal delivery.

In one embodiment, the compositions of the invention are prepared with carriers that will protect the composition against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of a composition calculated to produce the desired therapeutic effect in association with the required pharmaceutical carrier. The specification for the dosage unit forms of the invention are dictated by and directly dependent on the unique characteristics of the composition and the particular therapeutic effect to be achieved, and the limitations inherent in the art of compounding such a composition for the treatment of individuals.

The pharmaceutical compositions can be included in a container, pack, or dispenser together with instructions for administration.

The pharmaceutical compositions can be included in a container along with one or more additional compounds or compositions and instructions for use. For example, the invention also provides for packaged pharmaceutical products containing two agents, each of which exerts a therapeutic effect when administered to a subject in need thereof. A pharmaceutical composition may also comprise a third agent, or even more agents yet, wherein the third (and fourth, etc.) agent can be another agent against the disorder, such as a cancer treatment (e.g., an anticancer drug and/or chemotherapy) or an HIV cocktail. In some cases, the individual agents may be packaged in separate containers for sale or delivery to the consumer. The agents of the invention may be supplied in a solution with an appropriate solvent or in a solvent-free form (e.g., lyophilized). Additional components may include acids, bases, buffering agents, inorganic salts, solvents, antioxidants, preservatives, or metal chelators. The additional kit components are present as pure compositions, or as aqueous or organic solutions that incorporate one or more additional kit components. Any or all of the kit components optionally further comprise buffers.

The present invention also includes packaged pharmaceutical products containing a first agent in combination with (e.g., intermixed with) a second agent. The invention also includes a pharmaceutical product comprising a first agent packaged with instructions for using the first agent in the presence of a second agent or instructions for use of the first agent in a method of the invention. The invention also includes a pharmaceutical product comprising a second or additional agents packaged with instructions for using the second or additional agents in the presence of a first agent or instructions for use of the second or additional agents in a method of the invention. Alternatively, the packaged pharmaceutical product may contain at least one of the agents and the product may be promoted for use with a second agent.

In yet another aspect, the invention provides an article of manufacture of cochleates and/or cochleate compositions of the invention. The article of manufacture includes packaging material and a lipid contained within the packaging material. The packaging material includes a label or package insert indicating the use of the lipid for forming cochleates or cochleate compositions of the invention. The article of manufacture can further include instructions or guidelines for the formation of cochleates or cochleate compositions of the invention, e.g., mixing a biologically relevant molecule with a solvent and dripping it into a solution of the lipids. Optionally, the article of manufacture can include a solvent, a biologically relevant molecule, a multivalent cation (e.g., calcium and/or magnesium), a control biologically relevant molecule, and/or a chelating agent (e.g., EDTA). The article of manufacture may further include other ingredients or apparatus that can be used to manufacture the compositions of the present invention.

The instructions and/or guidelines may generally include one or more of the following statements:
1. Prepare a liposomal suspension by vigorously mixing the appropriate lipids according to the present invention in water or buffer.
2. Monitor lipid concentrations: low concentration would require a large volume of buffer in order to formulate an adequate amount of end product and high concentration may produce large cochleate aggregates upon the addition of calcium.
3. Experimentally determine whether to use water or buffered solution: the presence of salts and the pH of the suspension may affect the formation of the biologically relevant molecule-liposome intermediate depending on the properties of the biologically relevant molecule.
4. Optionally filter or perform other standard procedures to prepare liposomes of defined size and/or to sterilize the suspension.
5. Prepare a biologically relevant molecule solution with an appropriate solvent: many solvents may potentially be used in this process, e.g., DMSO.
6. Add the biologically relevant molecule-solvent solution, preferably dropwise, to the liposome suspension with vigorous mixing.
7. Experimentally determine the optimal rate of addition and speed of mixing: a suspension of biologically relevant molecule-liposomes essentially free of unencochleated biologically relevant molecule when viewed by light microscopy should be produced.
8. Calculate the amount of calcium to be added by assuming one mole of calcium for every two moles of lipid, and adding extra calcium to bring the buffer to between 2 and 6 mM.
9. Induce cochleate formation through addition of a calcium salt. The salt may be added as a solution, e.g., 0.1 M calcium chloride, or may be slowly added as a solid calcium salt, e.g., calcium chloride, with vigorous mixing.
10. If the presence of solvent in the buffer is unwanted, optionally harvest the biologically relevant molecule-cochleates, e.g., by centrifugation or filtration, and resuspending them in an appropriate medium. The association of calcium ions with PS is easily reversible, therefore, in order to remain intact and in their crystalline state, cochleate formulations can be resuspended in a medium containing at least 1 to 2 mM calcium ions.
11. Optionally evaluate the quality of the cochleate formulation. The presence of sufficient calcium ions initiates and maintains the cochleate structure. One method of evaluating the quality of a cochleate formulation is visualization of the liposomes that are produced upon removal of the calcium ions from a cochleate crystal. This may be accomplished using light microscopy. An aliquot of a biologically relevant molecule-cochleate suspension may be visualized by phase contrast microscopy at 1000x magnification. A small amount of a concentrated solution of a chelating agent, e.g., EDTA, may be added to the edge of the cover slip, thus reaching the sample through capillary action. A high-quality cochleate product will open into intact liposomes upon contact with the calcium-chelating agent. When using EDTA as the chelating agent, the pH of the EDTA solution should be about pH 9.5. Cochleates will not convert to liposomes at a pH below 6.5. If EDTA solutions at pH 7.4 are used, the release of hydrogen ions upon the binding of calcium to the acetate groups of the chelating agent lowers the pH of the solution and inhibits cochleate conversion to liposomes.

The choice of solvent and other materials, optimal rate of dropwise addition, speed of mixing, the amount of calcium, etc., can readily be determined by the skilled practitioner employing the teachings provided herein. For example, a liposome suspension might be provided already prepared, a combination of solvents might be used, excess calcium might be used to obviate the calculation of calcium, alternative or additional cations might be used, etc.

### EXAMPLES

### Example 1: Aminoglycoside Formulations for Oral Delivery and Administration

Infection by *Mycobacterium avium* (Ma) is common in immunocompromised patients and individuals with chronic lung conditions. Effective therapy of Ma infection is limited to few compounds, and the use of amikacin is restricted by the route of administration (intravenous or intramuscular) and toxicity.

Cochleates are stable, crystalline, anhydrous precipitates composed of phosphatidylserine (PS) and calcium (Ca), with a unique multilayered structure consisting of a large, continuous, solid, lipid bilayer sheet rolled up in a spiral or as stacked sheets. Cochleates reduce drug toxicity and mediate the oral delivery of drugs which are currently only available as injectable formulations.

Amikacin cochleate (Amkcch) formulations were optimized for amikacin encochleation efficiency and particle size by varying the type of PS used, the PS:drug ratio, PS:Ca ratio, and NaCl concentration. The efficacy of Amkcch against intracellular Ma infections was evaluated *in vitro* using mouse peritoneal macrophage infected with *M. avium* strains MAC 101 or MAC 109. Mouse peritoneal macrophages (Mo) Raw 264.7 cells were seeded at 10⁵ cells/well. Mo monolayers were infected at ratio 1:10 for 1 h and extracellular bacteria removed. Monolayers were treated with free amikacin and/or cochleate preparations for 4 days and the number of intracellular bacteria determined. Assays were repeated three times.

Untreated control Ma strains grew within Mo from 3.8 × 10⁵ to 4.9 × 10⁶. Ma within Mo treated with free amikacin (10 and 20 µg/ml) were killed to 6.1 and 3.4 × 10⁴ bacteria, respectively. Optimized Amkcch (10 and 20 µg/ml) demonstrated greater than 10-fold enhanced efficacy, reducing bacterial count to 3.9 and 1.7 × 10³ bacteria within Mo (p<0.05 compared with free amikacin).

Cochleate preparations of amikacin showed significant and enhanced activity against Ma strains in macrophages, suggesting that cochleates achieved higher intracellular concentration for a longer time than free amikacin. Animal experiments are ongoing.

Specifically, it was desired to develop a formulation of at least one existing aminoglycoside that can be orally administered. The aminoglycoside amikacin was selected to prepare amikacin cochleates.

Cochleate delivery vehicles are stable, crystalline phospholipid-cation precipitates composed of simple, naturally occurring materials such as phosphatidylserine and calcium. They have a unique multilayered structure consisting of a large, continuous, solid, lipid bilayer sheet rolled up in a spiral or as stacked sheets, with no internal aqueous space. This unique structure provides protection from degradation for associated "encochleated" molecules. Since the entire cochleate structure is a series of solid layers, components within the interior of the cochleate structure remain intact, even though the outer layers of the cochleate may be exposed to harsh environmental conditions or enzymes. Divalent cation concentrations *in vivo* in serum and mucosal secretions are such that the cochleate structure is maintained. Hence, the majority of cochleate-associated molecules are present in the inner layers of a solid, stable, impermeable structure. Once within the interior of a cell, however, the low calcium concentration results in the opening of the cochleate crystal and release of the molecule that had been formulated into cochleates.

To determine if an improved oral formulation of amikacin would be effective against *Mycobacterium avium*, cochleate delivery vehicles were utilized to prepare amikacin cochleates. Based upon previous studies, molecules formulated into cochleates are effective at intracellular delivery.

*Mycobacterium avium* infection is common in immunocompromised patients and individuals with chronic lung conditions. Effective therapy of *Mycobacterium avium* infection is limited to a few compounds, and the use of the aminoglycoside amikacin is restricted by the route of administration (intravenous or intramuscular) and toxicity. Widespread use of aminoglycosides has been limited due to their toxicity (nephrotoxicity and ototoxicity) and inconvenient route of administration (intravenous or intramuscular). The inefficient intracellular delivery of the aminoglycoside amikacin is responsible, in part, for the diverse adverse effects related to high plasma drug levels. In addition, use of amikacin for first-line therapy is limited due to poor oral absorption, a short half-life, and serious renal and auditory toxicities which necessitate frequent intravenous administration, with concomitant monitoring of drug levels to ensure that the levels in plasma remain within a narrow therapeutic window.

Particle scavenging cells, phagocytes, which include macrophages, neutrophils, and dendritic cells, represent a major first line of defense against microbial infection. Many pathogens including *M. avium* have evolved strategies that enable these microorganisms to subvert or even utilize the phagocyte physiology to multiply and cause disease. A major challenge to the effective treatment of intracellular microorganisms is the difficulty in achieving highly efficient delivery of antimicrobial agents across the plasma membrane of infected cells.

### a. Preparation of cochleate formulations, including varying lipid to drug ratios and varying salt concentration

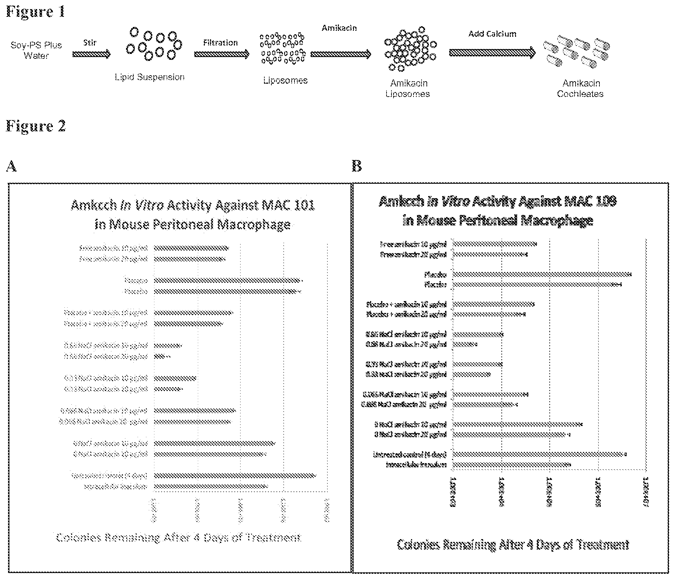

Amikacin cochleate (Amkcch) formulations were optimized for amikacin encochleation efficiency and particle size by varying the type of PS used, the PS:drug ratio, PS:Ca ratio, and NaCl concentration (Figure 1)

In order to make a concentrated suspension of crystalline cochleates of amikacin with a lipid:drug ratio of about 10:1, at 0 M NaCl, amikacin of 200 mg was mixed in 20 ml D.D (double distilled) water was filtered through a 0.22 µm filter and combined with 2000 mg of 50% soy PS liposomes in 200ml sterile water (the PS liposomes were first filtered through 5, 0.8, and 0.45µm filters) to form liposomes containing the amikacin. To the resultant mixture was then added 17ml of 0.1 M calcium chloride with vigorous mixing (stirring) during the addition to form cochleates. The Amikacin Cochleates were then dried under lyophilization. Sterile water was added to the dried powder Amikacin Cochleates to prepare a suspension of about 6.7mg amikacin/ml (1.0 mg/150µl) with a lipid:amikacin ratio of about 10:1.

In order to make a concentrated suspension of crystalline cochleates of amikacin with a lipid:drug ratio of about 10:1, and reduced particle size, at 0.66 M NaCl, amikacin of 200mg in 20ml D.D (double distilled) water was filtered through a 0.22µm filter and combined with 2000mg of 50% soy PS liposomes in 200ml sterile water (the PS liposomes were first filtered through 5, 0.8, and 0.45µm filters) to form liposomes containing the amikacin. To the resultant mixture was then added 17ml of 0.1 M calcium chloride with vigorous mixing (stirring) during the addition to form cochleates. To reduce the aggregation size of the cochleate crystals, 1125.2 mg sodium chloride in 3.88 ml sterile water was then added to the mixture of the cochleates. The Amikacin Cochleates were then dried under lyophilization. Sterile water was added to the dried powder Amikacin Cochleates to prepare a suspension of about 6.7mg amikacin/ml (1.0 mg/150µl) with a lipid:amikacin ratio of about 10:1, and a high (0.66M) NaCl concentration.

Table 1 reports the amount of amikacin in the supernatants obtained from the Amikacin Cochleate suspensions before and following lyophilization and reconstitution with sterile water. Amikacin was measured using the ninhydrin colorimetric assay.

**Table 1: Percentage of Amikacin Remaining with Pelleted Cochleates**

| | Ratio of the Soy PS to Drug | 0 NaCl Amikacin cochleates (before lyophilization) | 0 NaCl Amikacin cochleates (after lyophilization) | High NaCl Amikacin cochleates (before lyophilization) | High NaCl Amikacin cochleates (after lyophilization) |
|---|---|---|---|---|---|
| Cochleate Supt. | 10:1 | 33.3% | 36.2% | 38.8% | 40.5% |
| Encochleated AMK | 10:1 | 66.7% | 63.8% | 61.2% | 59.5% |

The formulations that were tested in the studies described below are not washed, neither for the *in vitro* nor for the *in vivo* studies. The amount of amikacin dosed is calculated upon the initial amount of amikacin in the starting formulation. The dosing is not dependent upon the amikacin encochleation efficiency. Gentamicin Cochleates (Gecch) were prepared in the same manner.

### b. Biological evaluations of cochleate preparations: In vitro macrophage assays

The efficacy of Amkcch against intracellular Ma infections was evaluated *in vitro* using mouse peritoneal macrophage infected with *M. avium* strains MAC 101 or MAC 109. Mouse peritoneal macrophages (Mo) Raw 264.7 cells were seeded at 10⁵ cells/well. Mo monolayers were infected at ratio 1:10 for 1 hour and extracellular bacteria removed. Monolayers were treated with free amikacin and/or cochleate preparations for 4 days and the number of intracellular bacteria determined. Assays were repeated three times.

The cochleate preparations used to evaluate biological activity against *Mycobacterium avium in vitro* included: 1. 2.0 ml /vial of 0 M NaCl amikacin formulation with 0.5mg/ml; 2. 2.0 ml /vial of 0.066M NaCl amikacin formulation with 0.5mg/ml; 3. 2.0 ml /vial of 0.33M NaCl amikacin formulation with 0.5mg/ml; 4. 2.0 ml /vial of 0.66M NaCl amikacin formulation with 0.5mg/ml; 5. 2.0 ml /vial of 0 M NaCl placebo formulation with 5mg PS/ml; 6. 2.0 ml /vial of placebo formulation plus amikacin with 0.5mg/ml; and 7. 2.0 ml /vial of free amikacin in water with 0.5mg/ml.

Tables 2-3 shows bacterial counts from each experiment. The data is represented graphically in Figures 2A-2B. Untreated control Ma strains grew within Mo from 3.8 × 10⁵ to 4.9 × 10⁶. Ma within Mo treated with free amikacin (10 and 20 µg/ml) were killed to 4.9 × 10⁴ and 3.8 × 10⁴ bacteria, respectively. Optimized Amkcch (10 and 20 µg/ml) demonstrated greater than 10-fold enhanced efficacy, reducing bacterial count to 3.9 and 1.7 × 10³ bacteria within Mo (p<0.05 compared with free amikacin). Similar results were obtained for experiments performed using Gentamicin Cochleate preparations (Table 4). Specifically, at the highest NaCl concentrations, the results with the Gentamicin Cochleates are at least 1 log better than free gentamicin.

**Table 2: Bacterial counts after M. avium 101-infected macrophages treated with Amkcch**

| Treatment | # bacteria day 4 | p value |
|---|---|---|
| Intracellular inoculum | 3.8 ± 0.4 × 10⁵ | |
| Untreated control (4 days) | 4.9 ± 0.5 × 10⁶ | |
| | | |
| 0 NaCl amikacin 20 µg/l | 3.3 ± 0.6 × 10⁵ | (2) |
| 0 NaCl amikacin 10 µg/l | 5.8 ± 0.4 × 10⁵ | (2) |
| 0.066 NaCl amikacin 20 µg/l | 5.3 ± 0.3 × 10⁴ | (1) |
| 0.066 NaCl amikacin 10 µg/l | 7.1 ± 0.4 × 10⁴ | (2) |
| | | |
| 0.33 NaCl amikacin 20 µg/l | 4.1 ± 0.4 × 10³ | (1) (3) |
| 0.33 NaCl amikacin 10 µg/l | 8.8 ± 0.3 × 10³ | (1) |
| | | |
| 0.66 NaCl amikacin 20 µg/l | 1.7 ± 0.6 × 10³ | (1) |
| 0.66 NaCl amikacin 10 µg/l | 3.9 ± 0.4 × 10³ | (1) |
| | | |
| Placebo + amikacin 20 µg/l | 3.4 ± 0.4 × 10⁴ | (1) |
| Placebo + amikacin 10 µg/l | 6.1 ± 0.4 × 10⁴ | (1) |
| | | |
| Placebo | 1.9 ± 0.5 × 10⁶ | |
| Placebo | 2.3 ± 0.4 × 10⁶ | |
| | | |
| Free amikacin 20 µg/l | 3.8 ± 0.5 × 10⁴ | (1) |
| Free amikacin 10 µg/l | 4.9 ± 0.3 × 10⁴ | (1) |

| | | |
|---|---|---|
| (1) p < 0.05 compared with the intracellular inoculum (2) p < 0.05 compared with untreated control (4 days) (3) p < 0.05 compared with the amikacin/cochleate placebo or free amikacin | | |

**Table 3: Bacterial counts after M. avium 109-infected macrophages treated with Amkcch**

| Treatment | # bacteria day 4 | p value |
|---|---|---|
| Intracellular inoculum | 2.4 ± 0.3 × 10⁵ | |
| Untreated control (4 days) | 3.1 ± 0.6 × 10⁶ | |
| | | |
| 0 NaCl amikacin 20 µg/l | 2.0 ± 0.6 × 10⁵ | (2) |
| 0 NaCl amikacin 10 µg/l | 4.1 ± 0.4 × 10⁵ | (2) |
| 0.066 NaCl amikacin 20 µg/l | 1.6 ± 0.5 × 10⁴ | (1) |
| 0.066 NaCl amikacin 10 µg/l | 3.2 ± 0.3 × 10⁴ | (1) |
| | | |
| 0.33 NaCl amikacin 20 µg/l | 5.3 ± 0.3 × 10³ | (1) (3) |
| 0.33 NaCl amikacin 10 µg/l | 9.3 ± 0.5 × 10³ | (1) |
| | | |
| 0.66 NaCl amikacin 20 µg/l | 2.6 ± 0.4 × 10³ | (1) (3) |
| 0.66 NaCl amikacin 10 µg/l | 9.6 ± 0.5 × 10³ | (1) |
| | | |
| Placebo + amikacin 20 µg/l | 2.5 ± 0.5 × 10⁴ | (1) |
| Placebo + amikacin 10 µg/l | 4.4 ± 0.3 × 10⁴ | (1) |
| | | |
| Placebo | 2.5 ± 0.5 × 10⁶ | |
| Placebo | 4.4 ± 0.3 × 10⁶ | |
| | | |
| Free amikacin 20 µg/l | 2.9 ± 0.4 × 10⁴ | (1) |
| Free amikacin 10 µg/l | 5.0 ± 0.3 × 10⁴ | (1) |

| | | |
|---|---|---|
| (1) p < 0.05 compared with the intracellular inoculum (2) p < 0.05 compared with untreated control (4 days) (3) p < 0.05 compared with the amikacin/cochleate placebo or free amikacin | | |

**Table 4: Bacterial counts after M. avium 101-infected macrophages treated with Gecch**

| | **Day 0** | **# Bacteria day 4** | **p value** |
|---|---|---|---|
| intracellular inoculum (time 0) | 3.9 ± 0.5 × 10⁵ | -- | -- |
| untreated control (4 days) | | 6.4 ± 0.5 × 10⁶ | -- |
| 0 NaCl gentamicin 30 µg/ml | | 6.1 ± 0.4 × 10⁵ | (2) |
| 0.066 NaCl gentamicin 30 µg/ml | | 8.9 ± 0.3 × 10⁴ | (1) (2) |
| 0.33 NaCl gentamicin 30 µg/ml | | 5.1 ± 0.6 × 10⁴ | (1) (2) (3) |
| 0.66 NaCl gentamicin 30 µg/ml | | 2.2 ± 0.3 × 10⁴ | (1) (2) (3) |
| Phosphate/low calcium gentamicin 30 µg/ml | | 1.2 ± 0.3 × 10⁵ | (1) (2) |
| Phosphate/high calcium gentamicin 30 µg/ml | | 1.6 ± 0.4 × 10⁵ | (1) (2) |
| Placebo + gentamicin 30 µg/ml | | 2.7 ± 0.5 × 10⁵ | (2) |
| Placebo | | 5.9 ± 0.3 × 10⁶ | |
| Free gentamicin 30 µg/ml | | 1.9 ± 0.3 × 10⁵ | (2) |

| | | | |
|---|---|---|---|
| (1) p < 0.05 compared with the intracellular inoculum (2) p < 0.05 compared with untreated control (day 4) (3) p < 0.05 compared with the gentamicin/cochleate control or free gentamicin | | | |

### c. Biological evaluations of cochleate preparations: In vivo C57/BL/6 mouse experiments

The *in vivo* efficacy of amikacin cochleates against *Mycobacterium avium* complex was evaluated using C57BL/6 black mice. Mice, 12/group, were infected with *M. avium* 101, (8.1 × 10⁷ bacteria/ mouse) by tail vein injection. After 7 days, 6 mice were harvested and the number of MAC in spleen was quantified to establish the baseline bacterial load (Time 0). Mice were treated with various amikacin preparations (indicated below) at 1mg amikacin/mouse/day for 2 weeks. Mice were harvested at week 3 and 2 days later (after 2 weeks of treatment), and spleens homogenized and plated onto 7H10 agar. Colonies on plates were counted and the data analyzed.

The mouse groups included the following: Group 1-1 week infected control; Group 2-3 week untreated infected control; Group 3- cochleate-AMK high salt oral 1mg/mouse/day; Group 4- cochleate-AMK high salt I.P. 1mg/mouse/day; Group 5- cochleate-AMK no salt oral 1mg/mouse/day; Group 6- cochleate-AMK no salt I.P. 1mg/mouse/day; Group 7- free amikacin oral 1mg/mouse/day; Group 8- free amikacin IP 1mg/mouse/day; Group 9- placebo cochleates oral; and Group 10- placebo cochleates IP.

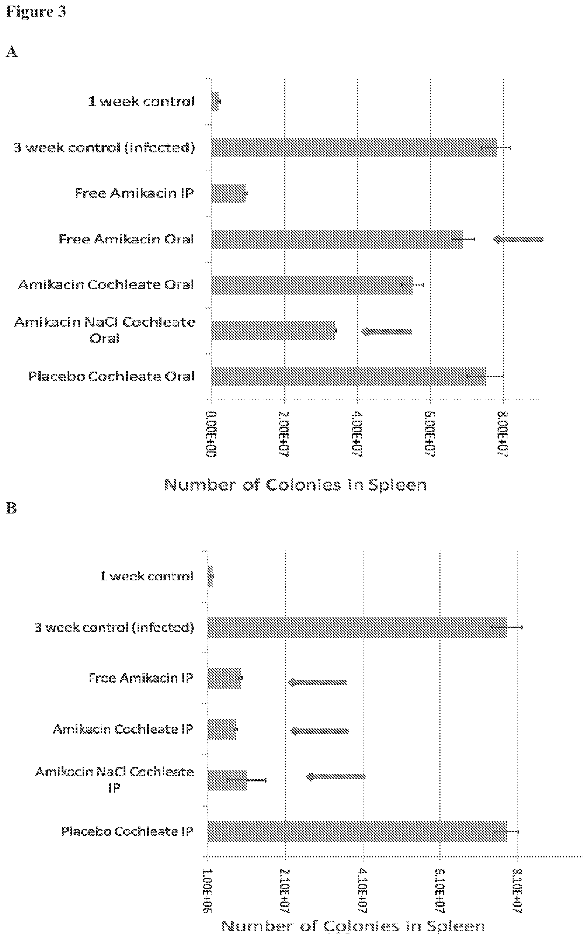

The bacterial count in spleen of C57BL6 mice after 2 weeks of treatment are shown in Table 5. Graphical depiction of in vivo efficacy results are shown in Figure 3. Thus, cochleate preparations, whether given I.P. or orally, were active and reduced the number of bacterial load in the spleen. In addition, the amikacin cochleate preparation with high salt concentration dosed orally was significantly more active than free amikacin. Also, the dose of amikacin used in the study was below the tolerated dose. Greater doses can be used with potentially improved effect. Finally, the results show the potential of cochleate preparations to deliver amikacin orally in effective, anti- *M. avium* concentrations.

**Table 5: Splenic bacterial counts (CFU) in M. avium 101-infected C57BL/6 mice after 2 weeks of treatment**

| Experimental group | Treatment route of administration | CFU/spleen | p value |
|---|---|---|---|
| 1 week (control) | --- | 2.1 ± 0.4 × 10⁶ | --- |
| 3 weeks (control infected) | --- | 7.8 ± 0.4 × 10⁷ | --- |
| AmKCochleates | oral | 5.5 ± 0.3 × 10⁷ | NS |
| AmKCochleates | I.P. | 8.1 ± 0.4 × 10⁶ | (1) (2) |
| high NaCl AmKCochleates | oral | 3.4 ± 0.2 × 10⁷ | (1) (3) |
| highNaCl AmKCochleates | I.P. | 1.1 ± 0.5 × 10⁷ | (1) |
| Placebo cochleate | oral | 7.5 ± 0.5 × 10⁷ | NS |
| Placebo cochleate | I.P. | 7.8 ± 0.3 × 10⁷ | NS |
| Free AmK | oral | 6.9 ± 0.3 × 10⁷ | NS |
| Free AmK | I.P. | 9.3 ± 0.4 × 10⁶ | (1) |

| | | | |
|---|---|---|---|
| (1) p < 0.05 compared with control at 2 weeks (2) p < 0.05 compared with I.P./oral administration of the cochleate preparation (3) p < 0.05 compared with free amikacin administered by the same route | | | |

Thus, the data demonstrate that amikacin cochleates are a promising formulation of an aminoglycoside for oral administration at effective, anti-*M*. *avium* concentrations.

### Example 2: Amphotericin B Formulations for oral Delivery and Administration (part of the disclosure)

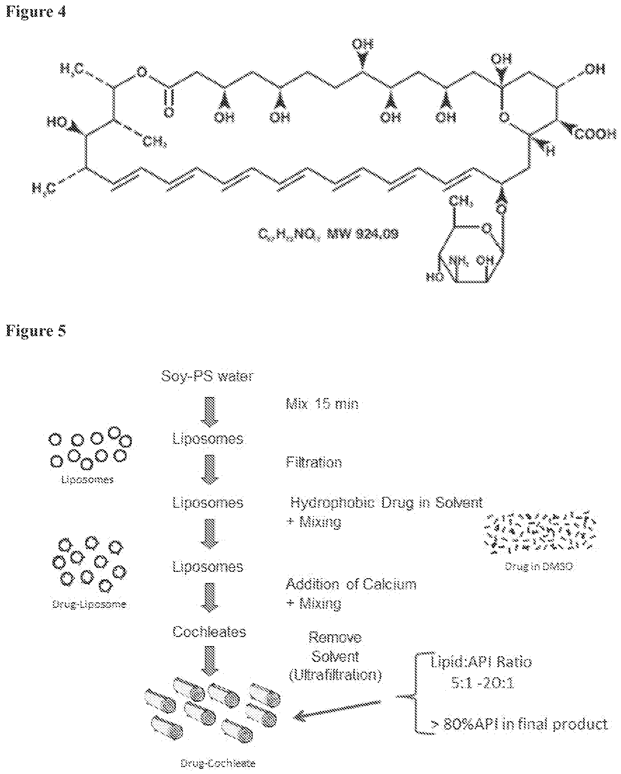

Although Amphotericin B (AmB; Figure 4) is still one of the most effective antifungals after 60 years of use, commercial formulations are restricted to intravenous delivery and the molecule is highly toxic. The lead product in development using cochleate technology is amphotericin B-cochleates (CAMB; also known as Bioral™ Amphotericin B) and it is a model for encochleation of amphiphilic or hydrophobic drugs. Figure 5 shows a schematic representation of a manufacturing strategy for making CAMB. The following results demonstrate that oral administration of CAMB is as effective as equivalent, injectable doses of the leading AmB formulation (Fungizone) in mouse models of systemic candidiasis and aspergillosis. Fungizone is roughly 5-10 nm in diameter, whereas CAMB are roughly 200-300 nm in diameter. CAMB also demonstrate substantially lower toxicity than existing commercial AmB products. In addition, CAMB showed good safety in rats and dogs in 7 and 28 day toxicity studies.

### a. Inhibition of Candida growth within macrophages in vitro

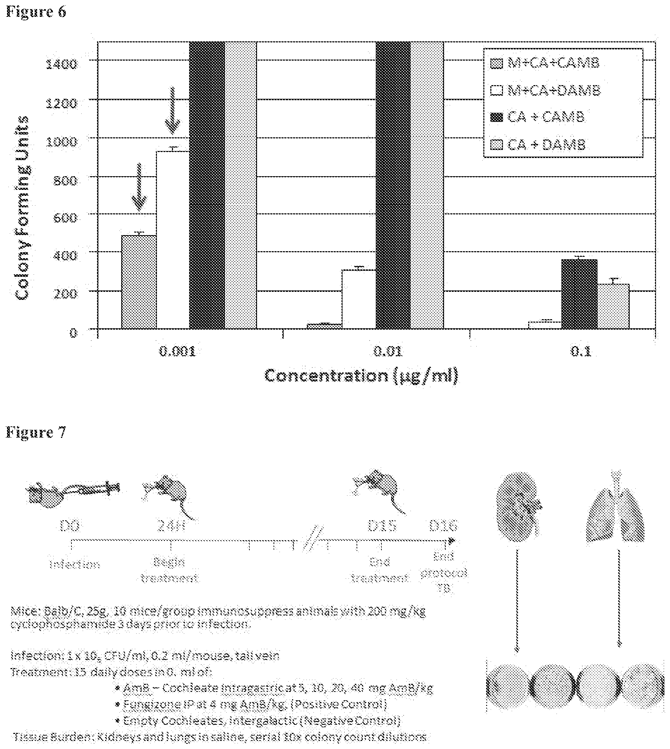

The results shown in Figure 6 demonstrate that CAMB is more effective than Fungizone (DAMB) in inhibiting *Candida albicans* infection in macrophage *in vitro.* Specifically, all concentrations of CAMB (Amphotericin B cochleates) are as efficacious as DAMB (Fungizone) at inhibiting the growth of *Candida* cultures in the absence of macrophages. However, in the presence of Candida infected macrophages, CAMB is significantly more efficacious than DAMB, showing nearly zero CFU's down to 0.01 µg/ml while DAMB that showed nearly 300 CFU's at this concentration. The results demonstrate that CAMB more effectively delivers AmB into macrophages than Fungizone.

### b. Inhibition of Aspergillosis fumigatis infection in vivo

Figure 7 shows a schematic protocol for assessing survival and tissue burden analysis of systemic aspergillosis infection of mice and subsequent treatment with oral amphotericin B-cochleates (CAMB) and Fungizone (DAMB). Briefly, Initial evaluation of CAMB formulations was performed in a murine model for disseminated aspergillosis. Mice were rendered susceptible to infection by A. fumigatus through transient immunosuppression with cyclophosphamide. The mice (20-25 g) were treated with cyclophosphamide (200 mg/kg iv through lateral tail vein) 3 days prior to infection of the tail vein with 10⁵⁻⁶ A. fumigatus spores. Treatment was started immediately after infection by oral administration of CAMB at 0-40 mg/kg/day and lasted for 10 days. Fungizone was served as a positive control and were administered IP at 4 mg/kg/day. It typically produced 50-80 % survival. Untreated mice showed 100% mortality within 8-10 days. Kidneys, liver and lungs were collected from each animal and fungal load will be assessed by serial dilution and plating of tissue homogenates. Mortality data was statistically evaluated by Kaplan-Meier analysis followed by the Wilcoxon test. A p-value of P<0.05 was considered a statistically significant difference. Comparisons of colony counts among different treatment groups were performed by the Kruskal-Wallis test with multiple comparison. A P value of <0.05 was considered statistically significant. All statistical analysis was performed with the software package GBSTAT (Dynamic Microsystems, Inc., Silver Spring, MD.).

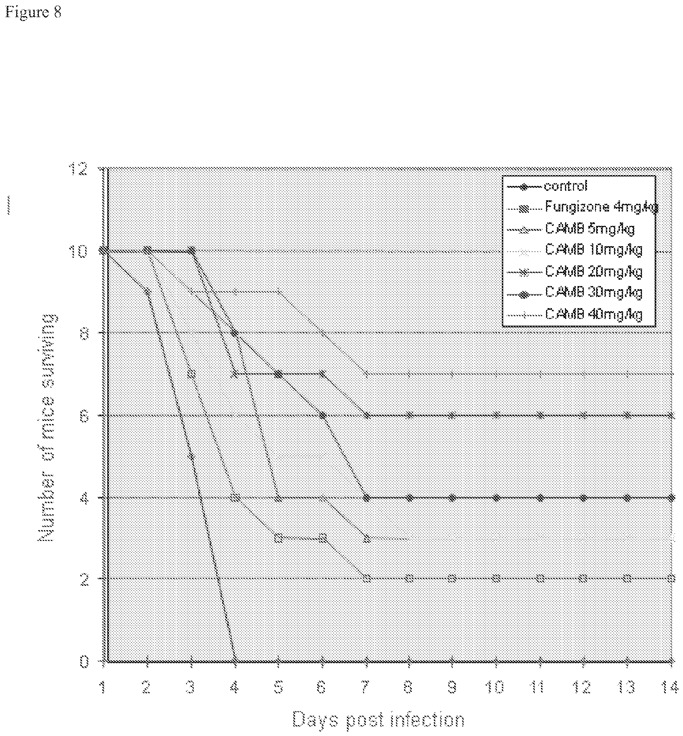

CAMB was demonstrated to increase survival of mice in a dose-dependent manner (Figure 8). DAMB at 4 mg/kg showed only moderate protection of 20% of the cohort, whereas higher doses of DAMB were toxic to the animals. By contrast, 60% of mice survived when treated with 20 mg/kg CAMB and 70% of mice survived when treated with 40 mg/kg CAMB.

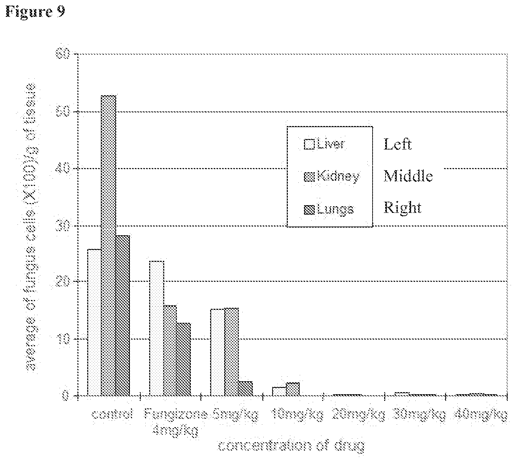

The effects of CAMB on mouse survival is accompanied by a reduction in the tissue fungal burden of target organs. Specifically, a 2 to 3 log reduction in CFU per gram of tissue was observed for CAMB (oral; p.o.) at doses of 10 mg/kg/day (Figure 9). *Aspergillus* infection was nearly eradicated at doses above 20 mg/kg/day, which is roughly comparable to intraperitoneal administration of DAMB at 2.0 mg/kg/day. Kidney tissue burden analysis showed that CAMB was more potent than Fungizone® (DAMB) at 1 mg/kg/day and equivalent to AmBisome® (LAMB) at 10 mg/kg/day.

### c. CAMB pharmacokinetic data in vivo from animal models

An investigational new drug (IND) study for AmB-cochleates was opened and phase I human clinical trials have been successfully initiated based on the observed safety in animals, efficacy in animals, shelf life stability, reproducible GMP manufacturing process, and cost-effectiveness.

A 28-day toxicity study was conducted to determine potential toxic effects, target organs of toxicity, and a no observable adverse effect level (NOAEL) in rats and dogs following daily oral dosing with CAMB. Large scale GLP batches of CAMB and placebo (cochleate vehicle without AmB) were prepared for these 28 day studies with an AmB concentration of 5 mg/mL. CAMB was administered daily as single doses by oral gavage to 10 dogs per group (5 m, 5 f) at doses of 15, 30, and 45 mg/kg and to 48 rats (24 m, 24 f) at doses of 30, 45, and 90 mg/kg. Cardiovascular parameters in dog were evaluated pre-study and in week 4. In rats, 18 animals (9 m, 9 f) per dose group served as a satellite group for TK samples (blood, urine, and fecal samples for drug analysis). Animals were sacrificed on day 29 or on Day 42, 2 weeks after last dose administration. TK blood (Days 1 and 28 (dog) and 23 (rat)), urine and feces (weeks 1 and 4), and tissue samples (at necropsy) were collected for AmB quantitation by validated LC-MS methods. Clinical pathology, serum chemistry tests, urinalysis, and histopathology analysis were done on all animals.

It was determined that CAMB was well tolerated by rats and dogs at all doses with no mortalities or clinical abnormalities found in either species based on comparison with historical controls. Pharmacokinetic data in dogs for Day 1 were comparable to previous 7-day studies. The increase in maximum plasma AmB concentration (Cₘₐₓ) in dog was not dose-dependent after 28 days, similar to what had been observed on Day 7 in the 7-day study. In dog, the kidney had quantifiable concentrations of AmB at all doses while liver, lung, and spleen had AmB concentrations detectable at all 3 doses. In rats, quantifiable AmB concentrations were found in liver, lung, and kidney, but not in spleen. Additional analytical and histopathological analyses will be conducted in dog and rat.

Regarding mortality/morbidity and clinical observations, all animals survived until their scheduled necropsy, except two female dogs in the high dose group. Microscopic evaluation confirmed that the deaths of the two dogs were not related to the test article but to errors in gavage administration. No clinical changes of toxicological significance were found in the control or CAMB-treated groups. No meaningful differences in body weight and body weight gain between control and CAMB-treated groups were observed. No statistically significant changes in food consumption in control versus treated groups. Changes in body temperature (females in treated groups, Day 28) were within normal range. Regarding *serum chemistry and coagulation*, all changes at pre-study and post-dose administration were sporadic, slight, and within their respective normal reference ranges and were not considered to be treatment-related. Importantly, in rats and dogs, creatinine and blood urea nitrogen were within normal ranges. No differences between control and CAMB-treated groups were observed based on urinanalyses. There were no electrocardiographic abnormalities or blood pressure changes related to oral administration of CAMB. Any microscopic findings for dogs surviving until study termination were considered unrelated to the administration of the vehicle (controls) or CAMB. The findings were considered related to spontaneous disease or conditions, or to procedures related to necropsy activities. In dogs, plasma levels of AmB were below the lower limit of quantitation (LLOQ) of 20 ng/mL at several time-points following the administration of low dose (15 mg/kg), especially on Day 1. Overall, plasma drug levels on Day 28 were higher than those on Day 1 at all 3 doses of CAMB. All urine concentrations were above the LLOQ of 20 ng/mL. Urinary excretion over 24 hr on Days 1 and 28 showed no clear trend of drug accumulation in urine in contrast to plasma. AmB excretion in urine was negligible, ranging from 0.02 to 0.05% of dose administered.

Regarding toxicokinetic (TK) analyses, the mean tₘₐₓ values from all dose groups ranged from 7.2 to 17.6 hr on Day 1 and from 3.6 to 8.4 hr on Day 28. There appeared to be an accumulation of AmB with repeat dose administration, as indicated by increased Cₘₐₓ and AUCₗₐₛₜ values on Day 28 when compared with corresponding values on Day 1. The mean Cₘₐₓ values on Day 28 were ∼ 1.1- to 2.3-fold higher than those obtained for Day 1. Likewise, the mean AUCₗₐₛₜ estimates on Day 28 were ∼ 1.9- to 7.5-fold higher on Day 28 than those estimated for Day 1. Plasma t_{1/2} could not be estimated for several animals, especially at the lowest dose and on Day 1, due to the limited number of plasma concentrations after tₘₐₓ that were > LLOQ of the bioanalytical assay, or due to the lack of a good fit (r < 0.8) of the best-fit line in the terminal elimination phase. There appeared to be no major differences in the plasma toxicokinetics of AmB between males and females on both days of blood sampling.

Based on the results, this study demonstrated that single daily oral doses of CAMB administered for 28 days are well tolerated in all dose groups in the rat and dog. The NOAEL for males and females was considered to be at least 45 mg/kg for dog and 90 mg/kg for rat. Quantifiable concentrations of AmB were measured in kidney of the dog and the rat as well as the liver and lung of the rat. Daily oral administration of CAMB to male and female beagle dogs at 15, 30, or 45 mg/kg/day and male and female rats at 30, 45, or 90 mg/kg/day did not produce overt adverse effects. The NOAEL is at least 45 mg/kg/day in dogs and 90 mg/kg/day in rats for daily oral dose administration of CAMB for 28 consecutive days. No test article-related effects were seen in the following parameters: clinical observations, body weights, food consumption, body temperature, clinical pathology (hematology, serum chemistry and coagulation), urinalysis, ophthalmology, cardiology, organ weights, macroscopic and microscopic evaluations. Toxicokinetic analysis indicated an accumulation of AmB in all 3 dose groups on Day 28 after repeat administration as indicated by increased Cₘₐₓ and AUCₗₐₛₜ. Urinary excretion of drug was negligible when compared with the actual dose administered. There were no major differences between male and female dogs in the disposition of drug at the dose levels studied. Finally, tissue AmB analysis showed quantifiable concentrations in dog kidney but only detectable levels in liver, lung, and spleen. In the rat, the liver, lung, and kidney showed quantifiable AmB concentrations but not in spleen.

### d. CAMB pharmacokinetic data in vivo from human subjects

Sixteen human subjects were recruited for each cohort (12 CAMB, 4 placebo empty cochleate vehicle). CAMB or volume-matched Bioral placebo (delivery vehicle without amphotericin B) was administered orally in double-blind fashion following overnight fast. Each subject was given a single dose. Safety and pharmacokinetic evaluations were conducted for 2 weeks after administration. Subjects were recruited in 3 cohorts given escalating doses of 200, 400, and 800 mg. This was a single-dose, double-blind, dose-escalating, pharmacokinetic (PK) study in healthy volunteers, designed to determine the safety, tolerability, and PK profile of Amphotericin B and to estimate relative bioavailability with cross-study comparison to other Amphotericin formulations. A total of 48 subjects were planned for participation in this study, across 3 cohorts, 16 per treatment cohort. Within each treatment cohort, 12 subjects received active drug and 4 received placebo. Subjects checked into the clinic on Day 0 and were randomized to active treatment (200, 400, or 800 mg CAMB depending on cohort) or placebo. Blood samples for PK analysis were collected predose (0 hours), and postdose on Day 0 (1, 2, 4, 8, and 12 hours) and Day 1 (24 hours). Subjects checked out of the clinic when all scheduled procedures were completed. Additional blood samples were collected on an outpatient basis on Days 2, 3, 4, 8, 9, and 14 (48, 72, 96, 192, 216, and 336 hours). Safety was evaluated by monitoring adverse events (AEs), concomitant medication, physical examination and medical history, and clinical laboratory and vital sign measurements.

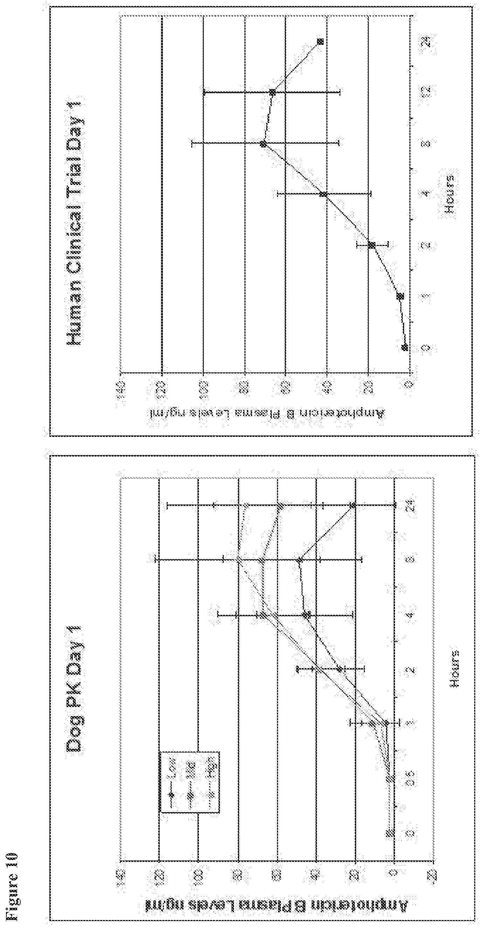

For example, Figure 10 shows the results of CAMB pharmacokinetic data from administration to dogs and humans. The results demonstrate that plasma levels of CAMB in rats and dogs are associated with therapeutic CAMB tissue levels. Specifically, CAMB was well-tolerated at single oral doses of 200 and 400 mg. Plasma concentrations of amphotericin were obtained from this oral dosage form and are comparable to prior results from animal toxicology studies. Figure 10, as well as Table 6, further demonstrate that dog and human CAMB plasma levels are extremely similar. Adverse events were reported for the full cohort of 16 subjects. Gastrointestinal adverse events were seen in 6%, 38% and 56% of subjects at 200, 400 and 800 mg respectively, and all were mild at doses of 200 and 400 mg. The most common AE was nausea, seen in 6% of subjects taking 200 mg and 19% of subjects taking 400 mg, and were of mild severity in all cases at those dose levels. One subject became pregnant and underwent elective termination. There were no abnormalities in clinical laboratory testing of blood or urine. Pharmacokinetic evaluation of amphotericin was conducted. Thus, there were no serious adverse events and no withdrawals due to adverse events. No abnormalities in laboratory or other safety testing were observed, including those associated with renal function.

**Table 6: CAMB single dose pharmacokinetic analysis**

| Comparison of Dogs and Humans | | | | | |
|---|---|---|---|---|---|
| Species | Dose | HED (Human equivalent dose) | Cₘₐₓ ng/ml | Tₘₐₓ Hours | AUC₀₋₂₄ ng-hr/ml |
| Human | ∼3mg/kg - 4mg/kg | - | Mean 70.3 | 8-12 | 1231 |
| Dog* | 15mg/kg | 8.1 mg/kg | 48.5 | 12 | 833 |
| Dog* | 30mg/kg | 16.2 mg/kg | 67.2 | 12 | 1400 |
| Dog* | 45mg/kg | 24.3 mg/kg | 80.2 | 6 | 1653 |

| | | | | | |
|---|---|---|---|---|---|
| * Dog data from Day 1 of the 28 day GLF study in 2008. | | | | | |

A commercially viable and cost effective manufacturing process for AmB-cochleates has been developed and scaled-up 100 liter GMP batches of AmB-cochleates have been produced.

### Example 3: Contemplated Amikacin-Cochleate Formulations for Oral Delivery and Administration

A good laboratory practices (GLP) scaled-up process can be developed for amikacin-cochleate formulations can be prepared similar to that for AmB-cochleates by preparing 1 L, 5L, and 20 L, or more batches. The physical and chemical properties of the Amkcch will be measured for all batches prepared. These measurements will include amikacin encochleation efficiency, lipid:drug ratio, and particle size. Lot-to-lot reproducibility of the product will be determined.

### a. Determination of amikacin concentration by HPLC

Many procedures for the quantitation of aminoglycosides, including amikacin, in fluids and tissues have been developed and reported, (PaPP, E, Knupp, C., and Barbhaiya, R.H., J. Chomatography, 574: 93 -99, 1992; Soltes, L. Biomedical Chromatography 13: 3-10, 1999). A quantitative anikacin HPLC assay can be designed.

### b. Determination of amikacin activity by microtiter-plate based assay

Amikacin activity can also be determined using microtiter-plate based assays using, for example, E. Coli. For example, the influence of different concentration of amikacin on growth of *E. coli* reference strain ATCC 25922 (American Type Culture Collection) was determined by microtiter plate method as described by José A. Rufián-Henares, Francisco J. Morales (Food Chemistry, 111: 1069 - 1074, 2008). Overnight suspension of E. coli was growth at 37°C in LB broth in the shaker at speed of 250 rpm for 24 hours. (New Brunswick Scientific mode: INNVOA 40R). Bacteriological growth media LB broth was supplied by Life Technologies. A clear, polystyrene 96-well cell culture cluster with flat bottom and low evaporation lid sterile plate was supplied by Fisher distributor.

Preliminary experiments were performed in order to define the initial concentration of inoculums of E.coli strain. Two fold serial dilutions with LB broth from the overnight culture were made. 250µl of bacterial cell suspension and 250ul LB broth were then added into a sterile 96 micro-plate (LB broth for blank). Then the 96 micro-plate is placed into the Molecular Devices chambers for the OD reading with endpoint at 600nm in order to get the optical densities. Once the appropriate concentration of E.coli density was obtained from OD, the culture of bacteria was diluted to an appropriate dilution factor in fresh LB broth. Based on E.coli routine growth characteristics, bacterial strains were diluted to optical density (OD) readings taken during growth experiments using a Molecular Devices SPECRAmax 340 microplate reader at a wavelength of 600nm.

A two-fold serial dilution of free amikacin in LB broth for positive control of bacteriostatic activity. Aliquoted appropriate suspension of placebo and amikacin cochleates into a 15ml sterile tube, then centrifuge the samples at 2800 rpm and 4°C (SORVALL RT 6000B) for approximately 30 minutes. The supernatant was collected and stored in sterile tubes at 4°C for subsequent microtiter activity assay. The placebo and amikacin cochleates pellets were resuspended with 2mM calcium chloride solution, then the samples were centrifuged again at same conditions as above. The supernatant was removed and the placebo and amikacin pellets were resuspended with 2mM calcium chloride solution, then centrifuged again at same conditions as above. The placebo and amikacin cochleates pellet were resuspended with 2mM calcium chloride solution and brought to original volume.

The microtiter plate activity assay is very sensitive. Two-fold dilutions for all samples above would be needed in order to be able assay in the standard curve range. A standard control curve of amikacin in LB broth was made by increasing the concentrations from 1 to 40ug/ml. A control blank of 2mM calcium chloride solution was also made. Subsequently, carefully dispensed 50ul of the samples, LB broth solution, 2mM calcium chloride solution and standard samples into the 96 micro-plate, 200ul of bacterial cell suspension into the 96 micro-plate, 250ul LB broth solution (blank), 250ul bacterial cell suspension (positive control), into the 96 micro-plate. The micro-plate was placed into the plate reader chamber without covers, then started to initial read at wavelength 600nm with endpoint to have a time 0 reading. Switched to microbial growth kinetic record assay on a Molecular Device Micro-plates reader with 37°C (SPECTRA max 340). The 96 well microplates was agitated by the microplate reader for 10 seconds before first reading and 10 seconds between each reading to achieve homogenous suspension. There were total 37 reads in a 5-minute interval and 19 seconds for a minimum interval. At the times of 3 hours, 5 hours, and 22 hours, the samples were also read at wavelength 600nm with endpoint. The standard curve was calculated by the softmax Pro 3.1.2 software. Each experiment was performed by triplicate.

### c. Determination of phospholipid phosphorus

Determination of phosphate concentration in phospholipids samples by a modified version of the Bartlett assay (Bartlett, G.R., and (1959) J. Bio. Chem. 224, 466). This assay is suitable for determining the phospholipid concentration in cochleate formulations, if the phospholipids are the only phosphate containing compounds in the formulation. Determination of total phospholipid concentration in drug containing or empty control phospholipid cochleate formulations. The formulation drug concentration is divided by the formulation phospholipid concentration to give determine the drug to phospholipid ratio of the formulation.

This method is used to determine the phospholipid concentration in cochleate formulations. The method is a modified version of the Bartett phosphate assay (Bartlett, G.R., (1959) J. Bio. Chem. 224, 466) which measures inorganic phosphate concentration. This method can be used to measure the concentration of any phosphate containing lipid such as phosphatidylserine, phosphatidylcholine, phosphatidic acid, and phosphatidylethanolamine. Common phospholipids have one phosphate group per phospholipid. The assay does not distinguish between different phospholipids. The assay measures the total molar phosphate concentration due to the various phospholipids. Since the method detects phosphate concentration, any other source of phosphate in the formulation, e.g. from the drug, phosphate containing buffers, or other excipients, will interfere with the assay. Accurate phospholipid determination by this method requires other phosphate sources in the formulation must be eliminated or accounted for in the calculations. The sample is first heated in the presence of sulfuric acid at 200 °C for 1 hr in a chemical hood to decompose the lipid. Hydrogen peroxide is then added to decolorize any charred material. The samples are then reacted with an ascorbic acid / ammonium molybdate solution for 20 minutes at 45 °C. Under these conditions the phosphate reacts with the molybdate to form a blue colored molybdenum compound. A 1.00 mM sodium phosphate solution is used to generate a standard curve for the assay. The phosphate concentration is determined by measuring the standard and sample O.D. at 820 nm in a spectrophotometer. The sample phosphate concentration will be determined for each sample formulation with a N=3. The average mM phosphate concentration and standard deviation will be calculated. Care must be taken to insure that any water used in this assay is sufficiently deionized and that the glassware is not contaminated with extraneous phosphate from detergents or other sources. In addition, unlike many other Bartlett based phosphate assays, this method does not use a perchloric acid heating step. Perchloric acid vapors deposit explosive salts. Heating the sample with sulfuric acid works just as well and is much safer.

### d. Determination of cochleate-product particle size

Determination the of particle diameter volume-weighted distribution of cochleate formulations by diffraction-based light-scattering. Final results will include a plot of the distribution, the maximum particle diameters (µm) of each differential peak, the less than 50 % total volume particle diameter (µm), the percentage of the volume with particle diameter less than 1 µm, and the less than 99 % total volume particle diameter (µm).

Diffraction-based light-scattering measurements can determine the particle size distributions in the 0.04 µm to 2000 µm range. This technique is particularly well suited to determine particle size distributions greater than 1 µm. Particle sizes less that 1 µm are more accurately measured by photon correlation spectroscopy (PCS). Particle size distributions of amphotericin B phosphatidylserine cochleates and phosphatidylserine cochleates are measured by diffraction-based light-scattering using a Coulter LS230 particle size with a "small volume module plus" sample attachment. Polarization Intensity Differential Scattering (PIDS) data is included. The instrument is allowed to warm-up for 2 hours prior to the measurement. The sample chamber is rinsed 2X volumes with distilled water and 1X volume with 2 mM CaCl₂. The sample chamber is then filled with 2 mM CaCl₂. The pump debubbling program is run to degas the 2 mM CaCl₂. The pump is run at speed (%) 50. A phosphatidylserine cochleate suspension is added until the PIDS obscuration percentage is between 45 to 55%. Data is collected and stored in three consecutive blocks of 180 seconds and processed using the fraunhofer optical model. Results are plotted as log normal distribution using differential volume (%) versus log particle diameter and < cumulative volume (%) versus log particle diameter. Particle diameter distribution statistics are calculated by arithmetic statistics. Values used to characterize the particle diameter distribution include the volume distribution mode, median, arithmetic mean and arithmetic S.D (standard deviation). The shape of the distribution is defined by the C.V. (coefficient of variation) value and the arithmetic skewness value. The distribution is also characterized by the percent of the volume with a diameter < 1 µm and the diameters corresponding to < 10, 50, and 99 % of the volume.

### e. Determination of cochleate-product stability

Amikacin-cochleate product prepared during the scale-up process will be stored as suspensions and dry powders (following lyophilization) at 4°C, 25°C and 40°C. Amikacin content (HPLC) and activity (*E. coli* microtiterplate assay) will be measured monthly for 6 months.

### f. Determination of oral efficacy of the cochleate-product

Oral efficacy studies of the Amikacin-Cochleate Product will be measured using a mouse model of Mycobacterium avium infection. These studies will include dose range and maximum tolerated dose studies. Oral efficacy studies of the Amikacin-Cochleate Product in combination with a second drug will be measured using a mouse model of Mycobacterium avium infection. These studies will include dose range and maximum tolerated dose studies. Oral amikacin-cochleates will be compared to free amikacin IP. Both uninfected and infected animals will be studied. Oral amikacin-cochleates will be compared to free amikacin IP. Both uninfected and infected animals will be studied. Serum will be measure for creatinine and blood urea nitrogen (nephrotoxicity) and kidneys (for nephrotoxicity) and inner year (for ototoxicity) will be obtained for histopathology examination.

The optimal dose will be evaluated. Effective parenteral dose of AK range from 50 to 100 mg/Kg/day of weight in mice. The effect of 10, 25, 50 and 100 mg/Kg/day giving orally as a cochleate preparation will be assessed. Treatment will be given for 4 weeks, after what the mice will be killed and the bacterial load determined in spleen and liver. Organs will be obtained, homogenized, serially diluted and plated for CFU onto Middlebrook 7H11 agar plates with oleic acid, albumin, dextrose and catalase (OADC). C57/BL6 black mice will be infected intravenously (tail vein) with 0.1 ml of a suspension containing 10⁷ *M. avium* strain 101, a virulent strain in mice (Bermudez LE, et al., AAC 45: 2210,2001). One week after infection a group of 8 mice will be killed and the bacterial colony forming units (CFU) determined in liver and spleen, to establish the infection inoculum. The remaining of the mice (12 mice/experimental group) will be administered treatment according with the following groups: a. Infected, untreated; b. Infected, 10 mg/Kg/day, orally; c. Infected, 25 mg/Kg/day, orally; d. Infected 50 mg/kg/day, orally; e. Infected, 100 mg/Kg/day, orally; f. Infected, free amikacin, 100 mg/Kg/day, orally; g. Infected, empty cochleate; and h. Infected, free amikacin, 100 mg/Kg/day, intra-peritoneal, Gold-Standard; for a total of 104 mice. Mice will be treated for 4 weeks, and then killed. The bacterial load will be determined in the liver and spleen and compared with the CFU/organ at 4 weeks and at week 0, before initiation of treatment. Mice will be monitored for side effects associated to treatment. Data will be interpreted according to the comparisons. When comparing the organ load between experimental groups after 4 weeks of treatment, the decrease in CFU/organ in relation to untreated control would mean that the medication has a bacteriostatic effect. The decrease in CFU/organ in relation to the CFU/organ at time 0, will mean a bactericidal activity. The results and comparisons will be analyzed by using Student's T test and ANOVA. Twelve mice per group gives a power of 0.90 for the experiment.

### g. Determination of Amkcch efficacy in a lung model of M. avium infection

*M.avium* infects the host using two different routes. One is the gastro-intestinal route, from where the bacteria can disseminate or/and cause lymph node infection. The other is the respiratory route, by which the bacterium causes infection in individuals with chronic pulmonary conditions (bronchiectasis, emphysema, cystic fibrosis). Because the latter route of infection is common in individuals with underlying lung disease, and because the lung infection is associated with the formation of biofilm (Carter G, et al, AAC 48:4907, 2004; Yamazaki Y, et al Cell Microbiol, 8: 808. 2006), the testing of the cochleate-AK preparation in an animal model of *M.avium* lung disease is important.

The experiment will be performed as previously described (Yamazaki et al, Cell Microbiol 8: 808, 2006; Bermudez LE et al. J Infect Dis 2007). Briefly, a 0.1ml of a suspension of 10⁸ *M.avium* bacteria will be delivered into the C57BL/6 mice nostril and infection will be allow to establish for two weeks. A group of 8 mice will be killed and the initial inoculum in the lungs determined. Treatment will then be initiated according to the following experimental groups: a. Infected, no treatment; b. Infected, Free Amikacin 100 mg/kg/day intra-peritoneal. Gold Standard; c. Infected, cochleate-AK, 100 mg/Kg/day (or other effective dose to be determined in the experiment described above); and d. Infected, empty cochleate; for a total of 56 mice.

The mice will be treated for 4 weeks and then killed. The lungs will be obtained, homogenized, serially diluted and plated for bacterial load onto Middlebrook 7H11 agar plates with OADC. Bacterial growth occurs between 10 and 15 days. The bacterial load will be determined in the liver and spleen and compared with the CFU/organ at 4 weeks and at week 0, before initiation of treatment. Mice will be monitored daily for evidence of toxicity and side effects. Data will be interpreted according to the comparisons. When comparing the organ load between experimental groups after 4 weeks of treatment, the decrease in CFU/organ in relation to untreated control would mean that the medication has a bacteriostatic effect. The decrease in CFU/organ in relation to the CFU/organ at time 0, will mean a bactericidal activity. The results and comparisons will be analyzed by using Student's T test and ANOVA. Twelve mice per group gives a power of 0.90 for the experiment.

### h. Determination of resistance to Amkcch

C57BL/6 mice will be infected intravenously with *M.avium* strain 101 according to the following groups: a. Infected, untreated and b. Infected, cochleate-AK, for a total number of 70 x 2 groups + 8 or 148 mice. By day 7 after infection, 8 mice will be harvested to establish the baseline infection load, and frequency of resistance to amikacin. Then, mice will be treated for 12 weeks with cochleate-AK, orally. Control animals will not receive treatment. The frequency of resistance of *M.avium* to amikacin will be determined at weeks 0, 2, 4, 6, 8, 10 and 12. Spleens will be harvested and homogenate will be plated on both 7H11 agar with amikacin and without amikacin.

Mice (10 mice/timepoint) will be treated for several weeks, and then killed. The bacterial load will be determined in the spleen. The frequency of resistance will be determined as explained in Study Design. Colonies that grow in amikacin plate will be tested for MIC of amikacin as previously reported (Bermudez et al. J Infect Dis 174: 1218, 1996). Data will be interpreted according to the comparisons between organs plated on medium containing amikacin and without amikacin. The appearance of any colony in medium containing the cutoff concentration of amikacin will mean the presence of resistant colony. The number of resistant CFU will be compared with the number of resistant CFU in bacteria obtained from spleen before treatment as well as with bacteria obtained from spleen at the same timepoint. The frequency of resistance to free amikacin is 10⁻¹¹. The results and comparisons will be analyzed by using ANOVA. Ten mice will be used per timepoint. The experiment will determine the frequency of resistance to the oral preparation of cochleate-AK. Very likely it will be similar to the frequency of resistance to free amikacin, but because the intestinal absorption can impact serum concentration, it will be important to examine the frequency of resistance.

### i. Evaluation of Amkcch in combination with clarithromycin and ethambutol

Treatment of *M.avium* infection, both disseminated or pulmonary rely on the activity of few drugs, like clarithromycin (or azithromycin) and ethambutol. A macrolide is crucial for a efficacious oral regimen (Chaisson R, et al. Ann Intern Med 121: 905, 1994), but the addition of ethambutol results in synergistic effect and decrease the emergency of resistance to clarithromycin (Bermudez LE, et al. J Infect. Dis 174: 1218, 1996).

Cochleate-AK will be administered in combination with clarithromycin, ethambutol or both. C57BL/6 mice will be infected intravenously according to the following groups: a. Infected, untreated; b. Infected, empty cochleate; c. Infected, clarithromycin, 100 mg/Kg/day; d. Infected, ethambutol, 100 mg/Kg/day; e. Infected, cochleate-AK, dose to be determined; f. Infected, clarithromycin + ethambutol; g. Infected, clarithromycin + cochleate-AK; h. Infected, ethambutol + cochleate-AK; and i. Infected, clarithromycin + ethambutol + cochleate-AK; for a total of 116 mice.

After 7 days, 8 mice will be harvested to establish the baseline infection load in liver and spleen. Treatment will begin and administered for 4 weeks. After, the mice will be killed and the bacterial load in spleen, liver, and lungs will be quantified, by plating onto Middlebrook 7H11 agar. Twelve mice will be used per experimental group. The bacterial load will be determined in the liver and spleen and compared with the CFU/organ at 4 weeks and at week 0, before initiation of treatment. Mice will be monitored in a daily basis to side effects and toxicity. Data will be interpreted according to the comparisons. When comparing the organ load between experimental groups after 4 weeks of treatment, the decrease in CFU/organ in relation to untreated control would mean that the medication has a bacteriostatic effect. The decrease in CFU/organ in relation to the CFU/organ at time 0, will mean a bactericidal activity. The results and comparisons will be analyzed by using Student's T test and ANOVA. Twelve mice per group gives a power of 0.90 for the experiment. The experiment will determine if the oral preparation of cochleate-AK is synergistic or/and additive to any of the routinely used compounds to treat *M.avium* infections.

### j. Determine the activity of Amkcch against clarithromycin-resistant strains

Clarithromycin or azythromycin-resistant strains of *M.avium* represent a major problem in the treatment of the condition. The macrolides are very potent and key component of the therapy. Disease caused by macrolide-resistant strain represents a challenge for treatment. A clinical isolate, clarithromycin-resistant (MAC184 strain, with a mutation in A₂₂₇₅ to C₂₂₇₅, MIC 64 mcg/ml) and with MIC to ethambutol of 8 mcg/ml (compared to MIC of 4 mcg/ml of a susceptible *M.avium* strain, such as MAC101) will be used to infect C57BL/6 Black mice intravenously according to the following groups: a. Infected, untreated; b. Infected, clarithromycin 100 mg/Kg/day; c. Infected, ethambutol 100 mg/Kg/day; d. Infected, cochleate-AK, dose to be determined; e. Infected, ethambutol + cochleate-AK; f. Infected, ethambutol + free amikacin (IM); and g. Infected, empty cochleate; for a total of 92 mice. After 7 days, 8 mice will be harvested to determine the bacterial load in spleen, liver and lungs before treatment. Then, treatment will be initiated. Mice (12/experimental group) will be treated for 4 weeks, and then killed. The bacterial load will be determined in the liver, spleen and lung and compared with the CFU/organ at 4 weeks and at week 0, before initiation of treatment. Mice will be monitored in a daily basis to side effects and toxicity. Data will be interpreted according to the comparisons. When comparing the organ load between experimental groups after 4 weeks of treatment, the decrease in CFU/organ in relation to untreated control would mean that the medication has a bacteriostatic effect. The decrease in CFU/organ in relation to the CFU/organ at time 0, will mean a bactericidal activity.
The results and comparisons will be analyzed by using Student's T test and ANOVA. Twelve mice per group gives a power of 0.90 for the experiment.

The experiment will determine if the oral preparation of cochleate-AK is active against clarithromycin (and azithromycin) -resistant *M.avium.* This results are important because they will determine if cochleate-AK has activity against clarithromycin-resistant strains and if the activity of the cochleate preparation is comparable to free amikacin at the same dose.

### k. Evaluation of toxicity and tissue and serum concentration of the Cochleate-AK preparation

Infected (systemically) or non-infected mice will receive treatment with free amikacin and 100 mg/Kg/day of oral cochleate according to the following groups: a. uninfected, intra-peritoneal free amikacin for 4 weeks; b. uninfected, oral cochleate-AK, for 4 weeks; c. infected, intra-peritoneal free amikacin for 4 weeks; d. infected, oral cochleate-AK for 4 weeks; and infected, untreated mice, for toxicity studies; for a total of 50 mice.

Amikacin distribution and toxicity will be evaluated by histopathology and serum and tissue levels will be determined by HPLC. Each experimental group will have 10 mice. For the drug levels, serum, lungs and spleen will be obtained. For toxicity evaluation, kidneys and inner year will be obtained for histopathology examination.

### 1. Evaluation of Amkcch on nontuberculous mycobacteria (NTM) treatment

Nontuberculous mycobacteria (NTM) are organisms common in soil and environmental and potable water that have been associated with lung disease in select patient groups. Treatment requires lengthy multi-drug regimens that can be poorly tolerated and poorly effective, especially in patients with severe disease or in those who have failed prior treatment attempts. There have been very few clinical trials to support current treatment recommendations and no new drugs have been assessed for this disease in many years. Amikacin is an established drug that is effective against a variety of NTM. However, its use is limited by the need to administer it intravenously and by toxicity to hearing, balance, and kidney function. Nanocochleateamikacin is a novel formulation that offers both oral bioavailability and the potential for reduced toxicity. Preclinical studies suggest greater efficacy against *Mycobacterium avium* complex compared with off-the-shelf amikacin.

A study can be carried out to evaluate the safety and efficacy of nanocochleateamikacin in 34 (30 evaluable + 12% drop out) adult patients with lung infections due to *Mycobacterium avium* complex (MAC) or *Mycobacterium abscessus* that have failed to respond to standard, guideline-based treatment regimens. Participants who have been on a stable mycobacterial drug regimen for at least 3 months with persistent positive cultures will undergo a screening evaluation. They will then be stratified based on the presence or absence of cystic fibrosis and randomized in a 2:1 ratio to receive either 84 days of oral nanocochleateamikacin or 84 days of oral placebo. This will be followed by an 84 day open label period during which all participants will receive daily dosing of nanocochleateamikacin. Participants will additionally continue on the same antimycobacterial drugs they were taking before starting the study. The screening visit will occur within the 14 days prior to study day 1. All subjects will be followed for 28 days after last dose of study drug. Baseline and 28 day follow-up visits conducted during the study period will assess clinical laboratory parameters, audiology testing, clinical adverse events, and pulmonary function to determine the qualitative and quantitative safety and tolerability of nanocochleateamikacin. Expectorated sputum will be collected to determine changes in mycobacterial smear and culture status. Computed tomography of the chest will be done at baseline and at days 84 and 168 of dosing. Six-minute walk distance, oxygen saturation, and spirometry will be measured, symptoms will be assessed using the Pulmonary Symptom Severity Score, and pulmonary quality of life will be evaluated using the St. George's Respiratory Questionnaire (SGRQ) at each visit. Sputum, blood, and urine specimens will be collected to assess drug concentrations. The primary objectives of the study will be: 1) Evaluate safety and tolerability of 84 days administration of nanocochleateamikacin versus placebo; 2) Evaluate the 84 day efficacy of nanocochleateamikacin versus placebo for recalcitrant NTM lung disease. The secondary objectives will be:1) Evaluate the safety and efficacy of 84 day vs 168 day administration of nanocochleateamikacin; 2) Evaluate the pharmacokinetics (PK) of nanocochleateamikacin; and 3) Evaluate long-term safety of nanocochleateamikacin (168 days). The primary outcomes for this study are:1) The frequency of treatment related adverse events through 84 days of daily dosing; in particular, this includes the following occurrences: a. Adverse events leading to permanent discontinuation of study medication; b. Audiovestibular adverse events; c. Renal adverse events; d. Serious adverse events through 84 days of study drug administration; and 2) Proportion of patients on nanocochleateamikacin achieving a reduction in sputum mycobacterial culture growth at 84 days compared to placebo. Secondary outcomes include: 1) Proportion of patients on nanocochleateamikacin achieving a reduction in organisms seen on sputum mycobacterial smear at 84 days in nanocochleateamikacin compared to placebo; 2) Proportion with culture conversion to negative at 84 and 168 days of nanocochleateamikacin compared with 84 days placebo; 3) Time to reduction in sputum mycobacterial culture growth; 4) Time to need for "rescue" anti-mycobacterial drugs; 5) Time to and duration of treatment for acute bacterial exacerbations of bronchiectasis; 6) Change in computed tomography scan abnormalities at 84 and 168 days; 7) Change in Quality of Life using the St. George's Respiratory Questionnaire at 84 and 168 days; 8) Change in Pulmonary Symptoms Severity Score at 84 and 168 days; 9) Change in 6-minute walk distance and oxygen saturation at 84 and 168 days.

### m. Evaluation of oral aminoglycoside cochleate formulations for biodefense

Tularemia and Brucellosis are serious diseases in humans, caused by infection with *Francisella tularensis* and *Brucella abortus,* respectively. Such infections by microorganisms are difficult to treat due to their ability to evade the innate immune response and replicate within phagocytes. Aminoglycosides, such as amikacin and gentamicin, effectively inhibit the growth of these
microorganisms *in vitro,* but have limited therapeutic use due to the inflexible IV route of delivery, substantial toxicity, and limited ability to achieve therapeutic intracellular concentrations. Aminoglycoside-cochleate formulations could impart oral bioavailability, low toxicity and
increase the therapeutic index of aminoglycosides. This could substantially increase the usefulness of aminoglycosides in the treatment of *Francisella tularensis* and *Brucella abortus*, and potentially additional intracellular bacterial infections.

First-generation formulations of amikacin cochleates and gentamicin cochleates have already been produced. For example, a suspension of soy bean-derived phosphatidylserine (SPS) liposomes in water (10 mg lipid/ml) is formed by adding dry powdered SPS to water and mixing for 15 minutes. The liposome suspension is then filtered through a 5 flm filter to remove any insoluble material and produce a more uniform population of liposomes. A solution of aminoglycoside in water is prepared (usually ∼10x the desired final concentration). One tenth the
volume of the aminoglycoside solution is added dropwise to the suspension of liposomes. The final ratio of lipid:drug varies between 5:1 and 20:1. The aminoglycoside-liposome suspension is allowed to mix for 10 minutes. To form cochleates, a 0.1 M solution of CaCh is added dropwise with mixing to the aminoglycoside-liposome suspension. The final suspension is allowed to mix for 10-15 minutes. The final formulation appears as a fine, white, particulate suspension. Visualized in the light microscope at 1,000x, the formulation is composed of small cochleate aggregates. The efficiency of drug loading into cochleates is determined by pelleting the cochleates and quantitating the amount of aminoglycoside in the pellet and supernatant using a ninhydrin-based colorimetric assay.

A mouse peritoneal macrophage cell line (Raw 246.7), which is a commonly used cell line, and THP-1, a human macrophage cell line, can be used for the experiments. The cells will be cultured in DMEM and RPMI-1640, respectively, supplemented with 5% heat-inactivated fetal bovine serum. Macrophage monolayers will be established by adding 10⁵ macrophages to a 24-well tissue culture plate. Monolayers with THP-1 macrophages will need to be treated with phorbol ester, to induce maturation and adhesion to the plastic. After 24 hours, monolayers will be infected with either 10⁵ *F. tularensis* or *B. abortus.* The infection will be allowed to happen for 1 hour, and then the extracellular bacteria will be removed by washing. Some of the well contents will be lysed and plated onto Middlebrook 7H10 agar plate, to determine the intracellular inoculum of the bacterium. The remaining wells will be treated daily for 5 days with: 1- free gentamycin; or 2-free amikacin; or 3- gentamicin cochleate preparations; or 4- amikacin cochleate preparations according to the following groups: 1. Control, harvest at 24 h; 2. Control, harvested at day 5; 3. Free amikacin; 4. Cochleate amikacin; 5. Free gentamycin; 6. Cochleate gentamycin; 7. Free cochleate; and 8. Free doxycycline (for Brucella). After treatment, cell monolayers will be lysed and the lysate plated into 7H10 agar to quantify the intracellular load.

The assays will be repeated three times. Results will be obtained as colony forming units (CFU)/ml of macrophage lysate. Comparisons will be made between the number of bacteria in mono layers lysed before treatment (time 0) and the bacterial load in monolayers lysed after 5 days of treatment. The results will be interpreted as bactericidal activity when the number of bacteria in the treated experimental group is smaller than the number of bacteria inside cells at time 0, and as bacteriostatic activity when the number of bacteria at 5 days of treatment is smaller than the untreated control, at the same time point, but greater than the number of bacteria at time 0.

*Francisella tularensis* is very infectious. A small number (10-50 or so organisms) can cause disease. *If F. tularensis* were used as a weapon, the bacteria would likely be made airborne for exposure by inhalation. People who inhale an infectious aerosol would generally experience severe respiratory illness, including life-threatening pneumonia and systemic infection, if they are not treated. By definition of the CDC, *Francisella tularensis* is considered a Category A pathogen; *Brucella* species are considered Category B pathogens. Thus, experiments with *Francisella tularensis* and *Brucella abortus* will be carried on in a Biosafety Level3 (BSL-3) laboratory.

Preliminary experiments have been performed according to the following protocol: 1. Raw 246.7 macrophages at 10⁵ per monolayer; 2*. F. tularensis* LVS: 5 x 10⁵ bacteria/monolayer; 3. Allow infection to happen for 1 h.; 4. Wash the wells to remove bacteria that were not ingested by macrophages; 5. Lyse some wells/monolayers before the beginning of the treatment, to establish the number of intracellular bacteria; 6. Start treatment of the monolayers with compounds; 7. After 4 days, stop treatment; 8. Allow 1 day without adding treatment to the wells; 9. Lyse monolayers with water; 10. Serial dilution; and 11. Plate the lysate onto chocolate agar plates to determine the number of intracellular CFU (CFU/ml).. The results are presented in Table 7.

**Table 7: Bacterial counts after F. tularensis LVS-infected macrophages treated with Gecch**

| **Treatment groups** | **Day 0** | **Day 4** | **p value** |
|---|---|---|---|
| 1 h control (no treatment) | 2 ± 0,4 × 104 | - | - |
| Gentamicin (0 M NaCl) 2 µg/ml | - | 1.5 ± 0.5 × 10³ | (1) (2) (3) |
| Gentamicin (0.066 M NaCl) 2 µg/ml | - | 8.6 ± 0.4 × 10² | (1) (2) (3) |
| Gentamicin (0.33 M NaCl) 2 µg/ml | - | 6.9 ± 0.2 × 10² | (1) (2) (3) (4) |
| Gentamicin (0.6 M NaCl) 2 µg/ml | - | 5.1 ± 0.3 × 10² | (1) (2) (3) (4) |
| Contamicin (free) 2 µg/ml | - | 9.3 ± 0.3 × 10² | (1) (2) (3) |
| Amikacin (0 M NaCl) 4 µg/ml | - | 8.9 ± 0.4 × 10² | (1) (2) (3) |
| Amikacin (0.066 M NaCl 4 µg/ml | - | 6.4 ± 0.5 × 10² | (1) (2) (3) |
| Amikacin (0.33 M NaCl)4 µg/ml | - | 4.7 ± 0.3 × 10² | (1) (2) (3) (4) |
| Amikacin (0.66 M NaCl) 4 µg/ml | - | 2.4 ± 0.2 × 10² | (1) (2) (3) (4) |
| Amikacin (free) 4 µg/ml | - | 7.6 ± 0.3 × 10³ | (1) (2) (3) |
| Control empty cochleates | - | 2.7 ± 0.5 × 10⁶ | (1) (2) (3) |
| Control no treatment | - | 3.0 ± 0.3 × 10⁶ | (1) (2) (3) |

| | | | |
|---|---|---|---|
| (1) p < 0.05 compared to 1 h control (2) p < 0.05 compared to control no treatment at day 4 (3) p < 0.05 compared to empty cochleate (4) p < 0.05 compared to free drug | | | |

### Example 4: Cochleate Formulations for Enhanced Immune Responses and Protection From Microbial Infection (part of the disclosure and invention)

Cochleates are anhydrous, stable, multi-layered lipid crystals which spontaneously form upon the interaction of phosphatidylserine and calcium. Cochleate formulations remain intact in physiological fluids, including mucosal secretions, plasma and gastrointestinal fluid. Thus, cochleates can be used to mediate the delivery of biologically active compounds by many routes of administration, including oral, mucosal and intravenous.

Cochleate formulations were prepared by associating an API with a suspension of phosphatidylserine liposomes followed by the dropwise addition of a CaCl2 solution. The properties of the API (hydrophobicity or charge) are used to form the liposome-API intermediate. All studies reported were in mouse models. Oral administration of cochleate-API formulations, which are crystalline suspensions, was by oral gavage (usually 0.1 ml).

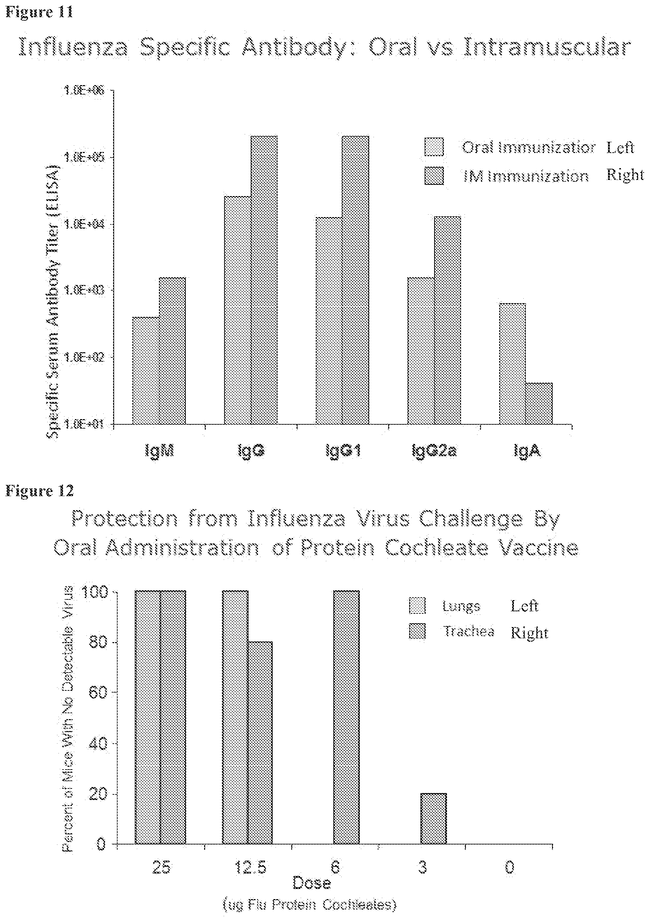

*Elicited immune responses*: Influenza protein cochleates were prepared by extracting the influenza virion envelope containing HA and NA proteins and incorporating these proteins into the cochleate matrix. Influenza protein cochleates were administered by either an intramuscular route or an oral route. Circulating antibody to influenza virus and protection from virus challenge were measured. Figures 11 and 12 demonstrate that oral administration induces systemic immune responses and the detection of neutralizing antibodies indicates the intact structure of influenza proteins presented to the immune system and delivered by cochleates.

In addition, oral administration of protein-cochleates containing the envelop proteins of influenza virus resulted in strong systemic cellular and humoral immunity, including high titers of neutralizing antibody, and protection from intranasal virus challenge. Oral administration of DNA-cochleates containing a plasmid expressing the *env, tat* and *rev* genes of HIV virus resulted in strong systemic cellular and humoral immunity against gp160.

*Protection from microbial infection* - A single dose of siRNA-cochleates targeted to influenza virus resulted in a 200 fold (intranasal administration) and a 20 fold (intravenous administration) reduction in virus titer in the lung. Oral administration of Amikacin-cochleates resulted in protection from *M. avium* infection. Oral administration of Amphotericin B-cochleates resulted in protection from both *Aspergillis* and *Candida* infection.

The results indicate that oral administration of cochleate formulations of proteins and DNA plasmids resulted in systemic humoral and cellular immune responses, demonstrating that cochleates protect these molecules from digestion in the GI tract and mediate intracellular delivery. Oral delivery of cochleate formulations of small molecule drugs results in therapeutic systemic delivery. These data provide a strong experimental rationale for investigating the use of cochleates for the oral delivery of therapeutic proteins and oligonucleotides.

### Example 5: Cochleate Formulations for Enhanced Encochleation of Hydrophilic Molecules

Cochleates are anhydrous, stable, multi-layered lipid crystals which spontaneously form upon the interaction of phosphatidylserine and calcium. Cochleate formulations remain intact in physiological fluids, including mucosal secretions, plasma and gastrointestinal fluid, thereby mediating the delivery of biologically active compounds by many routes of administration, including oral, mucosal and intravenous. Initially, cochleates have been used to formulate hydrophobic drugs. For example, oral formulations of encochleated amphotericin B (AmB) have entered human clinical trials. However, it has been difficult to appreciably encochleate hydrophilic molecules or large molecules with hydrophilic domains.

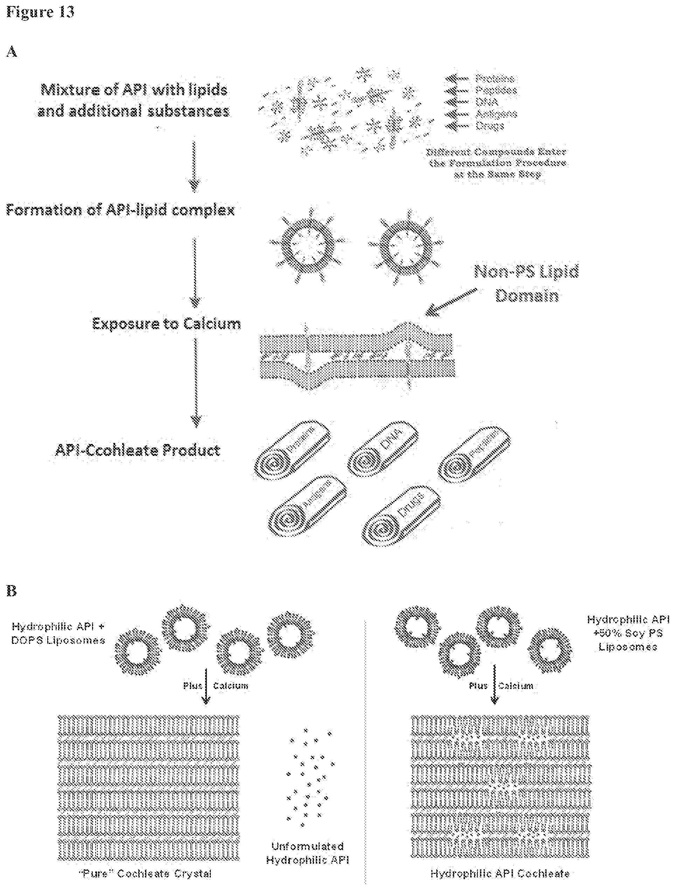

Specifically, cochleate formulations have traditionally been prepared by associating an API with a suspension of phosphatidylserine vesicles followed by the dropwise addition of a CaCl₂ solution. AmB was used to represent a typical hydrophobic drug, whereas amikacin and gentamicin are hydrophilic drugs. It has been discovered herein that hydrophilic molecules or large molecules with hydrophilic domains can be formulated into cochleates by associating the API with a lipid domain that does not interact with calcium and acts like a "raft" that remains intact and imbedded within the cochleate crystal matrix (Figures 13A-13B).

For example, incorporation of phosphatidylserine (PS) combined with 99.9% pure dioleoylPS, 99.9% pure soyPS, 75% soyPS and 50% soyPS, were used to manufacture cochleates. The lipid composition of 99.9% pure PS was modified by the addition of sphingomyelin, phospatidylcholine and/or cholesterol. Encochleation efficiency was determined by assaying the concentration of drug in the supernatant following centrifugation.

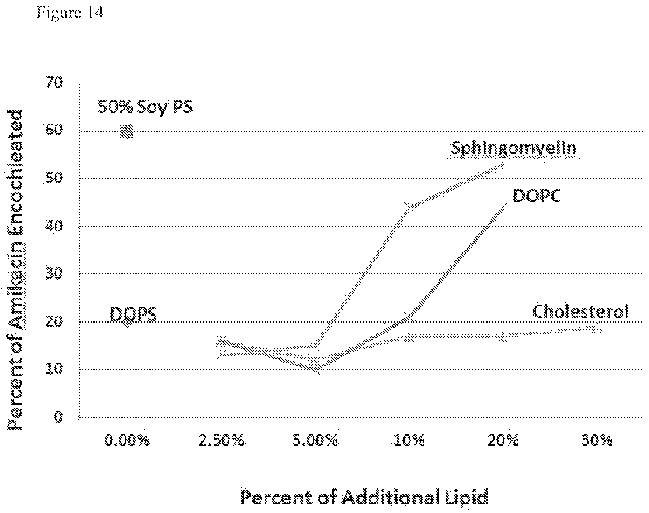

The encochleation efficiency of AmB is greater than 85%, and was independent of the lipid composition of the PS vesicles. By contrast, the encapsulation efficiencies of both amikacin and gentamicin were only 15% - 20% using 99.9% pure PS as compared to 60% - 65% using 50% soy PS. The addition of sphingomyelin, and/or phosphatidylcholine to pure PS vesicles increased the encochleation efficiencies approaching that of 50% soyPS. The results are presented in Figure 14.

In summary, the addition of Ca2⁺ to PS vesicles induces the formation of cochleates, an "anhydrous" complex of closely apposed membranes with highly ordered crystalline acyl chains and a very high transition temperature. The encochleation efficiencies of hydrophobic molecules, which can insert within the acyl chain bilayers is, within broad limits, independent of the composition of the PS vesicle. Hydrophilic molecules can be formulated into cochleates by associating the API with a lipid domain that does not interact with calcium and acts like a "raft" that remains fluid, intact and imbedded within the calcium-PS crystal matrix.

## Claims

1. A cochleate composition comprising a population of cochleates, wherein the cochleates comprise:
a) a negatively charged first lipid;
b) a cation, wherein the cation is a divalent cation selected from the group consisting of calcium, zinc, barium, and magnesium cations;
c) a neutral second lipid; and
d) a biologically relevant molecule, wherein the biologically relevant molecule is an aminoglycoside;
wherein the ratio of the negatively charged first lipid to the neutral second lipid is between 1:1 to 9:1;
wherein the negatively charged first lipid is phosphatidylserine and comprises at least 50% of the total lipid; and
wherein the neutral second lipid is phosphatidylcholine or sphingomyelin.

2. The cochleate composition of claim 1, wherein the ratio of the negatively charged first lipid to the neutral second lipid is between 4:1 to 9:1.

3. The cochleate composition of claim 1, wherein the second lipid comprises lipid capable of forming hydrogen bonds to the biologically relevant molecule.

4. The cochleate composition of claim 1, wherein the second lipid is embedded among the negatively charged first lipid.

5. The cochleate composition of claim 1, wherein the negatively charged lipid comprises dioleoyl PS (DOPS) or soybean-derived phosphatidyl serine (soy PS).

6. The cochleate composition of claim 1, wherein the cochleates further comprise a minor amount of a third lipid, wherein the third lipid is selected from the group consisting of a zwitterionic lipid, a PEGylated lipid, a cationic lipid, or a polycationic lipid.

7. The cochleate composition of claim 1, wherein the aminoglycoside is selected from the group consisting of gentamicin, netilmicin, tobramycin, amikacin, kanamycin A, kanamycin B, neomycin, paromomycin, neamine, streptomycin, dihydrostreptomycin, apramycin, ribostamycin, or spectinomycin.

8. The cochleate composition of claim 1, further comprising an aggregation inhibitor.

9. The cochleate composition of claim 8 wherein the aggregation inhibitor is sodium chloride.

10. A pharmaceutical composition comprising an effective amount of the cochleate composition of claim 1 and a pharmaceutically acceptable carrier.

11. A method of making the cochleate composition of claim 1 comprising:
a) mixing the aminoglycoside with a liposome comprising a negatively charged first lipid and a second lipid wherein the second lipid is a neutral second lipid; and
b) adding a cation, wherein the cation is a divalent cation selected from the group consisting of calcium, zinc, barium, and magnesium cations, to make the cochleate composition,
wherein the ratio of the negatively charged first lipid to the neutral second lipid is between 1:1 to 9:1;
wherein the negatively charged first lipid is phosphatidylserine and comprises at least 50% of the total lipid; and
wherein the neutral second lipid is phosphatidylcholine or sphingomyelin.

12. The method of claim 11, wherein the ratio of the total lipid to biologically relevant molecule is at least 4:1.

13. The method of claim 11, wherein the calcium cation is provided from calcium chloride, wherein the calcium chloride concentration of the mixture is 2 mM to 10 mM.

14. The method of claim 11, further comprising a step c), wherein an aggregation inhibitor is mixed with the cochleate compositions.

15. The composition according to claim 1 for use in a method of treating a subject comprising the administration of the composition of claim 1 to a subject, wherein the subject is any animal, including a human, a mouse, a rat, a rabbit, a non-human primate, or any other mammal.

16. The composition for use according to claim 15, wherein the administration is by the oral, intranasal, intraocular, intraanal, intravaginal, or intrapulmonary route.

17. The composition for use according to claim 16, wherein the cochleate composition further comprises a bile salt.

## Patentansprüche

1. Cochleat-Zusammensetzung, umfassend eine Population von Cochleaten, wobei die Cochleate umfassen:
(a) ein negativ geladenes erstes Lipid;
(b) ein Kation, wobei das Kation ein divalentes Kation, ausgewählt aus der Gruppe bestehend aus Calcium-, Zink-, Barium- und Magnesiumkationen, ist;
(c) ein neutrales zweites Lipid; und
(d) ein biologisch relevantes Molekül, wobei das biologisch relevante Molekül ein Aminoglycosid ist;
wobei das Verhältnis des negativ geladenen ersten Lipids zu dem neutralen zweiten Lipid zwischen 1:1 und 9:1 beträgt;
wobei des negativ geladene erste Lipid Phosphatidylserin ist und zumindest 50 % des gesamten Lipids umfasst; und wobei das neutrale zweite Lipid Phosphatidylcholin oder Sphingomyelin ist.

2. Cochleat-Zusammensetzung gemäss Anspruch 1, wobei das Verhältnis des negativ geladenen ersten Lipids zu dem neutralen zweiten Lipid zwischen 4:1 und 9:1 beträgt.

3. Cochleat-Zusammensetzung gemäss Anspruch 1, wobei das zweite Lipid ein Lipid umfasst, das in der Lage ist, Wasserstoffbrückenbindungen an das biologisch relevante Molekül zu bilden.

4. Cochleat-Zusammensetzung gemäss Anspruch 1, wobei das zweite Lipid zwischen den negativ geladenen ersten Lipiden eingebettet ist.

5. Cochleat-Zusammensetzung gemäss Anspruch 1, wobei das negativ geladene Lipid Dioleoyl PS (DOPS) oder von Sojabohnen stammendes Phosphatidylserin (Soja-PS) umfasst.

6. Cochleat-Zusammensetzung gemäss Anspruch 1, wobei die Cochleate ferner eine geringe Menge eines dritten Lipids umfassen, wobei das dritte Lipid aus der Gruppe bestehend aus einem zwitterionischen Lipid, einem PEGyliertem Lipid, einem kationischen Lipid oder einem polykationischen Lipid ausgewählt ist.

7. Cochleat-Zusammensetzung gemäss Anspruch 1, wobei das Aminoglycosid aus der Gruppe bestehend aus Gentamicin, Netilmicin, Tobramycin, Amikacin, Kanamycin A, Kanamycin B, Neomycin, Paromomycin, Neamin, Streptomycin, Dihydrostreptomycin, Apramycin, Ribostamycin oder Spectinomycin ausgewählt ist.

8. Cochleat-Zusammensetzung gemäss Anspruch 1, die ferner einen Aggregationsinhibitor umfasst.

9. Cochleat-Zusammensetzung gemäss Anspruch 8, wobei der Aggregationsinhibitor Natriumchlorid ist.

10. Pharmazeutische Zusammensetzung, die eine wirksame Menge der Cochleat-Zusammensetzung gemäss Anspruch 1 und einen pharmazeutisch annehmbaren Träger umfasst.

11. Verfahren zur Herstellung der Cochleat-Zusammensetzung gemäss Anspruch 1, umfassend:
(a) Mischen des Aminoglycosids mit einem Liposom, umfassend ein negativ geladenes erstes Lipid und ein zweites Lipid, wobei das zweite Lipid ein neutrales zweites Lipid ist; und
(b) Zugabe eines Kations, wobei das Kation ein divalentes Kation, ausgewählt aus der Gruppe bestehend aus Calcium-, Zink-, Barium- und Magnesiumkationen, ist, um die Cochleat-Zusammensetzung herzustellen,
wobei das Verhältnis des negativ geladenen ersten Lipids zu dem neutralen zweiten Lipid zwischen 1:1 und 9:1 beträgt;
wobei des negativ geladene erste Lipid Phosphatidylserin ist und zumindest 50 % des gesamten Lipids umfasst; und
wobei das neutrale zweite Lipid Phosphatidylcholin oder Sphingomyelin ist.

12. Verfahren gemäss Anspruch 11, wobei das Verhältnis des gesamten Lipids zu dem biologisch relevanten Molekül zumindest 4:1 beträgt.

13. Verfahren gemäss Anspruch 11, wobei das Calciumkation aus Calciumchlorid bereitgestellt wird, wobei die Calciumchloridkonzentration der Mischung 2 bis 10 mM beträgt.

14. Verfahren gemäss Anspruch 11, die ferner einen Schritt (c) umfasst, worin ein Aggregationsinhibitor mit den Cochleat-Zusammensetzungen gemischt wird.

15. Zusammensetzung gemäss Anspruch 1 zur Verwendung in einem Verfahren zur Behandlung eines Individuums, umfassend die Verabreichung der Zusammensetzung gemäss Anspruch 1 an ein Individuum, wobei das Individuum irgend ein Tier, einschliesslich eines Menschen, einer Maus, einer Ratte, eines Kaninchens, eines nichtmenschlichen Primaten oder irgendeines anderen Säugers, ist.

16. Zusammensetzung zur Verwendung gemäss Anspruch 15, wobei die Verabreichung auf oralem, intranasalem, intraokularem, intraanalem, intravaginalem oder intrapulmonalem Weg erfolgt.

17. Zusammensetzung zur Verwendung gemäss Anspruch 16, wobei die Cochleat-Zusammensetzung ferner ein Gallensalz umfasst.

## Revendications

1. Composition de cochléates comprenant une population de cochléates, dans laquelle les cochléates comprennent :
a) un premier lipide chargé négativement ;
b) un cation, lequel cation est un cation divalent choisi parmi le groupe constitué du calcium, du zinc, du baryum, et des cations magnésiums ;
c) un deuxième lipide neutre ; et
d) une molécule biologiquement pertinente, laquelle molécule biologiquement pertinente est un aminoglycoside ;
dans laquelle le rapport du premier lipide chargé négativement au deuxième lipide neutre est entre 1:1 à 9:1 ;
dans laquelle le premier lipide chargé négativement est la phosphatidylsérine et comprend au moins 50% du lipide total ; et
dans laquelle le deuxième lipide neutre est la phosphatidylcholine ou la sphingomyéline.

2. La composition de cochléates de la revendication 1, dans laquelle le rapport du premier lipide chargé négativement au deuxième lipide neutre est entre 4:1 à 9:1.

3. La composition de cochléates de la revendication 1, dans laquelle le deuxième lipide comprend un lipide capable de former des liaisons hydrogène à la molécule biologiquement pertinente.

4. La composition de cochléates de la revendication 1, dans laquelle le deuxième lipide est enchevêtré dans le premier lipide chargé négativement.

5. La composition de cochléates de la revendication 1, dans laquelle le lipide chargé négativement comprend du dioléoyl PS (DOPS) ou de la phosphatidylsérine dérivée du soja (PS de soja).

6. La composition de cochléates de la revendication 1, dans laquelle les cochléates comprennent en outre une quantité minoritaire d'un troisième lipide, dans laquelle le troisième lipide est choisi parmi le groupe constitué d'un lipide zwitterionique, un lipide pégylé, un lipide cationique, ou un lipide polycationique.

7. La composition de cochléates de la revendication 1, dans laquelle l'aminoglycoside est choisi parmi le groupe de la gentamicine, la nétilmicine, la tobramycine, l'amikacine, la kanamycine A, la kanamycine B, la néomycine, la paromomycine, la néamine, la streptomycine, la dihydrostreptomycine, l'apramycine, la ribostamycine, ou la spectinomycine.

8. La composition de cochléates de la revendication 1, comprenant en outre un inhibiteur d'agrégation.

9. La composition de cochléates de la revendication 8, dans laquelle l'inhibiteur d'agrégation est le chlorure de sodium.

10. Composition pharmaceutique comprenant une quantité efficace de la composition de cochléates de la revendication 1 et un véhicule pharmaceutiquement acceptable.

11. Procédé de fabrication de la composition de cochléates de la revendication 1 comprenant :
a) le mélange de l'aminoglycoside avec un liposome comprenant un premier lipide chargé négativement et un deuxième lipide lequel deuxième lipide est un deuxième lipide neutre ; et
b) l'ajout d'un cation, lequel cation est un cation divalent choisi parmi le groupe constitué du calcium, du zinc, du baryum, et des cations magnésiums, pour faire la composition de cochléates,
dans laquelle le rapport du premier lipide chargé négativement au deuxième lipide neutre est entre 1:1 à 9:1 ;
dans laquelle le premier lipide chargé négativement est la phosphatidylsérine et comprend au moins 50% du lipide total ; et
dans laquelle le deuxième lipide neutre est la phosphatidylcholine ou la sphingomyéline.

12. Le procédé de la revendication 11, dans lequel le rapport du total lipide à la molécule biologiquement pertinente est d'au moins 4:1.

13. Le procédé de la revendication 11, dans lequel le cation calcium est fourni par du chlorure de calcium, dans lequel la concentration de chlorure de calcium du mélange est de 2 mM à 10 mM.

14. Le procédé de la revendication 11, comprenant en outre une étape c), dans laquelle un inhibiteur d'agrégation est mélangé avec la composition de cochléates.

15. La composition selon la revendication 1 pour l'utilisation dans une méthode de traitement d'un sujet comprenant l'administration de la composition de la revendication 1 à un sujet, dans laquelle le sujet est un animal quelconque, incluant un humain, une souris, un rat, un lapin, un primate non humain, ou un autre mammifère quelconque.

16. La composition pour l'utilisation selon la revendication 15, dans laquelle l'administration est par voie orale, intra-nasale, intraoculaire, intraanale, intravaginale, ou intrapulmonaire.

17. La composition pour l'utilisation selon la revendication 16, dans laquelle la composition de cochléates comprend en outre un sel biliaire.
